# EUROPEAN PATENT APPLICATION

(11) **EP 4 389 887 A1**
(43) Date of publication of application: **26.06.2024**
(21) Application number: 22306968.3
(22) Date of filing: 21.12.2022
(51) Int. Cl.: C12N 15/10, C12M 1/00

(54) **ISOLATING AND LYSING CELLS**

(71) Applicant: Mobidiag Oy, 02150 Espoo (FI)
(72) Inventor: FERRANTE, Ivan, 75011 PARIS (FR); MARCY, Yann, 75011 PARIS (FR); NIINIVAARA, Anne, H., 02150 ESPOO (FI); PITONI, Adriano, 75011 PARIS (FR); SANSIPERSICO, Francesca, 75011 PARIS (FR)
(74) Representative: Regimbeau

(57) **Abstract**

A system and method for heating a biological sample includes, or employs, a housing defining a filter chamber with a fluid inlet and outlet and a filter assembly within the chamber. The filter assembly includes filter media and a conductive mesh and fluid sample flowing through the chamber passes through both the filter media and the mesh. The conductive mesh is inductively heated by an induction coil disposed outside the chamber adjacent to a wall of the housing. The filter chamber may be part of a fluid cartridge including one or more chambers for containing fluid sample and other process fluids or functional connections for connecting external receptacles containing fluid sample and other process fluids. A fluid flow network includes fluid channels and flow control valves for selectively controlling fluid flow within the cartridge and through the filter assembly within the chamber.

## Description

### FIELD OF THE DISCLOSURE

This disclosure relates to systems and methods for isolating and lysing cells from a fluid component of a sample.

### BACKGROUND

Concentration methods to isolate pathogens from blood have been employed to isolate cells of interest in a much smaller volume than an initial sample for downstream nucleic acid amplification and detection. After concentration, it is necessary to lyse the isolated pathogens to release the nucleic acids. Lysis methods typically involve at least one of the following methods: (i) mechanical methods (e.g., bead mill, ultrasound, and French press); (ii) non-mechanical physical methods (e.g., thermolysis, decompression, and osmotic shock); and (iii) non-mechanical chemical and enzymatic methods (e.g., detergents, solvents, and enzymes). Enzymatic methods may be prone to species differences in performance, while chemical methods, although practiced more universally, are frequently not effective for hard to lyse cells and can also affect downstream processes by passing along chemicals that may be incompatible with amplification processes. Furthermore, the use of different enzymatic or chemical liquids in a lysing procedure increases the final volume of the sample mixture, thereby diminishing the benefits of concentrating the pathogens. In that respect, physical methods may be a more suitable. In the context of pathogen concentration techniques employing filter media, however, it is preferable to be able to localize the lysing process on a surface of the filter, and thus the efficacy of physical methods may be limited in such applications.

Heat is often used as a lysing agent, but devices for generating and applying heat to the cells are often slow and inefficient in reaching the desired temperature. In addition, externally applied heat may be detrimental to the use of plastic consumables typically employed in such concentration techniques due to the slow heat transfer properties of plastic and the need to bring a heating element (e.g., a resistive heater or thermoelectric device) into proximity with the plastic. Furthermore, integrated heating systems within sample processing devices require electrical contacts to provide power to the resistive heater or thermoelectric device, which increases the cost and complexity of such devices.

### SUMMARY

The following presents a simplified summary in order to provide a basic understanding of some aspects described herein. This summary is not an extensive overview of the claimed subject matter. It is intended to neither identify key or critical elements of the claimed subject matter nor delineate the scope thereof. Its sole purpose is to present some concepts in a simplified form as a prelude to the more detailed description that is presented later.

Devices, systems, and methods described herein employ induction heating to generate localized heat to lyse cells retained on a filter. A conductive mesh is assembled in close contact with a porous filter membrane into an integrated filter chamber, with a mesh size being substantially larger than the largest membrane pores so as not to interfere with the filtration process. Induction heating is generated by bringing into proximity of the device alternating electromagnetic fields by means of a coil situated outside the filter chamber. An advantage of such devices, systems, and methods is the ability to generate and apply localized heat in very close proximity to the cells captured on the filter without the need for electrical contacts to a heating mechanism positioned inside the filter chamber. The use of induction heating using a conductive mesh allows for free flow through a filter, generates localized heat where the cells are retained, and does not require external electrical contacts.

Embodiment A1 is a system for lysing cells contained in a sample, the system comprising: a housing defining a filter chamber and having a fluid inlet and a fluid outlet; filter media disposed within the filter chamber, wherein the filter media includes pores or interstices that are sized so as to prevent the passage of at least some cells contained in a sample to thereby separate the cells from a fluid component of the sample flowing through the filter media from the fluid inlet to the fluid outlet; a conductive mesh disposed within the filter chamber adjacent to the filter media, wherein the conductive mesh is configured to permit fluid flow therethrough; and an induction coil disposed outside the filter chamber and positioned to effect inductive heating in the conductive mesh when the induction coil is energized.

Embodiment A2 is the system of embodiment A1, wherein the housing is made from plastic.

Embodiment A3 is the system of embodiment A1, wherein the housing is made from a plastic selected from the group consisting of polypropylene, polycarbonate, polyacrylamide, polyethylene, polymethyl - methacrylate (PMMA), polydimetyl - siloxane (PDMS), and polyvinyl chloride (PVC).

Embodiment A4 is the system of embodiment A2, wherein the filter media is affixed to the housing by ultrasonic welding.

Embodiment A5 is the system of any one of embodiments A1 - A4, wherein the induction coil at least partially overlies or underlies the conductive mesh.

Embodiment A6 is the system of any one of embodiments A1 - A5, wherein the filter media comprises one or more filter elements.

Embodiment A7 is the system of any one of embodiments A1 - A6, wherein the filter media comprises a filter membrane.

Embodiment A8 is the system of any one of embodiments A1 - A7, wherein the conductive mesh is comprised of a metal or combination of metals.

Embodiment A9 is the system of any one of embodiments A1 - A8, wherein the conductive mesh is affixed within the filter chamber by heat stake welding.

Embodiment A10 is the system of embodiment A8 or A9, wherein the conductive mesh is comprised of iron, copper, stainless steel, nickel, and/or aluminum.

Embodiment A11 is the system of any one of embodiments A1 - A10, wherein the induction coil is made of copper.

Embodiment A12 is the system of any one of embodiments A1 -A11, wherein the induction coil is spaced from the conductive mesh by about 1.5 mm to 4.0 mm.

Embodiment A13 is the system of embodiment A12, wherein the induction coil is spaced from the conductive mesh by about 3.0 mm.

Embodiment A14 is the system of any one of embodiments A1 - A13, wherein the induction coil comprises a flat coil.

Embodiment A15 is the system of embodiment A14, wherein the flat coil surrounds the fluid outlet.

Embodiment A16 is the system of any one of embodiments A1 - A15, wherein the conductive mesh overlies or underlies the filter media or wherein the conductive mesh overlies and underlies the filter media.

Embodiment A17 is the system of any one of embodiments A1 - A16, wherein at least part of the conductive mesh is in contact with at least part of the filter media.

Embodiment A18 is the system of any one of embodiments A1 - A17, wherein the filter media and the conductive mesh are flat.

Embodiment A19 is the system of embodiment A18, wherein the filter media and the conductive mesh are generally parallel to each other.

Embodiment A20 is the system of any one of embodiments A1 - A19, wherein the filter media and the conductive mesh have the same general shape.

Embodiment A21 is the system of any one of embodiments A1 - A20, wherein the thickness of the conductive mesh ranges from 130 µm - 350 µm.

Embodiment A22 is the system of any one of embodiments A1 - A21, wherein the conductive mesh has openings of about 60 µm.

Embodiment A23 is the system of any one of embodiments A1 - A22, wherein the conductive mesh includes portions contacting opposed sides of the filter media.

Embodiment A24 is the system of any one of embodiments A1 - A17, wherein the filter media has a generally cylindrical configuration and the conductive mesh has a generally cylindrical configuration arranged coaxially with the filter media.

Embodiment A25 is the system of embodiment A24, wherein the conductive mesh is positioned radially outwardly of the filter media so that the conductive mesh at least partially encompasses the filter media.

Embodiment A26 is the system of embodiment A24 or A25, comprising a plenum positioned within the housing to receive fluid at the fluid inlet and introduce the fluid into the filter chamber at a location that is radially inward of the filter media, wherein the fluid flows radially outwardly through the filter media and the conductive mesh, and wherein the fluid outlet is located radially outwardly of the conductive mesh.

Embodiment A27 is the system of any one of embodiments A24 - A26, wherein the filter media is pleated.

Embodiment A28 is the system of any one of embodiments A1 - A27, wherein the conductive mesh comprises a woven wire mesh.

Embodiment A29 is the system of embodiment A28, wherein the woven wire mesh has openings of about 40 µm to about 300 µm.

Embodiment A30 is the system of any one of embodiments A1 - A27, wherein the conductive mesh comprises sintered fibers.

Embodiment A31 is the system of embodiment A30, wherein gaps between the sintered fibers define openings of about 3 µm to 100 µm.

Embodiment A32 is the system of any one of embodiments A1 - A27, wherein the conductive mesh comprises a perforated plate.

Embodiment A33 is the system of embodiment A32, wherein openings formed through the perforated plate each have a maximum width ranging from about 0.04 mm to about 0.3 mm.

Embodiment A34 is the system of embodiment A33, wherein the openings formed through the perforated plate are spaced apart from each other by a distance ranging from about 0.16 mm to about 0.6 mm.

Embodiment A35 is the system of any one of embodiments A1 - A34, wherein the filter media comprises a porous substrate and optionally comprises a sponge.

Embodiment A36 is the system of embodiment A35, wherein the porous substrate is made of polyethersulfone, cellulose, nylon, polyvinylidene fluoride (Poly(1,1-difluoroethylene), PVDF), polycarbonate, or glass fiber.

Embodiment A37 is the system of embodiment A35 or A36, wherein the porous substrate includes pores with a size of between about 0.1 µm and about 1.0 µm.

Embodiment A38 is the system of any one of embodiments A35 - A37, wherein the porous substrate has a thickness of 160 µm to 185 µm.

Embodiment A39 is the system of any one of embodiments A35 - A38, wherein the porous substrate is positioned and arranged within the filter chamber so that the sample flows through the porous substrate from a first side of the porous substrate to a second side of the porous substrate, and wherein the porous substrate includes pores decreasing in size from the first side to the second side.

Embodiment A40 is the system of embodiment A39, wherein the porous substrate comprises multiple layers of decreasing pore size progressing from the first side to the second side.

Embodiment A41 is the system of embodiment A40, wherein the porous substrate comprises two layers.

Embodiment A42 is the system of any one of embodiments A39 - A41, wherein the first side includes pores with a size of between about 5 µm and about 20 µm, and wherein the second side includes pores with a size of up to about 0.2 µm.

Embodiment A43 is the system of any one of embodiments A1 - A34, wherein the filter media comprises a track etched membrane.

Embodiment A44 is the system of embodiment A43, wherein the filter media further comprises a layer of sintered plastic particles.

Embodiment A45 is the system of embodiment A44, wherein the plastic particles are made of polyethylene, polypropylene, polytetrafluoroethylene, or polyvinyllidene fluoride.

Embodiment A46 is the system of any one of embodiments A43 - A45, wherein the track etched membrane has pores of about 0.2 µm in size.

Embodiment A47 is the system of any one of embodiments A43 - A46, wherein the track etched membrane has a thickness of about 22 µm.

Embodiment A48 is the system of embodiment A44 or A45, wherein interstices between the sintered plastic particles range in size from about 5 µm to about 15 µm.

Embodiment A49 is the system of any one of embodiments A44, A45, and A48, wherein the layer of sintered plastic particles has a thickness of about 450 µm to 650 µm.

Embodiment A50 is the system of any one of embodiments A1 - A49, further comprising a power source connected to the induction coil and configured to generate an alternating current in the induction coil so that the induction coil generates alternating electromagnetic fields.

Embodiment A51 is the system of any one of embodiments A1 - A50, further comprising a temperature sensor configured to measure a temperature of the conductive mesh.

Embodiment A52 is the system of embodiment A51, wherein the temperature sensor comprises a contactless temperature sensor.

Embodiment A53 is the system of embodiment A51 or A52, wherein the temperature sensor comprises an infrared temperature sensor.

Embodiment A54 is the system of any one of embodiments A1 - A49, further comprising: a power source connected to the induction coil and configured to generate alternating current in the induction coil; a temperature sensor configured to measure a temperature of the conductive mesh; and a temperature feedback control circuit connected to the power source and the temperature sensor and configured to control the alternating current generated by the power source based on temperature measured by the temperature sensor.

Embodiment A55 is the system of embodiment A54, wherein the temperature sensor comprises a contactless temperature sensor.

Embodiment A56 is the system of embodiment A54 or A55, wherein the temperature sensor comprises an infrared temperature sensor.

Embodiment A57 is the system of any one of embodiments A1 - A56, further comprising a syringe coupled to the fluid inlet of the filter chamber and comprising a syringe chamber and a plunger disposed within the syringe chamber, wherein the plunger is configured to be actuated to propel fluid from the syringe chamber into the filter chamber through the fluid inlet.

Embodiment B1 A system for lysing cells contained in a sample, the system comprising: a fluid cartridge comprising: a filter chamber with a fluid inlet and a fluid outlet; filter media disposed within the filter chamber, wherein the filter media includes pores or interstices that are sized so as to prevent the passage of at least some cells contained in a sample to thereby separate the cells from a fluid component of the sample flowing through the filter media; and a conductive mesh disposed within the filter chamber adjacent to the filter media, wherein the conductive mesh is configured to permit fluid flow therethrough; and an instrument configured to operate in cooperation with the fluid cartridge and including an induction coil disposed outside the filter chamber and configured to effect inductive heating of the conductive mesh of the fluid cartridge when the induction coil is energized.

Embodiment B2 is the system of embodiment B 1, wherein the fluid cartridge includes at least one inlet channel connected to the fluid inlet and at least one outlet channel connected to the fluid outlet and one or more valves controlling fluid flow in at least one of the fluid inlet channel and the fluid outlet channel.

Embodiment B3 is the system of embodiment B2, wherein the instrument comprises an actuator for selectively opening or closing each of the one or more valves.

Embodiment B4 is the system of any one of embodiments B1 - B3, further comprising a source of pressure for effecting fluid flow into the fluid inlet, through the filter chamber, and out the fluid outlet.

Embodiment B5 is the system of embodiment B4, wherein the source of pressure comprises a syringe coupled to the fluid cartridge, wherein the fluid cartridge or the syringe includes a syringe chamber and a plunger disposed within the syringe chamber.

Embodiment B6 is the system of embodiment B5, wherein the instrument further comprises an actuator configured to actuate the plunger.

Embodiment B7 is the system of any one of embodiments B1 - B5, wherein the instrument includes a dock for supporting the fluid cartridge within the instrument.

Embodiment B8 is the system of any one of embodiments B1 - B7, wherein the fluid cartridge is a microfluidic device.

Embodiment B9 is the system of embodiment B1, wherein the fluid cartridge further comprises: a syringe chamber for receiving a syringe plunger or a syringe port for coupling a syringe to the fluid cartridge; a sample reservoir or a sample port for connecting an external sample receptacle to the fluid cartridge; one or more process fluid reservoirs; a waste reservoir; a fluid discharge port from which a fluid is discharged from the cartridge; and a fluid flow network comprising fluid channels and flow control valves for selectively permitting fluid flow: (i) from the sample reservoir or the sample port to the syringe chamber or the syringe port, (ii) from the syringe chamber or the syringe port to the fluid inlet of the filter chamber and from the fluid outlet of the filter chamber to the waste chamber, (iii) from at least one of the one or more process fluid reservoirs to the syringe chamber or the syringe port, or (iv) from the syringe chamber or the syringe port to the fluid inlet of the filter chamber and from the fluid outlet of the filter chamber to the fluid discharge port.

Embodiment B10 is the system of embodiment B9, wherein the fluid cartridge further comprises one or more chambers containing dried reagents.

Embodiment B11 is the system of embodiment B9 or B10, comprising a syringe port for coupling a syringe to the fluid cartridge and a syringe configured to be removably coupled to the syringe port.

Embodiment B12 is the system of any one of embodiments B9 - B 11, wherein the sample port comprises a sample container well for coupling a sample receptacle to the fluid cartridge.

Embodiment B13 is the system of embodiment B12, further comprising a sample receptacle coupled to the sample container well.

Embodiment B14 is the system of any one of embodiments B9 - B13, wherein the instrument comprises an actuator configured to selectively open or close each of the flow control valves.

Embodiment B15 is the system of embodiment B14, wherein the actuator comprises: a plunger associated with each flow control valve, wherein each plunger is movable between an engaged position in which the plunger engages the associated flow control valve to close the associated flow control valve and a disengaged position in which the plunger disengages the associated flow control valve to open the associated flow control valve; and a cam actuator configured to mechanically actuate the plungers between their engaged positions and their disengaged positions.

Embodiment B16 is the system of embodiment B15, wherein the cam actuator comprises a rotary cam that is rotatable about an axis of rotation and includes a first surface and a cam recess, wherein as the rotary cam rotates about its axis of rotation, each plunger coupled to the first surface is disposed in its respective engaged position and each plunger coupled with the cam recess is disposed in its disengaged position.

Embodiment B17 is the system of embodiment B16, wherein the cam actuator comprises an actuating ball associated with each plunger and disposed between the rotary cam and the associated plunger to contact the first surface of the rotary cam and the plunger when the plunger is coupled to the first surface and to contact the cam recess of the rotary cam and the plunger when the plunger is coupled to the cam recess.

Embodiment B18 is the system of any one of embodiments B9 - B 17, wherein each flow control valve comprises a recess formed in an outer surface of the cartridge, with a through hole connecting two of the fluid channels formed in a bottom of the recess, and a flexible material extended over the recess, wherein the flexible material is deformable under the force of an external actuator into the recess to block the through hole.

Embodiment B19 is the system of embodiment B14, wherein each flow control valve comprises a recess formed in an outer surface of the cartridge, with a through hole connecting two of the fluid channels formed in a bottom of the recess, and a flexible material extended over the recess, wherein the flexible material is deformable under the force of an external actuator into the recess to block the through hole, and wherein each actuator comprises a plunger associated with each recess, and each plunger is movable between an engaged position in which the plunger presses the flexible material into the associated recess to block the through opening and close the associated flow control valve and a disengaged position in which the plunger does not press the flexible material into the associated recess so the flexible material extends over the associated recess and the through opening is not blocked and the associated flow control valve is open.

Embodiment B20 is the system of embodiment B18 or B19, wherein the flexible material is made of a thermoplastic.

Embodiment B21 is the system of any one of embodiments B18 - B20, wherein the flexible material is made of polypropylene.

Embodiment B22 is the system of any one of embodiments B1 - B21, wherein the fluid cartridge comprises a cartridge body made from plastic.

Embodiment B23 is the system of embodiment B22, wherein the cartridge body is made from a plastic selected from the group consisting of polypropylene, polycarbonate, polyacrylamide, polyethylene, polymethyl - methacrylate (PMMA), polydimetyl - siloxane (PDMS), and polyvinyl chloride (PVC).

Embodiment B24 is the system of any one of embodiments B1 - B23, wherein the filter media is affixed to the filter chamber by ultrasonic welding.

Embodiment B25 is the system of any one of embodiments B1 - B24, wherein the induction coil at least partially overlies or underlies the conductive mesh.

Embodiment B26 is the system of any one of embodiments B1 - B25, wherein the filter media comprises one or more filter elements.

Embodiment B27 is the system of any one of embodiments B 1 - B26, wherein the filter media comprises a filter membrane.

Embodiment B28 is the system of any one of embodiments B1 - B27, wherein the conductive mesh is comprised of a metal or combination of metals.

Embodiment B29 is the system of any one of embodiments B1 - B28, wherein the conductive mesh is affixed within the filter chamber by heat stake welding.

Embodiment B30 is the system of embodiment B28 or B29, wherein the conductive mesh is comprised of iron, copper, stainless steel, nickel, and/or aluminum.

Embodiment B31 is the system of any one of embodiments B1 - B30, wherein the induction coil is made of copper.

Embodiment B32 is the system of any one of embodiments B1 - B31, wherein the induction coil is spaced from the conductive mesh by about 1.5 mm - 4.0 mm.

Embodiment B33 is the system of embodiment B32, wherein the induction coil is spaced from the conductive mesh by about 3 mm.

Embodiment B34 is the system of any one of embodiments B1 - B33, wherein the induction coil comprises a flat coil.

Embodiment B35 is the system of any one of embodiments B1 - B34, wherein the conductive mesh overlies or underlies the filter media or wherein the conductive mesh overlies and underlies the filter media.

Embodiment B36 is the system of any one of embodiments B1 - B35, wherein at least part of the conductive mesh is in contact with at least part of the filter media.

Embodiment B37 is the system of any one of embodiments B1 - B36, wherein the filter media and the conductive mesh are flat.

Embodiment B38 is the system of embodiment B37, wherein the filter media and the conductive mesh are generally parallel to each other.

Embodiment B39 is the system of any one of embodiments B1 - B38, wherein the filter media and the conductive mesh have the same general shape.

Embodiment B40 is the system of any one of embodiments B1 - B39, wherein the thickness of the conductive mesh ranges from 130 µm - 350 µm.

Embodiment B41 is the system of any one of embodiments B1 - B40, wherein the conductive mesh has openings of about 60 µm.

Embodiment B42 is the system of any one of embodiments B1 - B41, wherein the conductive mesh includes portions contacting opposed sides of the filter media.

Embodiment B43 is the system of any one of embodiments B1 - B36, wherein the filter media has a generally cylindrical configuration and the conductive mesh has a generally cylindrical configuration arranged coaxially with the filter media.

Embodiment B44 is the system of embodiment B43, wherein the conductive mesh is positioned radially outwardly of the filter media so that the conductive mesh at least partially encompasses the filter media.

Embodiment B45 is the system of embodiment B43 or B44, comprising a plenum positioned within the filter chamber to receive fluid at the fluid inlet and introduce the fluid into the filter chamber at a location that is radially inward of the filter media, wherein the fluid flows radially outwardly through the filter media and the conductive mesh, and wherein the fluid outlet is located radially outwardly of the conductive mesh.

Embodiment B46 is the system of any one of embodiments B43 - B45, wherein the filter media is pleated.

Embodiment B47 is the system of any one of embodiments B1 - B46, wherein the conductive mesh comprises a woven wire mesh.

Embodiment B48 is the system of embodiment B47, wherein the woven wire mesh has openings of about 40 µm to about 300 µm.

Embodiment B49 is the system of any one of embodiments B1 - B46, wherein the conductive mesh comprises sintered fibers.

Embodiment B50 is the system of embodiment B49, wherein gaps between the sintered fibers define openings of about 3 µm to 100 µm.

Embodiment B51 is the system of any one of embodiments B1 - B46, wherein the conductive mesh comprises a perforated plate.

Embodiment B52 is the system of embodiment B51, wherein openings formed through the perforated plate each have a maximum width ranging from about 0.04 mm to about 0.3 mm.

Embodiment B53 is the system of embodiment B52, wherein the openings formed through the perforated plate are spaced apart from each other by a distance ranging from about 0.16 mm to about 0.6 mm.

Embodiment B54 is the system of any one of embodiments B1 - B53, wherein the filter media comprises porous substrate and optionally comprises a sponge.

Embodiment B55 is the system of embodiment B54, wherein the porous substrate is made of polyethersulfone, cellulose, nylon, polyvinylidene fluoride (Poly(1,1-difluoroethylene), PVDF), polycarbonate, or glass fiber.

Embodiment B56 is the system of embodiment B54 or B55, wherein the porous substrate includes pores with a size of between about 0.1 µm and about 1.0 µm.

Embodiment B57 is the system of any one of embodiments B54 - B56, wherein the porous substrate has a thickness of 160 µm to 185 µm.

Embodiment B58 is the system of any one of embodiments B54 - B57, wherein the porous substrate is positioned and arranged within the filter chamber so that the sample flows through the porous substrate from a first side of the porous substrate to a second side of the porous substrate, and wherein the porous substrate includes pores decreasing in size from the first side to the second side.

Embodiment B59 is the system of embodiment B58, wherein the porous substrate comprises multiple layers of decreasing pore size progressing from the first side to the second side.

Embodiment B60 is the system of embodiment B59, wherein the porous substrate comprises two layers.

Embodiment B61 is the system of any one of embodiments B58 - B60, wherein the first side includes pores with a size of between about 5 µm and about 20 µm, and wherein the second side includes pores with a size of up to about 0.2 µm.

Embodiment B62 is the system of any one of embodiments B1 - B53, wherein the filter media comprises a track etched membrane.

Embodiment B63 is the system of embodiment B62, wherein the filter media further comprises a layer of sintered plastic particles.

Embodiment B64 is the system of embodiment B63, wherein the plastic particles are made of polyethylene, polypropylene, polytetrafluoroethylene, or polyvinyllidene fluoride.

Embodiment B65 is the system of any one of embodiments B62 - B64, wherein the track etched membrane has pores of about 0.2 µm in size.

Embodiment B66 is the system of any one of embodiments B62 - B65, wherein the track etched membrane has a thickness of about 22 µm.

Embodiment B67 is the system of embodiment B63 or B64, wherein interstices between the sintered plastic particles range in size from about 5 µm to about 15 µm.

Embodiment B68 is the system of any one of embodiments B63, B64, and B67, wherein the layer of sintered plastic particles has a thickness of about 450 µm to 650 µm.

Embodiment B69 is the system of any one of embodiments B1 - B68, further comprising a power source connected to the induction coil and configured to generate alternating electromagnetic fields in the induction coil.

Embodiment B70 is the system of any one of embodiments B1 - B69, further comprising a temperature sensor configured to measure a temperature of the conductive mesh.

Embodiment B71 is the system of embodiment B70, wherein the temperature sensor comprises a contactless temperature sensor.

Embodiment B72 is the system of embodiment B70 or B71, wherein the temperature sensor comprises an infrared temperature sensor.

Embodiment B73 is the system of any one of embodiments B1 - B68, further comprising: a power source connected to the induction coil and configured to generate alternating electromagnetic fields in the induction coil; a temperature sensor configured to measure a temperature of the conductive mesh; and a temperature feedback control circuit connected to the power source and the temperature sensor and configured to control electromagnetic fields generated by the power source based on temperature measured by the temperature sensor.

Embodiment B74 is the system of embodiment B73, wherein the temperature sensor comprises a contactless temperature sensor.

Embodiment B75 is the system of embodiment B73 or B74, wherein the temperature sensor comprises an infrared temperature sensor.

Embodiment B76 is the system of embodiment B1, further comprising a syringe coupled to the fluid inlet of the filter chamber and comprising a syringe chamber and a plunger disposed within the syringe chamber, wherein the plunger is configured to be actuated to propel fluid from the syringe chamber into the filter chamber through the fluid inlet.

Embodiment C1 is a method comprising the steps of: (a) moving a sample through filter media and a conductive mesh disposed adjacent the filter media, wherein the filter media and the conductive mesh are contained within a filter chamber, and wherein the filter media includes pores or interstices that are sized so as to prevent the passage of at least some cells contained within the sample to thereby separate the cells from a fluid component of the sample flowing through the filter media; and (b) after step (a), energizing an induction coil disposed outside the filter chamber and positioned to effect inductive heating of the conductive mesh when the induction coil is energized to inductively heat the conductive mesh and thermally lyse cells separated from the fluid component of the sample by the filter media, such that nucleic acids are released from the separated cells.

Embodiment C2 is the method of embodiment C1, further comprising, prior to step (a), combining the sample with a lysing solution, and wherein step (a) comprises moving the sample and the lysing solution through the filter medial and the conductive mesh.

Embodiment C3 is the method of embodiment C1 or C2, further comprising, after step (a) and before step (b), the step of moving a wash buffer through the filter media and the conductive mesh.

Embodiment C4 is the method of any one of embodiments C1 - C3, further comprising, after step (b), the step of moving an elution buffer through the filter media and the conductive mesh.

Embodiment C5 is the method of any one of embodiments C1 - C4, wherein energizing the induction coil comprises generating an alternating current that is applied to the induction coil to generate an electromagnetic field.

Embodiment C6 is the method of any one of embodiments C1 - C5, wherein the conductive mesh is heated to a temperature of 80°C - 150°C in step (b).

Embodiment C7 is the method of any one of embodiments C1 - C6, further comprising the steps of: monitoring a temperature of the conductive mesh; and controlling the energizing of the induction coil based on the temperature of the conductive mesh.

Embodiment C8 is the method of any one of embodiments C1 - C7, wherein step (a) comprises: fluidly connecting a syringe to a sample container; actuating the syringe to draw an amount of sample from the sample container into the syringe; fluidly connecting the syringe to the filter chamber and disconnecting the syringe from the sample container; and actuating the syringe to expel the sample from the syringe and propel the sample through the filter media and conductive mesh.

Embodiment C9 is the method of any one of embodiments C1 - C8, further comprising the steps of: (c) after step (b), transporting the released nucleic acids from the filter chamber to an amplification chamber; and (d) after step (c), performing a nucleic acid amplification procedure on the nucleic acids in the amplification chamber.

Embodiment C10 is the method of embodiment C9, wherein the amplification procedure is an isothermal procedure or a thermocyclic procedure.

Embodiment C11 is the method of embodiment C9 or C10, further comprising the step of: (e) after step (d), detecting an optical signal that is representative of a target nucleic acid in the sample.

Embodiment C12 is the method of any one of embodiments C1 - C11, wherein step (a) comprise moving sample through at least one inlet channel connected to a fluid inlet of the filter chamber and at least one outlet channel connected to a fluid outlet of the filter chamber.

Embodiment C13 is the method of embodiment C12, further comprising selectively opening or closing one or more valves controlling fluid flow in at least one of the fluid inlet channel and the fluid outlet channel.

Embodiment C14 is the method of embodiment C1, wherein step (a) is performed in a fluid cartridge comprising: a syringe barrel for receiving a syringe plunger or a syringe port for coupling a syringe to the fluid cartridge; a sample reservoir or a sample port for connecting an external sample receptacle to the fluid cartridge; one or more process fluid reservoirs; a waste chamber; a fluid discharge port from which a fluid is discharged from the cartridge; and a fluid flow network comprising fluid channels and flow control valves, and wherein the method further comprises selectively opening and closing each of the flow control valves to selectively permit flow: (i) from the sample reservoir or the sample port to the syringe barrel or the syringe port, (ii) from the syringe barrel or the syringe port to the filter chamber and from the filter chamber to the waste chamber, (iii) from at least one of the one or more process fluid reservoirs to the syringe barrel or the syringe port, and (iv) from the syringe barrel or the syringe port to the filter chamber and from the filter chamber to the fluid discharge port.

Embodiment C15 is the method of embodiment C14, wherein the fluid cartridge further comprises one or more chambers containing dried reagents.

Embodiment C16 is the method of embodiment C14 or C15, wherein the fluid cartridge comprises a syringe configured to be received within the syringe barrel or coupled to the syringe port.

Embodiment C17 is the method of any one of embodiments C14 - C16, wherein each of the flow control valves is selectively opened and closed with an actuator.

Embodiment C18 is the method of embodiment C17, wherein the actuator comprises: a plunger associated with each flow control valve, and wherein opening and closing the plunger comprises moving the plunger between an engaged position in which the plunger engages the associated flow control valve to close the associated flow control valve and a disengaged position in which the plunger disengages the associated flow control valve to open the associated flow control valve; and wherein actuating the plunger comprises mechanically actuating the plungers with a cam actuator.

Embodiment C19 is the method of embodiment C18, wherein the cam actuator comprises a rotary cam that includes a first surface and a cam recess, and wherein mechanically actuating the plungers comprises rotating the rotary cam about an axis of rotation to couple each plunger with the first surface to position the respective plunger in its engaged position and to couple each plunger with the cam recess to position the respective plunger in its disengaged position.

Embodiment C20 is the method of embodiment C19, wherein the cam recess includes portions disposed on opposite sides of the axis of ration of the rotary cam.

Embodiment C21 is the method of embodiment C19 or C20, wherein the cam actuator comprises an actuating ball associated with each plunger and disposed between the rotary cam and the associated plunger to contact the first surface of the rotary cam and the plunger when the plunger is coupled to the first surface and to contact the cam recess of the rotary cam and the plunger when the plunger is coupled to the cam recess.

Embodiment C22 is the method of any one of embodiments C14 - C21, wherein the fluid cartridge comprises a body made from plastic.

Embodiment C23 is the method of embodiment C22, wherein the body is made from polypropylene, polycarbonate, polyacrylamide, polyethylene, polymethyl-methacrylate (PMMA), polydimetyl-siloxane (PDMS), or polyvinyl chloride (PVC).

Embodiment C24 is the method of embodiment C22 or C23, wherein each flow control valve comprises a recess formed in an outer surface of the body, with a through hole connecting two of the fluid channels formed in a bottom of the recess, and a flexible material extended over the recess, wherein closing the flow control valve comprises deforming the flexible material under the force of an external actuator into the recess to block the through hole.

Embodiment C25 is the method of embodiment C24, wherein the flexible material is made of a thermoplastic.

Embodiment C26 is the method of embodiment C24 or C25, wherein the flexible material is made of polypropylene.

Embodiment C27 is the method of any one of embodiments C1 - C26, wherein the induction coil at least partially overlies or underlies the conductive mesh.

Embodiment C28 is the method of any one of embodiments C1 - C27, wherein the filter media comprises one or more filter elements.

Embodiment C29 is the method of any one of embodiments C1 - C28, wherein the filter media comprises a filter membrane.

Embodiment C30 is the method of any one of embodiments C1 - C29, wherein the conductive mesh is comprised of a metal or combination of metals.

Embodiment C31 is the method of embodiment C30, wherein the conductive mesh is comprised of iron, copper, stainless steel, nickel, and/or aluminum.

Embodiment C32 is the method of any one of embodiments C1 - C31, wherein the induction coil comprises a flat coil.

Embodiment C33 is the method of any one of embodiments C1 - C32, wherein the conductive mesh overlies or underlies the filter media or wherein the conductive mesh overlies and underlies the filter media.

Embodiment C34 is the method of any one of embodiments C1 - C33, wherein at least part of the conductive mesh is in contact with at least part of the filter media.

Embodiment C35 is the method of any one of embodiments C1 - C34, wherein the filter media and the conductive mesh are flat.

Embodiment C36 is the method of embodiment C35, wherein the filter media and the conductive mesh are generally parallel to each other.

Embodiment C37 is the method of any one of embodiments C1 - C36, wherein the thickness of the conductive mesh ranges from 130 µm - 350 µm.

Embodiment C38 is the method of any one of embodiments C1 - C37, wherein the conductive mesh includes portions contacting opposed sides of the filter media.

Embodiment C39 is the method of any one of embodiments C1 - C38, wherein the filter media has a generally cylindrical configuration and the conductive mesh has a generally cylindrical configuration arranged coaxially with the filter media.

Embodiment C40 is the method of embodiment C39, wherein the conductive mesh is positioned radially outwardly of the filter media so that the conductive mesh at least partially encompasses the filter media.

Embodiment C41 is the method of embodiment C39 or C40, comprising a plenum positioned within the filter chamber to receive fluid at the fluid inlet and introduce the fluid into the filter chamber at a location that is radially inward of the filter media, wherein the fluid flows radially outwardly through the filter media and the conductive mesh, and wherein the fluid outlet is located radially outwardly of the conductive mesh.

Embodiment C42 is the method of any one of embodiments C1 -C41, wherein the conductive mesh comprises a woven wire mesh.

Embodiment C43 is the method of embodiment C42, wherein the woven wire mesh has openings of about 40 µm to about 300 µm.

Embodiment C44 is the method of any one of embodiments C1 - C41, wherein the conductive mesh comprises sintered fibers.

Embodiment C45 is the method of embodiment C44, wherein gaps between the sintered fibers define openings of about 3 µm to 100 µm.

Embodiment C46 is the method of any one of embodiments C1 - C41, wherein the conductive mesh comprises a perforated plate.

Embodiment C47 is the method of embodiment C46, wherein the openings formed through the perforated plate each have a maximum width ranging from about 0.04 mm to about 0.3 mm.

Embodiment C48 is the method of embodiment C47, wherein the openings formed through the perforated plate are spaced apart from each other by a distance ranging from about 0.16 mm to about 0.6 mm.

Embodiment C49 is the method of any one of embodiments C1 - C48, wherein the filter media comprises a porous substrate and optionally comprises a sponge.

Embodiment C50 is the method of embodiment C49, wherein the porous substrate is made of polyethersulfone, cellulose, nylon, polyvinylidene fluoride (Poly(1,1-difluoroethylene), PVDF), polycarbonate, or glass fiber.

Embodiment C51 is the method of embodiment C49 or C50, wherein the porous substrate includes pores with a size of between about 0.1 µm and about 1.0 µm.

Embodiment C52 is the method of any one of embodiments C49 - C51, wherein the porous substrate has a thickness of about 160 µm to about 185 µm.

Embodiment C53 is the method of any one of embodiments C49 - C52, wherein step (a) comprises moving a sample through the porous substrate from a first side of the porous substrate to a second side of the porous substrate, and wherein the porous substrate includes pores decreasing in size from the first side of the porous substrate to the second side of the porous substrate.

Embodiment C54 is the method of embodiment C53, wherein the porous substrate comprises multiple layers of decreasing pore size progressing from the first side of the porous substrate to the second side of the porous substrate.

Embodiment C55 is the method of embodiment C54, wherein the porous substrate comprises two layers.

Embodiment C56 is the method of any one of embodiments C53 - C55, wherein the first side of the porous substrate includes pores with a size of between about 5 µm and about 20 µm, and wherein the second side of the porous substrate includes pores with a size of up to about 0.2 µm.

Embodiment C57 is the method of any one of embodiments C1 - C48, wherein the filter media comprises a track etched membrane.

Embodiment C58 is the method of embodiment C57, wherein the filter media further comprises a layer of sintered plastic particles.

Embodiment C59 is the method of embodiment C58, wherein the plastic particles are made of polyethylene, polypropylene, polytetrafluoroethylene, or polyvinyllidene fluoride.

Embodiment C60 is the method of any one of embodiments C57 - C59, wherein the track etched membrane has pores of about 0.2 µm in size.

Embodiment C61 is the method of any one of embodiments C57 - C60, wherein the track etched membrane has a thickness of about 22 µm.

Embodiment C62 is the method of embodiment C58 or C59, wherein interstices between the sintered plastic particles range in size from about 5 µm to about 15 µm.

Embodiment C63 is the method of any one of embodiments C58, C58, and C62, wherein the layer of sintered plastic particles has a thickness of about 450 µm to 650 µm.

Embodiment D1 is a system for separating cells from a sample and for lysing the separated cells, the system comprising: walls defining a filter chamber having a fluid inlet and a fluid outlet; filter media disposed within the filter chamber, wherein the filter media includes pores or interstices that are sized so as to prevent the passage of at least some cells contained in the sample to thereby separate the cells from a fluid component of the sample flowing through the filter media; and a conductive mesh disposed within the filter chamber adjacent to the filter media, wherein the conductive mesh is configured to permit fluid flow therethrough, and wherein one of the walls defining the filter chamber, or a portion of such wall, is configured to permit the conductive mesh to be in sufficient proximity to an energized induction coil disposed outside the filter chamber adjacent the wall or wall portion so that the energized induction coil effects inductive heating in the conductive mesh to lyse at least a portion of the cells separated from the sample by the filter media.

Embodiment D2 is the system of embodiment D1, further comprising an induction coil disposed outside the filter chamber adjacent the wall or wall portion so that the energized induction coil effects inductive heating in the conductive mesh to lyse at least a portion of the cells separated from the sample by the filter media.

Embodiment D3 is the system of embodiment D1 or D2, wherein the walls defining the filter chamber comprise a first wall and a second wall, wherein the fluid inlet extends through the first wall, and the fluid outlet extends through the second wall, and wherein the filter media and the conductive mesh are generally flat and parallel to each other and are disposed in a gap between the first wall and the second wall.

Embodiment D4 is the system of any one of embodiments D1 - D3, wherein the walls are made from plastic.

Embodiment D5 is the system of embodiment D4, wherein the walls are made from a plastic selected from the group consisting of polypropylene, polycarbonate, polyacrylamide, polyethylene, polymethyl-methacrylate (PMMA), polydimetyl-siloxane (PDMS), and polyvinyl chloride (PVC).

Embodiment D6 is the system of embodiment D4 or D5, wherein the filter media is affixed within the filter chamber by ultrasonic welding.

Embodiment D7 is the system of any one of embodiments D1 - D6, wherein the induction coil at least partially overlies or underlies the conductive mesh.

Embodiment D8 is the system of any one of embodiments D1 - D7, wherein the filter media comprises one or more filter elements.

Embodiment D9 is the system of any one of embodiments D1 - D8, wherein the filter media comprises a filter membrane.

Embodiment D10 is the system of any one of embodiments D1 - D9, wherein the conductive mesh is comprised of a metal or combination of metals.

Embodiment D11 is the system of any one of embodiments D1 - D10, wherein the conductive mesh is affixed within the filter chamber by heat stake welding.

Embodiment D12 is the system of embodiment D10 or D11, wherein the conductive mesh is comprised of iron, copper, stainless steel, nickel, and/or aluminum.

Embodiment D13 The system of any one of embodiments D1 - D12, wherein the induction coil is made of copper.

Embodiment D14 The system of embodiment D2, wherein the induction coil is spaced from the conductive mesh by about 1.5 mm to 4.0 mm.

Embodiment D15 is the system of embodiment D14, wherein the induction coil is spaced from the conductive mesh by about 3.0 mm.

Embodiment D16 The system of embodiment D2, D14, or D15, wherein the induction coil comprises a flat coil.

Embodiment D17 The system of embodiment D16, wherein the flat coil surrounds the fluid outlet.

Embodiment D18 is the system of any one of embodiments D1 - D17, wherein the conductive mesh overlies or underlies the filter media or wherein the conductive mesh overlies and underlies the filter media.

Embodiment D19 is the system of any one of embodiments D1 - D18, wherein at least part of the conductive mesh is in contact with at least part of the filter media.

Embodiment D20 is the system of any one of embodiments D1 - D19, wherein the filter media and the conductive mesh are flat.

Embodiment D21 is the system of embodiment D20, wherein the filter media and the conductive mesh are generally parallel to each other.

Embodiment D22 is the system of any one of embodiments D1 - D21, wherein the filter media and the conductive mesh have the same general shape.

Embodiment D23 is the system of any one of embodiments D1 - D22, wherein the thickness of the conductive mesh ranges from 130 µm-350 µm.

Embodiment D24 is the system of any one of embodiments D1 - D23, wherein the conductive mesh includes portions contacting opposed sides of the filter media.

Embodiment D25 is the system of any one of embodiments D1 - D19, wherein the filter media has a generally cylindrical configuration and the conductive mesh has a generally cylindrical configuration arranged coaxially with the filter media.

Embodiment D26 is the system of embodiment D25, wherein the conductive mesh is positioned radially outwardly of the filter media so that the conductive mesh at least partially encompasses the filter media.

Embodiment D27 is the system of embodiment D25 or D26, comprising a plenum positioned within the filter chamber to receive fluid at the fluid inlet and introduce the fluid into the filter chamber at a location that is radially inward of the filter media, wherein the fluid flows radially outwardly through the filter media and the conductive mesh, and wherein the fluid outlet is located radially outwardly of the conductive mesh.

Embodiment D28 is the system of any one of embodiments D25 - D27, wherein the filter media is pleated.

Embodiment D29 is the system of any one of embodiments D1 - D28, wherein the conductive mesh comprises a woven wire mesh.

Embodiment D30 is the system of embodiment D29, wherein the woven wire mesh has openings of about 40 µm to about 300 µm.

Embodiment D31 is the system of any one of embodiments D1 - D28, wherein the conductive mesh comprises sintered fibers.

Embodiment D32 is the system of embodiment D31, wherein gaps between the sintered fibers define openings of about 3 µm to 100 µm.

Embodiment D33 is the system of any one of embodiments D1 - D28, wherein the conductive mesh comprises a perforated plate.

Embodiment D34 is the system of embodiment D33, wherein openings formed through the perforated plate each have a maximum width ranging from about 0.04 mm to about 0.3 mm.

Embodiment D35 is the system of embodiment D34, wherein the openings formed through the perforated plate are spaced apart from each other by a distance ranging from about 0.16 mm to about 0.6 mm.

Embodiment D36 is the system of any one of embodiments D1 - D35, wherein the filter media comprises a porous substrate and optionally comprises a sponge.

Embodiment D37 is the system of embodiment D36, wherein the porous substrate is made of polyethersulfone, cellulose, nylon, polyvinylidene fluoride (Poly(1,1-difluoroethylene), PVDF), polycarbonate, or glass fiber.

Embodiment D38 is the system of embodiment D36 or D37, wherein the porous substrate includes pores with a size of between about 0.1 µm and about 1.0 µm.

Embodiment D39 is the system of any one of embodiments D36 - D38, wherein the porous substrate has a thickness of 160 µm to 185 µm.

Embodiment D40 is the system of any one of embodiments D36 - D39, wherein the porous substrate is positioned and arranged within the filter chamber so that sample flows through the porous substrate from a first side of the porous substrate to a second side of the porous substrate, and wherein the porous substrate includes pores decreasing in size from the first side to the second side.

Embodiment D41 is the system of embodiment D40, wherein the porous substrate comprises multiple layers of decreasing pore size progressing from the first side to the second side.

Embodiment D42 is the system of embodiment D41, wherein the porous substrate comprises two layers.

Embodiment D43 is the system of any one of embodiments D40 - D42, wherein the first side includes pores with a size of between about 5 µm and about 20 µm, and wherein the second side includes pores with a size of up to about 0.2 µm.

Embodiment D44 is the system of any one of embodiments D1 - D35, wherein the filter media comprises a track etched membrane.

Embodiment D45 is the system of embodiment D44, wherein the filter media further comprises a layer of sintered plastic particles.

Embodiment D46 is the system of embodiment D45, wherein the plastic particles are made of polyethylene polypropylene, polytetrafluoroethylene, or polyvinyllidene fluoride.

Embodiment D47 is the system of any one of embodiments D44 - D46, wherein the track etched membrane has pores of up to about 0.2 µm in size.

Embodiment D48 is the system of any one of embodiments D44 - D47, wherein the track etched membrane has a thickness of about 22 µm.

Embodiment D49 is the system of any one of embodiments D45 or D46, wherein interstices between the sintered plastic particles range in size from about 5 µm to about 15 µm.

Embodiment D50 is the system of any one of embodiments D45, D46, and D49, wherein the layer of sintered plastic particles has a thickness of about 450 µm to 650 µm.

Embodiment D51 is the system of any one of embodiments D1 -D50, further comprising a temperature sensor configured to measure a temperature of the conductive mesh.

Embodiment D52 is the system of embodiment D51, wherein the temperature sensor comprises a contactless temperature sensor.

Embodiment D53 is the system of embodiment D51 or D52, wherein the temperature sensor comprises an infrared temperature sensor.

Embodiment D54 is the system of embodiment D2, further comprising a power source connected to the induction coil and configured to generate an alternating current in the induction coil so that the induction coil generates alternating electromagnetic fields.

Embodiment D55 is the system of embodiment D54, further comprising: a temperature sensor configured to measure a temperature of the conductive mesh; and a temperature feedback control circuit connected to the power source and the temperature sensor and configured to control electromagnetic fields generated by the power source based on temperature measured by the temperature sensor.

Embodiment D56 is the system of embodiment D55, wherein the temperature sensor comprises a contactless temperature sensor.

Embodiment D57 is the system of embodiment D55 or D56, wherein the temperature sensor comprises an infrared temperature sensor.

Embodiment D58 is the system of any one of embodiments D1 -D57, further comprising a syringe coupled to the fluid inlet of the filter chamber and comprising a syringe chamber and a plunger disposed within the syringe chamber, wherein the plunger is configured to be actuated to propel fluid from the syringe chamber into the filter chamber through the fluid inlet.

Embodiment E1 is a system for separating cells from a sample and for lysing the separated cells, the system comprising: a sample reservoir; an inductively heatable filter assembly positioned to filter sample flowing from the sample reservoir through the filter assembly, wherein the filter assembly comprises: filter media, wherein the filter media includes pores or interstices that are sized so as to prevent the passage of at least some cells contained within a sample to thereby separate cells from a fluid component of the sample flowing through the filter media; and a conductive mesh disposed adjacent to the filter media, wherein the conductive mesh is configured to permit fluid flow therethrough; and an induction coil disposed adjacent a wall or portion of a wall supporting the filter assembly and disposed on a side of the wall or wall portion opposite the filter assembly, wherein the induction coil, filter assembly, and wall or wall portion are constructed and arranged so that the induction coil effects inductive heating in the conductive mesh when the induction coil is energized.

Embodiment E2 is the system of embodiment E1, comprising a filter chamber that is distinct from the sample reservoir and includes a fluid inlet and a fluid outlet, wherein the inductively heatable filter assembly is disposed within the filter chamber.

Embodiment E3 is the system of embodiment E2, further comprising: a pump chamber; a first channel extending between the pump chamber and the sample reservoir; a first flow control valve for selectively connecting the pump chamber to the sample reservoir via the first channel; a second channel extending between the pump chamber and the filter chamber; and a second flow control valve for selectively connecting the pump chamber to the filter chamber via the second channel.

Embodiment E4 is the system of embodiment E3, further comprising: one or more process fluid reservoirs; an associated process fluid channel extending between each of the one or more process fluid reservoirs and the pump chamber; and an associated process fluid flow control valve for selectively connecting the pump chamber to the filter chamber via the associated process fluid channel.

Embodiment E5 is the system of any one of embodiments E3 or E4, further comprising a syringe plunger disposed within the pump chamber and configured to be actuated to draw fluid into the pump chamber or to expel fluid from the pump chamber.

Embodiment E6 is the system of any one of embodiments E1 - E5, wherein the sample reservoir comprises a sample well for receiving a sample container and a hole within the formed in a wall of the sample well for connecting an opening in the sample container to the fluid flow network.

Embodiment E7 is the system of embodiment E4, wherein the one or more process fluid reservoirs include at least one process fluid reservoir containing a lysing buffer and/or at least one process fluid reservoir containing an elution buffer.

Embodiment E8 is the system of embodiment E1, wherein the inductively heatable filter assembly is disposed within the sample reservoir.

Embodiment E9 is the system of embodiment E8, further comprising a fluid outlet extending from the sample reservoir through which sample fluid flowing from the sample reservoir passes, and wherein the inductively heatable filter assembly is positioned with respect to the fluid outlet so that sample fluid flowing from the sample reservoir into the fluid outlet flows through the filter assembly.

Embodiment E10 is the system of any one of embodiments E1, E8, and E9, further comprising: one or more process fluid reservoirs; an associated process fluid channel extending between each of the one or more process fluid reservoirs and the sample reservoir; and an associated process fluid flow control valve for selectively connecting each process fluid reservoir to the sample reservoir.

Embodiment E11 is the system of any one of embodiments E1 and E8 - E10, further comprising a source of pressure for effecting fluid flow from the sample reservoir and through the filter assembly.

Embodiment E12 is the system of embodiment E10, wherein each process fluid flow control valve comprises a recess formed in an outer surface of a body with a through hole formed in a bottom of the recess, and a flexible material extended over the recess, wherein the flexible material is deformable under the force of an external actuator into the recess to block the through hole.

Embodiment E13 is the system of any one of embodiments E1 - E12, wherein the filter media comprises one or more filter elements.

Embodiment E14 is the system of any one of embodiments E1 - E13, wherein the filter media comprises a filter membrane.

Embodiment E15 is the system of any one of embodiments E1 - E14, wherein the conductive mesh is comprised of a metal or combination of metals.

Embodiment E16 is the system of embodiment E15, wherein the conductive mesh is comprised of iron, copper, stainless steel, nickel, and/or aluminum.

Embodiment E17 is the system of any one of embodiments E1 - E16, wherein the induction coil is made of copper.

Embodiment E18 is the system of any one of embodiments E1 - E17, wherein the induction coil is spaced from the conductive mesh by about 1.5 mm to 4.0 mm.

Embodiment E19 is the system of embodiment E18, wherein the induction coil is spaced from the conductive mesh by about 3.0 mm.

Embodiment E20 is the system of any one of embodiments E1 - E19, wherein the induction coil comprises a flat coil.

Embodiment E21 is the system of any one of embodiments E1 - E20, wherein the conductive mesh overlies or underlies the filter media or wherein the conductive mesh overlies and underlies the filter media.

Embodiment E22 is the system of any one of embodiments E1 - E21, wherein at least part of the conductive mesh is in contact with at least part of the filter media.

Embodiment E23 is the system of any one of embodiments E1 - E22, wherein the filter media and the conductive mesh are flat.

Embodiment E24 is the system of embodiment E23, wherein the filter media and the conductive mesh are generally parallel to each other.

Embodiment E25 is the system of any one of embodiments E1 - E24, wherein the filter media and the conductive mesh have the same general shape.

Embodiment E26 is the system of any one of embodiments E1 - E25, wherein the thickness of the conductive mesh ranges from 130 µm-350 µm.

Embodiment E27 is the system of any one of embodiments E1 - E26, wherein the conductive mesh has openings of about 60 µm.

Embodiment E28 is the system of any one of embodiments E1 - E27, wherein the conductive mesh includes portions contacting opposed sides of the filter media.

Embodiment E29 is the system of any one of embodiments E1 -E28, wherein the conductive mesh comprises a woven wire mesh.

Embodiment E30 is the system of embodiment E29, wherein the woven wire mesh has openings of about 40 µm to about 300 µm.

Embodiment E31 is the system of any one of embodiments E1 - E27, wherein the conductive mesh comprises sintered fibers.

Embodiment E32 is the system of embodiment E31, wherein gaps between the sintered fibers define openings of about 3 µm to 100 µm.

Embodiment E33 is the system of any one of embodiments E1 - E28, wherein the conductive mesh comprises a perforated plate.

Embodiment E34 is the system of embodiment E33, wherein openings formed through the perforated pate each have a maximum width ranging from about 0.04 mm to about 0.3 mm.

Embodiment E35 is the system of embodiment E34, wherein the openings are spaced apart from each other by a distance ranging from about 0.16 mm to about 0.6 mm.

Embodiment E36 is the system of any one of embodiments E1 - E35, wherein the filter media comprises a porous substrate and optionally comprises a sponge.

Embodiment E37 is the system of embodiment E36, wherein the porous substrate is made of polyethersulfone, cellulose, nylon, polyvinylidene fluoride (Poly(1,1-difluoroethylene), PVDF), polycarbonate, or glass fiber.

Embodiment E38 is the system of embodiment E36 or E37, wherein the porous substrate includes pores with a size of between about 0.1 µm and about 1.0 µm.

Embodiment E39 is the system of any one of embodiments E36 - E38, wherein the porous substrate has a thickness of 160 µm to 185 µm.

Embodiment E40 is the system of any one of embodiments E36 - E39, wherein the porous substrate is positioned and arranged within the filter chamber so that the sample flows through the porous substrate from a first side of the porous substrate to a second side of the porous substrate, and wherein the porous substrate includes pores decreasing in size from the first side to the second side.

Embodiment E41 is the system of embodiment E40, wherein the porous substrate comprises multiple layers of decreasing pore size progressing from the first side to the second side.

Embodiment E42 is the system of embodiment E41, wherein the porous substrate comprises two layers.

Embodiment E43 is the system of any one of embodiments E40 - E42, wherein the first side includes pores with a size of between about 5 µm and about 20 µm, and wherein the second side includes pores with a size of up to about 0.2 µm.

Embodiment E44 is the system of any one of embodiments E1 - E35, wherein the filter media comprises a track etched membrane.

Embodiment E45 is the system of embodiment E44, wherein the filter media further comprises a layer of sintered plastic particles.

Embodiment E46 is the system of embodiment E45, wherein the plastic particles are made of polyethylene, polypropylene, polytetrafluoroethylene, or polyvinyllidene fluoride.

Embodiment E47 is the system of any one of embodiments E44 - E46, wherein the track etched membrane has pores of up to about 0.2 µm in size.

Embodiment E48 is the system of any one of embodiments E44 - E47, wherein the track etched membrane has a thickness of about 22 µm.

Embodiment E49 is the system of embodiment E45 or E46, wherein interstices between the sintered plastic particles range in size from about 5 µm to about 15 µm.

Embodiment E50 is the system of any one of embodiments E45, E46, and E49, wherein the layer of sintered plastic particles has a thickness of about 450 µm to 650 µm.

Embodiment E51 is the system of any one of embodiments E1 - E50, further comprising a power source connected to the induction coil and configured to generate an alternating current in the induction coil so that the induction coil generates alternating electromagnetic fields.

Embodiment E52 is the system of any one of embodiments E1 -E51, further comprising a temperature sensor configured to measure a temperature of the conductive mesh.

Embodiment E53 is the system of embodiment E52, wherein the temperature sensor comprises a contactless temperature sensor.

Embodiment E54 is the system of embodiment E52 or E53, wherein the temperature sensor comprises an infrared temperature sensor.

Embodiment E55 is the system of any one of embodiments E1 - E50, further comprising: a power source connected to the induction coil and configured to generate alternating current in the induction coil; a temperature sensor configured to measure a temperature of the conductive mesh; and a temperature feedback control circuit connected to the power source and the temperature sensor and configured to control the alternating current generated by the power source based on temperature measured by the temperature sensor.

Embodiment E56 is the system of embodiment E55, wherein the temperature sensor comprises a contactless temperature sensor.

Embodiment E57 is the system of embodiment E55 or E56, wherein the temperature sensor comprises an infrared temperature sensor.

Embodiment E58 is the system of any one of embodiments E1 - E57, further comprising a syringe coupled to the fluid inlet of the filter chamber and comprising a syringe chamber and a plunger disposed within the syringe chamber, wherein the plunger is configured to be actuated to propel fluid from the syringe chamber into the filter chamber through the fluid inlet.

Embodiment F1 is fluid cartridge comprising: a sample port for introducing a sample to the fluid cartridge; one or more process fluid reservoirs; a waste chamber; a filter chamber with a fluid inlet and a fluid outlet; a first outlet channel extending between the fluid outlet of the filter chamber and the waste chamber; a second outlet channel extending from the fluid outlet of the filter chamber; an inductively heatable filter assembly disposed within the filter chamber and comprising: filter media, wherein the filter media includes pores or interstices that are sized so as to prevent the passage of at least some cells contained in the sample to thereby separate cells from a fluid component of the sample flowing through the filter media; and a conductive mesh disposed adjacent to the filter media, wherein the conductive mesh is configured to permit fluid flow therethrough, and wherein a wall defining the filter chamber, or a portion of such wall, is configured to permit the conductive mesh to be in sufficient proximity to an energized induction coil disposed outside the filter chamber adjacent the wall or wall portion so that the energized induction coil effects inductive heating in the conductive mesh; a syringe barrel; and a fluid flow network comprising fluid channels and flow control valves for selectively permitting fluid flow: (i) from the sample port to the syringe barrel, (ii) from the syringe barrel to the fluid inlet of the filter chamber and from the fluid outlet of the filter chamber to the waste chamber via the first outlet channel, (iii) from at least one of the one or more process fluid reservoirs to the syringe barrel, and (iv) from the syringe barrel to the fluid inlet of the filter chamber and from the fluid outlet of the filter chamber into the second outlet channel.

Embodiment F2 is the fluid cartridge of embodiment F1, further comprising a syringe comprising a plunger having a plunger seal disposed within the syringe barrel.

Embodiment F3 is the fluid cartridge of embodiment F1 or F2, wherein the fluid cartridge is a microfluidic device.

Embodiment F4 is the fluid cartridge of any one of embodiments F1 - F3, wherein the fluid cartridge further comprises one or more chambers containing dried reagents.

Embodiment F5 is the fluid cartridge of any one of embodiments F1 - F4, further comprising a sample container well for coupling a sample receptacle to the fluid cartridge, wherein the sample container well is in fluid communication with the sample port.

Embodiment F6 is the fluid cartridge of embodiment F5, further comprising a sample receptacle coupled to the sample container well.

Embodiment F7 is the fluid cartridge of any one of embodiments F1 - F4, further comprising a sample reservoir for receiving a sample, wherein the sample reservoir is in fluid communication with the sample port.

Embodiment F8 is the fluid cartridge of any one of embodiments F1 - F7, wherein the fluid cartridge comprises a body made from plastic.

Embodiment F9 is the fluid cartridge of embodiment F8, wherein the body is made from polypropylene, polycarbonate, polyacrylamide, polyethylene, polymethyl-methacrylate (PMMA), polydimetyl-siloxane (PDMS), or polyvinyl chloride (PVC).

Embodiment F10 is the fluid cartridge of embodiment F8 or F9, wherein each flow control valve comprises: a recess formed in an outer surface of the body, with a through hole formed in a bottom of the recess and thereby defining an annular valve seat surrounding the through hole in the bottom of the recess; and a flexible material extended over the recess, wherein the flexible material is deformable under the force of an external actuator into contact with the valve seat to obstruct the through hole.

Embodiment F11 is the fluid cartridge of any one of embodiments F1 - F10, wherein the filter media is affixed to the filter chamber by ultrasonic welding.

Embodiment F12 is the fluid cartridge of any one of embodiments F1 - F11, wherein the filter media comprises one or more filter elements.

Embodiment F13 is the fluid cartridge of any one of embodiments F1 - F12, wherein the filter media comprises a filter membrane.

Embodiment F14 is the fluid cartridge of any one of embodiments F1 - F13, further comprising a fluid discharge port in communication with the second outlet channel through which fluid is discharged from the fluid cartridge.

Embodiment F15 is the fluid cartridge of any one of embodiments F1 -F14, wherein the conductive mesh is comprised of a metal or combination of metals.

Embodiment F16 is the fluid cartridge of any one of embodiments F1 - F15, wherein the conductive mesh is affixed within the filter chamber by heat stake welding.

Embodiment F17 is the fluid cartridge of embodiment F15 or F16, wherein the conductive mesh is comprised of iron, copper, stainless steel, nickel, and/or aluminum.

Embodiment F18 is the fluid cartridge of any one of embodiments F1 - F17, wherein the induction coil is spaced from the conductive mesh by about 1.5 mm to 4.0 mm.

Embodiment F19 is the fluid cartridge of embodiments F18, wherein the induction coil is spaced from the conductive mesh by about 3.0 mm.

Embodiment F20 is the fluid cartridge of any one of embodiments F1 - F19, wherein the induction coil comprises a flat coil.

Embodiment F21 is the fluid cartridge of any one of embodiments F1 - F20, wherein the conductive mesh overlies or underlies the filter media or wherein the conductive mesh overlies and underlies the filter media.

Embodiment F22 is the fluid cartridge of any one of embodiments F1 - F21, wherein at least part of the conductive mesh is in contact with at least part of the filter media.

Embodiment F23 is the fluid cartridge of any one of embodiments F1 - F22, wherein the filter media and the conductive mesh are flat.

Embodiment F24 is the fluid cartridge of embodiment F23, wherein the filter media and the conductive mesh are generally parallel to each other.

Embodiment F25 is the fluid cartridge of any one of embodiments F1 - F24, wherein the filter media and the conductive mesh have the same general shape.

Embodiment F26 is the fluid cartridge of any one of embodiments F1 - F25, wherein the thickness of the conductive mesh ranges from 130 µm-350 µm.

Embodiment F27 is the fluid cartridge of any one of embodiments F1 - F26, wherein the conductive mesh includes portions contacting opposed sides of the filter media.

Embodiment F28 is the fluid cartridge of any one of embodiments F1 - F22, wherein the filter media has a generally cylindrical configuration and the conductive mesh has a generally cylindrical configuration arranged coaxially with the filter media.

Embodiment F29 is the fluid cartridge of embodiment F28, wherein the conductive mesh is positioned radially outwardly of the filter media so that the conductive mesh at least partially encompasses the filter media.

Embodiment F30 is the fluid cartridge of embodiment F28 or F29, comprising a plenum positioned within the filter chamber to receive fluid at the fluid inlet and introduce the fluid into the filter chamber at a location that is radially inward of the filter media, wherein the fluid flows radially outwardly through the filter media and the conductive mesh, and wherein the fluid outlet is located radially outwardly of the conductive mesh.

Embodiment F31 is the fluid cartridge of any one of embodiments F28 - F30, wherein the filter media is pleated.

Embodiment F32 is the fluid cartridge of any one of embodiments F1 -F31, wherein the conductive mesh comprises a woven wire mesh.

Embodiment F33 is the fluid cartridge of embodiment F32, wherein the woven wire mesh has openings of about 40 µm to about 300 µm.

Embodiment F34 is the fluid cartridge of any one of embodiments F1 -F31, wherein the conductive mesh comprises sintered fibers.

Embodiment F35 is the fluid cartridge of embodiment F34, wherein gaps between the sintered fibers define openings of about 3 µm to 100 µm.

Embodiment F36 is the fluid cartridge of any one of embodiments F1 -F31, wherein the conductive mesh comprises a perforated plate.

Embodiment F37 is the fluid cartridge of embodiment F36, wherein openings formed through the perforated plate each have a maximum width ranging from about 0.04 mm to about 0.3 mm.

Embodiment F38 is the fluid cartridge of embodiment F37, wherein the openings formed through the perforated plate are spaced apart from each other by a distance ranging from about 0.16 mm to about 0.6 mm.

Embodiment F39 is the fluid cartridge of any one of embodiments F1 - F38, wherein the filter media comprises a porous substrate and optionally comprises a sponge.

Embodiment F40 is the fluid cartridge of embodiment F39, wherein the porous substrate is made of polyethersulfone, cellulose, nylon, polyvinylidene fluoride (Poly(1,1-difluoroethylene), PVDF), polycarbonate, or glass fiber.

Embodiment F41 is the fluid cartridge of embodiment F39 or F40, wherein the porous substrate includes pores with a size of between about 0.1 µm and about 1.0 µm.

Embodiment F42 is the fluid cartridge of any one of embodiments F39 - F41, wherein the porous substrate has a thickness of 160 µm to 185 µm.

Embodiment F43 is the fluid cartridge of any one of embodiments F39 - F42, wherein the porous substrate is positioned and arranged within the filter chamber so that the sample flows through the porous substrate from a first side of the porous substrate to a second side of the porous substrate, and wherein the porous substrate includes pores decreasing in size from the first side of the porous substrate to the second side of the porous substrate.

Embodiment F44 is the fluid cartridge of embodiment F43, wherein the porous substrate comprises multiple layers of decreasing pore size progressing from the first side of the porous substrate to the second side of the porous substrate.

Embodiment F45 is the fluid cartridge of embodiment F44, wherein the porous substrate comprises two layers.

Embodiment F46 is the fluid cartridge of any one of embodiments F43 - F45, wherein the first side of the porous substrate includes pores with a size of between about 5 µm and about 20 µm, and wherein the second side of the porous substrate includes pores with a size of up to about 0.2 µm in diameter.

Embodiment F47 is the fluid cartridge of any one of embodiments F1 - F38, wherein the filter media comprises a track etched membrane.

Embodiment F48 is the fluid cartridge of embodiment F47, wherein the filter media further comprises a layer of sintered plastic particles.

Embodiment F49 is the fluid cartridge of embodiment F48, wherein the plastic particles are made of polyethylene, polypropylene, polytetrafluoroethylene, or polyvinyllidene fluoride.

Embodiment F50 is the fluid cartridge of any one of embodiments F47 - F49, wherein the track etched membrane has pores of up to about 0.2 µm in size.

Embodiment F51 is the fluid cartridge of any one of embodiments F47 - F50, wherein the track etched membrane has a thickness of about 22 µm.

Embodiment F52 is the fluid cartridge of embodiment F48 or F49, wherein interstices between the sintered plastic particles range in size from about 5 µm to about 15 µm.

Embodiment F53 is the fluid cartridge of any one of embodiments F48, F49, and F52, wherein the layer of sintered plastic particles has a thickness of about 450 µm to 650 µm.

Embodiment G1 is a fluid cartridge comprising: a sample port for introducing a sample to the fluid cartridge; one or more process fluid reservoirs; a waste chamber; a filter chamber with a fluid inlet and a fluid outlet; a first outlet channel extending between the fluid outlet of the filter chamber and the waste chamber; a second outlet channel extending from the fluid outlet of the filter chamber; a filter assembly disposed within the filter chamber and comprising filter media, wherein the filter media includes pores or interstices that are sized so as to prevent the passage of at least some cells contained in a sample to thereby separate cells from a fluid component of the sample flowing through the filter media; a syringe barrel; and a fluid flow network comprising fluid channels and flow control valves for selectively permitting fluid flow: (i) from the sample port to the syringe barrel, (ii) from the syringe barrel to the fluid inlet of the filter chamber and from the fluid outlet of the filter chamber to the waste chamber via the first outlet channel, (iii) from at least one of the one or more process fluid reservoirs to the syringe barrel, and (iv) from the syringe barrel to the fluid inlet of the filter chamber and from the fluid outlet of the filter chamber into the second outlet channel.

Embodiment G2 is the fluid cartridge of embodiment G1, further comprising a syringe comprising a syringe plunger having a plunger seal disposed within the syringe barrel.

Embodiment G3 is the fluid cartridge of embodiment G1 or G2, wherein the fluid cartridge is a microfluidic device.

Embodiment G4 is the fluid cartridge of any one of embodiments G1 - G3, wherein the fluid cartridge further comprises one or more chambers containing dried reagents.

Embodiment G5 is the fluid cartridge of any one of embodiments G1 - G4, further comprising a sample container well for coupling a sample receptacle to the fluid cartridge, wherein the sample container well is in fluid communication with the sample port.

Embodiment G6 is the fluid cartridge of embodiment G5, further comprising a sample receptacle coupled to the sample container well.

Embodiment G7 is the fluid cartridge of any one of embodiments G1 - G4, further comprising a sample reservoir for receiving a fluid sample, wherein the sample reservoir is in fluid communication with the sample port.

Embodiment G8 is the fluid cartridge of any one of embodiments G1 - G7, comprising a body made from plastic.

Embodiment G9 is the fluid cartridge of embodiment G8, wherein the body is made from polypropylene, polycarbonate, polyacrylamide, polyethylene, polymethyl-methacrylate (PMMA), polydimetyl-siloxane (PDMS), or polyvinyl chloride (PVC).

Embodiment G10 is the fluid cartridge of embodiment G8 or G9, wherein each flow control valve comprises: a recess formed in an outer surface of the body, with a hole formed in a bottom of the recess and thereby defining an annular valve seat surrounding the hole in the bottom of the recess, and a flexible material extended over the recess, wherein the flexible material is deformable under the force of an external actuator into contact with the valve seat to obstruct fluid flow through the hole.

Embodiment G11 is the fluid cartridge of embodiment G10, wherein the flexible material is made of a thermoplastic.

Embodiment G12 is the fluid cartridge of embodiment G10 or G11, wherein the flexible material is made of polypropylene.

Embodiment G13 is the fluid cartridge of any one of embodiments G1 - G12, wherein the filter media is affixed within the filter chamber by ultrasonic welding.

Embodiment G14 is the fluid cartridge of any one of embodiments G1 - G13, wherein the filter media comprises one or more filter elements.

Embodiment G15 is the fluid cartridge of any one of embodiments G1 - G14, wherein the filter media comprises a filter membrane.

Embodiment G16 is the fluid cartridge of any one of embodiments G1 - G15, further comprising a fluid discharge port in communication with the first outlet channel through which fluid is discharged from the fluid cartridge.

Embodiment G17 is the fluid cartridge of any one of embodiments G1 - G16, wherein the filter media comprises a porous substrate and optionally comprises a sponge.

Embodiment G18 is the fluid cartridge of embodiment G17, wherein the porous substrate is made of polyethersulfone, cellulose, nylon, polyvinylidene fluoride (Poly(1,1-difluoroethylene), PVDF), polycarbonate, or glass fiber.

Embodiment G19 is the fluid cartridge of embodiment G17 or G18, wherein the porous substrate includes pores with a size of between about 0.1 µm and about 1.0 µm.

Embodiment G20 is the fluid cartridge of any one of embodiments G17 - G19, wherein the porous substrate has a thickness of 160 µm to 185 µm.

Embodiment G21 is the fluid cartridge of any one of embodiments G17 - G20, wherein the porous substrate is positioned and arranged within the filter chamber so that the sample flows through the porous substrate from a first side of the porous substrate to a second side of the porous substrate, and wherein the porous substrate includes pores decreasing in size from the first side of the porous substrate to the second side of the porous substrate.

Embodiment G22 is the fluid cartridge of embodiment G21, wherein the porous substrate comprises multiple layers of decreasing pore size progressing from the first side to the second side.

Embodiment G23 is the fluid cartridge of embodiment G22, wherein the porous substrate comprises two layers.

Embodiment G24 is the fluid cartridge of any one of embodiments G21 - G23, wherein the first side of the porous substrate includes pores with a size of betweeen about 5 µm and about 20 µm, and wherein the second side of the porous substrate includes pores with a size of up to about 0.2 µm in diameter.

Embodiment G25 is the fluid cartridge of any one of embodiments G1 - G16, wherein the filter media comprises a track etched membrane.

Embodiment G26 is the fluid cartridge of embodiment G25, wherein the filter media further comprises a layer of sintered plastic particles.

Embodiment G27 is the fluid cartridge of embodiment G26, wherein the plastic particles are made of polyethylene, polypropylene, polytetrafluoroethylene, or polyvinyllidene fluoride.

Embodiment G28 is the fluid cartridge of any one of embodiments G25 - G27, wherein the track etched membrane has pores of about 0.2 µm in size.

Embodiment G29 is the fluid cartridge of any one of embodiments G25 - G28, wherein the track etched membrane has a thickness of about 22 µm.

Embodiment G30 is the fluid cartridge of embodiments G26 or G27, wherein interstices between the sintered plastic particles range in size from about 5 µm to about 15 µm.

Embodiment G31 is the fluid cartridge of any one of embodiments G26, G27, and G29, wherein the layer of sintered plastic particles has a thickness of about 450 µm to 650 µm.

Embodiment G32 is the fluid cartridge of any one of embodiments G1 - G30, wherein the filter chamber is defined by a filter housing.

Embodiment G33 is the fluid cartridge of any one of embodiments G1 - G32, wherein the fluid inlet is disposed at the top of the filter chamber and the fluid outlet is disposed at the bottom of the filter chamber.

Embodiment H1 is a nucleic acid analysis system comprising: a sample port for introducing a sample to the system; one or more process fluid reservoirs; at least one waste chamber; a filter chamber with a fluid inlet and a fluid outlet; a filter assembly disposed within the filter chamber and comprising filter media, wherein the filter media includes pores or interstices that are sized so as to prevent the passage of at least some cells contained in a sample to thereby separate cells from a fluid component of the sample flowing through the filter media; a first syringe barrel; a first fluidic network comprising one or more fluid channels and one or more flow control valves for selectively permitting fluid flow: (i) from the sample port to the first syringe barrel, (ii) from the first syringe barrel to the fluid inlet of the filter chamber and the from fluid outlet of the filter chamber to the at least one waste chamber, (iii) from at least one of the one or more process fluid reservoirs to the first syringe barrel, and (iv) from the first syringe barrel to the fluid inlet of the filter chamber but not from the fluid outlet of the filter chamber to the at least one waste chamber; and an amplification chamber in which a nucleic acid amplification reaction proceeds, wherein the amplification chamber is directly or indirectly connected or connectable to the fluid outlet of the filter chamber.

Embodiment H2 is the nucleic acid analysis system of embodiment H1, further comprising a first syringe comprising a plunger having a plunger seal disposed within the first syringe barrel.

Embodiment H3 is the nucleic acid analysis system of embodiment H1 or H2, further comprising: an analysis chamber in which a process for detecting a nucleic acid occurs, wherein the analysis chamber is directly or indirectly connected or connectable to the amplification chamber; and a second fluidic network comprising one or more fluid channels and one or more flow control valves for selectively permitting fluid flow: (i) from the fluid outlet of the filter chamber directly or indirectly to the amplification chamber, and (ii) from the amplification chamber directly or indirectly to the analysis chamber.

Embodiment H4 is the nucleic acid analysis system of embodiment H3, further comprising a sample preparation chamber, wherein: the sample preparation chamber is directly or indirectly connected or connectable to the fluid outlet of the filter chamber to receive sample material that has passed through the filter assembly within the filter chamber, the amplification chamber is directly or indirectly connected or connectable to the sample preparation chamber, and the second fluidic network is configured to selectively permit fluid flow: (i) from the fluid outlet of the filter chamber to the sample preparation chamber and (ii) from the sample preparation chamber directly or indirectly to the amplification chamber.

Embodiment H5 is the nucleic acid analysis system of embodiment H4, further comprising a second syringe barrel, wherein the second fluidic network is configured to selectively permit fluid flow: (i) from the sample preparation chamber to the second syringe barrel, (ii) from the second syringe barrel to the amplification chamber, (iii) from the amplification chamber to the second syringe barrel, and (iv) from the second syringe barrel to the analysis chamber.

Embodiment H6 is the nucleic acid analysis system of embodiment H5, further comprising a second syringe comprising a plunger having a plunger seal disposed within the second syringe barrel.

Embodiment H7 is the nucleic acid analysis system of any one of embodiments H1 - H6, wherein the sample port, the one or more process fluid reservoirs, the filter chamber, the filter assembly, and the first syringe barrel are disposed within a first fluid cartridge, and the amplification chamber is disposed within a second fluid cartridge operatively coupled to the first fluid cartridge.

Embodiment H8 is the nucleic acid analysis system of any one of embodiments H4 - H6, wherein the sample preparation chamber is directly or indirectly connected to the at least one waste chamber.

Embodiment H9 is the nucleic acid analysis system of embodiment H8, wherein the at least one waste chamber comprises a first waste chamber and a second waste chamber, wherein the fluid outlet of the filter chamber is connected by at least one channel of the first fluidic network to the first waste chamber, and wherein the sample preparation chamber is directly or indirectly connected by at least one channel of the second fluidic network to the second waste chamber.

Embodiment H10 is the nucleic acid analysis system of any one of embodiments H4, H5, H6, H8, or H9, wherein the sample preparation chamber comprises a purification column.

Embodiment H11 is the nucleic acid analysis system of any one of embodiments H4, H5, H8, H9, or H10, further comprising: a binding substrate contained within or provided to the sample preparation chamber for selective binding to at least one nucleic acid of interest; and at least one wash buffer chamber containing a wash buffer for washing nucleic acid bound to the binding substrate, wherein the second fluidic network is configured to selectively permit fluid flow from the at least one wash buffer chamber directly or indirectly to the sample preparation chamber.

Embodiment H12 is the nucleic acid analysis system of embodiment H11, wherein the binding substrate comprises a gel, beads, or a paper filter.

Embodiment H13 is the nucleic acid analysis system of embodiment H11 or H12, wherein the binding substrate comprises agarose gel, silica beads or filter cellulose paper.

Embodiment H14 is the nucleic acid analysis system of embodiment H13, further comprising an elution buffer chamber containing an elution buffer for eluting nucleic acid bound to the binding substrate, wherein the second fluidic network is configured to selectively permit fluid flow from the elution buffer chamber directly or indirectly to the sample preparation chamber.

Embodiment H15 is the nucleic acid analysis system of any one of embodiments H4, H5, H6, H8 or H14, further comprising an amplification reagent chamber containing an amplification reagent for amplifying a nucleic acid bound to the binding substrate, wherein the second fluidic network is configured to selectively permit fluid flow from the amplification reagent chamber to the sample preparation chamber or the amplification chamber.

Embodiment H16 is the nucleic acid analysis system of any one of embodiments H3 - H6, further comprising a nucleic acid array within the analysis chamber, wherein the nucleic acid array comprises probe molecules immobilized on a surface thereof, wherein the probe molecules have a sequence specific to a nucleic acid of interest.

Embodiment H17 is the nucleic acid analysis system of any one of embodiments H1 - H16, further comprising a sample container well for coupling to a sample receptacle, wherein the sample container well is in fluid communication with the sample port.

Embodiment H18 is the nucleic acid analysis system of embodiment H17, further comprising a sample receptacle coupled to the sample container well.

Embodiment H19 is the nucleic acid analysis system of any one of embodiments H1 - H16, further comprising a sample reservoir for receiving the sample, wherein the sample reservoir is in fluid communication with the sample port.

Embodiment H20 is the nucleic acid analysis system of any one of embodiments H1 - H19, comprising a cartridge body made from plastic.

Embodiment H21 is the nucleic acid analysis system of embodiment H20, wherein each flow control valve comprises: a recess formed in an outer surface of the cartridge body, with a hole formed in a bottom of the recess and thereby defining an annular valve seat surrounding the hole in the bottom of the recess, and a flexible material extended over the recess, wherein the flexible material is deformable under the force of an external actuator into contact with the valve seat to obstruct fluid flow through the hole.

Embodiment H22 is the nucleic acid analysis system of embodiment H20 or H21, wherein the cartridge body is made from polypropylene, polycarbonate, polyacrylamide, polyethylene, polymethyl-methacrylate (PMMA), polydimetyl-siloxane (PDMS), or polyvinyl chloride (PVC).

Embodiment H23 is the nucleic acid analysis system of any one of embodiments H1 - H22, wherein the filter media comprises a porous substrate and optionally comprises a sponge.

Embodiment H24 is the nucleic acid analysis system of embodiment H23, wherein the porous substrate is made of polyethersulfone, cellulose, nylon, polyvinylidene fluoride (Poly(1,1-difluoroethylene), PVDF), polycarbonate, or glass fiber.

Embodiment H25 is the nucleic acid analysis system of embodiment H23 or H24, wherein the porous substrate includes pores with a size of between about 0.1 µm and about 1.0 µm m.

Embodiment H26 is the nucleic acid analysis system of any one of embodiments H23 - H25, wherein the porous substrate is positioned and arranged within the filter chamber so that the sample flows through the porous substrate from a first side of the porous substrate to a second side of the porous substrate, and wherein the porous substrate includes pores decreasing in size from the first side of the porous substrate to the second side of the porous substrate.

Embodiment H27 is the nucleic acid analysis system of embodiment H26, wherein the porous substrate comprises multiple layers of decreasing pore size progressing from the first side of the porous substrate to the second side of the porous substrate.

Embodiment H28 is the nucleic acid analysis system of embodiment H26 or H27, wherein the first side of the porous substrate includes pores with a size of between about 5 µm and about 20 µm, and wherein the second side porous substrate includes pores with a size of up to about 0.2 µm in diameter.

Embodiment H29 is the nucleic acid analysis system of any one of embodiments H1 - H22, wherein the filter media comprises a track etched membrane.

Embodiment H30 is the nucleic acid analysis system of embodiment H29, wherein the filter media further comprises a layer of sintered plastic particles.

Embodiment H31 is the nucleic acid analysis system of embodiment H30, wherein the plastic particles are made of polyethylene, polypropylene, polytetrafluoroethylene, or polyvinyllidene fluoride.

Embodiment H32 is the nucleic acid analysis system of any one of embodiments H29 - H31, wherein the track etched membrane has pores of about 0.2 µm in size.

Embodiment H33 is the nucleic acid analysis system of embodiment H30 or H31, wherein interstices between the sintered plastic particles range in size from about 5 µm to about 15 µm.

Embodiment H34 is the nucleic acid analysis system of any one of embodiments H1 - H33, wherein the filter assembly comprises a conductive mesh disposed within the filter chamber adjacent to the filter media, wherein the conductive mesh is configured to permit fluid flow therethrough, and wherein a wall defining the filter chamber, or a portion of such wall, is configured to permit the conductive mesh to be in sufficient proximity to an energized induction coil disposed outside the filter chamber adjacent the wall or wall portion so that the energized induction coil effects inductive heating in the conductive mesh to lyse at least a portion of the cells separated from the fluid component of the sample by the filter media.

Embodiment H35 is the nucleic acid analysis system of embodiment H34, wherein the filter chamber comprise a first wall and a second wall, wherein the fluid inlet extends through the first wall, and the fluid outlet extends through the second wall.

Embodiment H36 is the nucleic acid analysis system of embodiment H34 or H35, wherein the conductive mesh is comprised of a metal or combination of metals.

Embodiment H37 is the nucleic acid analysis system of embodiment H36, wherein the conductive mesh is comprised of iron, copper, stainless steel, nickel, and/or aluminum.

Embodiment H38 is the nucleic acid analysis system of any one of embodiments H34 - H37, further comprising an induction coil disposed outside the filter chamber adjacent the wall or wall portion so that, when energized, the induction coil effects inductive heating in the conductive mesh to lyse at least a portion of the cells separated from the fluid component of the sample by the filter media.

Embodiment H39 is the nucleic acid analysis system of embodiment H38, wherein the induction coil at least partially overlies or underlies the conductive mesh.

Embodiment H40 is the nucleic acid analysis system of any one of embodiments H38 or H39, wherein the induction coil is made of copper.

Embodiment H41 is the nucleic acid analysis system of any one of embodiments H38 - H40, wherein the induction coil is spaced from the conductive mesh by about 1.5-4.0 mm.

Embodiment H42 is the nucleic acid analysis system of any one of embodiments H38 - H41, wherein the induction coil comprises a flat coil.

Embodiment H43 is the nucleic acid analysis system of any one of embodiments H34 - H42, wherein the conductive mesh overlies or underlies the filter media or wherein the conductive mesh overlies and underlies the filter media.

Embodiment H44 is the nucleic acid analysis system of any one of embodiments H34 - H43, wherein at least part of the conductive mesh is in contact with at least part of the filter media.

Embodiment H45 is the nucleic acid analysis system of any one of embodiments H34 - H44, wherein the filter media and the conductive mesh are flat.

Embodiment H46 is the nucleic acid analysis system of embodiment H45, wherein the filter media and the conductive mesh are generally parallel to each other.

Embodiment H47 is the nucleic acid analysis system of any one of embodiments H34 - H46, wherein the filter media and the conductive mesh have the same general shape.

Embodiment H48 is the nucleic acid analysis system of any one of embodiments H34 - H47, wherein the thickness of the conductive mesh ranges from 130 µm-350 µm.

Embodiment H49 is the nucleic acid analysis system of any one of embodiments H34 - H48, wherein the conductive mesh includes portions contacting opposed sides of the filter media.

Embodiment H50 is the nucleic acid analysis system of any one of embodiments H34 - H49, wherein the filter media has a generally cylindrical configuration and the conductive mesh has a generally cylindrical configuration arranged coaxially with the filter media.

Embodiment H51 is the nucleic acid analysis system of embodiment H50, wherein the conductive mesh is positioned radially outwardly of the filter media so that the conductive mesh at least partially encompasses the filter media.

Embodiment H52 is the nucleic acid analysis system of embodiment H50 or H51, comprising a plenum positioned within the filter chamber to receive fluid at the fluid inlet and introduce the fluid into the filter chamber at a radially inward portion of the filter media, wherein the fluid flows radially outwardly through the filter media and the conductive mesh, and wherein the fluid outlet is located radially outwardly of the conductive mesh.

Embodiment H53 is the nucleic acid analysis system of any one of embodiments H50 - H52, wherein the filter media is pleated.

Embodiment H54 is the nucleic acid analysis system of any one of embodiments H34 - H53, wherein the conductive mesh comprises a woven wire mesh.

Embodiment H55 is the nucleic acid analysis system of embodiment H54, wherein the woven wire mesh has openings of about 40 µm to about 300 µm.

Embodiment H56 is the nucleic acid analysis system of any one of embodiments H34 - H53, wherein the conductive mesh comprises sintered fibers.

Embodiment H57 is the nucleic acid analysis system of embodiment H56, wherein gaps between the sintered fibers define openings of about 3 µm to 100 µm.

Embodiment H58 is the nucleic acid analysis system of any one of embodiments H34 - H53, wherein the conductive mesh comprises a perforated plate.

Embodiment H59 is the nucleic acid analysis system of embodiment H58, wherein openings formed through the perforated plate each having a maximum width ranging from about 0.04 mm to about 0.3 mm.

Embodiment H60 is the nucleic acid analysis system of embodiment H59, wherein the openings formed through the perforated plate are spaced apart from each other by a distance of about 0.16 mm to about 0.6 mm.

Embodiment H61 is the nucleic acid analysis system of any one of embodiments H34 - H60, further comprising a temperature sensor configured to measure a temperature of the conductive mesh.

Embodiment H62 is the nucleic acid analysis system of embodiment H61, wherein the temperature sensor comprises a contactless temperature sensor.

Embodiment H63 is the nucleic acid analysis system of embodiment H55 or H62, wherein the temperature sensor comprises an infrared temperature sensor.

Embodiment H64 is the nucleic acid analysis system of any one of embodiments H38 - H42, further comprising a power source connected to the induction coil and configured to generate an alternating current in the induction coil so that the induction coil generates alternating electromagnetic fields.

Embodiment H65 is the nucleic acid analysis system of embodiment H64, further comprising: a temperature sensor configured to measure a temperature of the conductive mesh; and a temperature feedback control circuit connected to the power source and the temperature sensor and configured to control alternating current generated by the power source based on temperature measured by the temperature sensor.

Embodiment H66 is the nucleic acid analysis system of embodiment H65, wherein the temperature sensor comprises a contactless temperature sensor.

Embodiment H67 is the nucleic acid analysis system of embodiment H65 or H66, wherein the temperature sensor comprises an infrared temperature sensor.

Embodiment I1 is a system for detecting at least one nucleic acid in a sample, the system comprising: a first plurality of functional chambers; a filter chamber having a fluid inlet and a fluid outlet; filter media disposed within the filter chamber, wherein the filter media includes pores or interstices that are sized so as to prevent the passage of at least some cells contained in a sample to thereby separate cells from a fluid component of the sample flowing through the filter media; a first distribution chamber; at least one waste chamber; a first network of fluid channels connecting each chamber of the first plurality of functional chambers to the first distribution chamber, connecting the first distribution chamber to the fluid inlet of the filter chamber, and connecting the fluid outlet of the filter chamber to the at least one waste chamber; and a first plurality of valves, one of the first plurality of valves being operatively associated with each respective channel of the first network of fluid channels connecting one of the first plurality of functional chambers to the first distribution chamber, one of the first plurality of valves being operatively associated with a respective channel of the first network of fluid channels connecting the first distribution chamber to the fluid inlet of the filter chamber, and one of the first plurality of valves being operatively associated with a respective channel of the first network of fluid channels connecting the fluid outlet of the filter chamber to the at least one waste chamber.

Embodiment I2 is the system of embodiment I1, further comprising: a second plurality of functional chambers; a second distribution chamber; a second network of fluid channels connecting one functional chamber of the second plurality of functional chambers to the fluid outlet of the filter chamber, connecting each chamber of the second plurality of functional chambers to the second distribution chamber, and connecting the second distribution chamber to the at least one waste chamber; and a second plurality of valves, one of the second plurality of valves being operatively associated with each respective channel of the second network of fluid channels connecting one of the second plurality of functional chambers to the second distribution chamber, one of the second plurality of valves being operatively associated with a respective channel of the second network of fluid channels connecting the one functional chamber of the second plurality of functional chambers to the fluid outlet of the filter chamber, and one of the second plurality of valves being operatively associated with a respective channel of the second network of fluid channels connecting the second distribution chamber to the at least one waste chamber.

Embodiment I3 is the system of embodiment I1 or I2, further comprising a conductive mesh disposed within the filter chamber adjacent to the filter media, wherein the conductive mesh is configured to permit fluid flow therethrough, and wherein a wall defining the filter chamber, or a portion of such wall, is configured to permit the conductive mesh to be in sufficient proximity to an energized induction coil disposed outside the filter chamber adjacent the wall or wall portion so that the energized induction coil effects inductive heating in the conductive mesh to lyse at least a portion of the cells separated from the fluid component of the sample by the filter media.

Embodiment I4 is the system of embodiment I3, further comprising an induction coil disposed outside the filter chamber adjacent the wall or wall portion so that, when energized, the induction coil effects inductive heating in the conductive mesh to lyse at least a portion of the cells separated from the fluid component of the sample by the filter media.

Other features and characteristics of the subject matter of this disclosure, as well as the methods of operation, functions of related elements of structure and the combination of parts, and economies of manufacture, will become more apparent upon consideration of the following description and the appended claims with reference to the accompanying drawings, all of which form a part of this specification, where like reference numerals designate corresponding parts in the various figures.

### BRIEF DESCRIPTION OF THE DRAWINGS

The accompanying drawings, which are incorporated herein and form part of the specification, illustrate various embodiments of the subject matter of this disclosure. In the drawings, like reference numbers indicate identical or functionally similar elements.
FIG. 1 is a perspective view of a system for separating cells from a sample and for lysing the separated cells, wherein the system is in position to be coupled to a reaction cartridge.
FIG. 2 is a top plan view of an embodiment of a sample fluid processing cartridge.
FIG. 3 is the top plan view of the sample fluid processing cartridge.
FIG. 4 is a top plan view of the sample fluid processing cartridge with a sample container and a syringe coupled to the cartridge.
FIG. 5 is a cross section in the direction V-V in FIG. 4.
FIG. 6 is a cross section in the direction VI-VI in FIG. 4.
FIG. 7 is a cross section in the direction VII-VII in FIG. 4.
FIG. 8 is a top perspective view of an alternate embodiment of a fluid sample processing cartridge.
FIG. 9 is an exploded, bottom perspective view of the fluid sample processing cartridge of FIG. 8.
FIG. 10 is a top plan view of the fluid sample processing cartridge of FIG. 8.
FIG. 11 is the same top plan view of the fluid sample processing cartridge as in FIG. 10.
FIG. 12 is an exploded, bottom perspective view of the fluid sample processing cartridge of FIG. 8.
FIG. 13 is an exploded, top perspective view of the fluid sample processing cartridge of FIG. 8.
FIG. 14 is a cross-sectional view along the line X-X in FIG. 8.
FIG. 15 is an enlarged view of a portion of FIG. 14.
FIG. 16 is a top perspective view of a filter cover.
FIG. 17 is a schematic, transverse cross-sectional view of an inductively heatable filter assembly, a filter housing, and an induction coil along with a control system for controlling the temperature of the induction coil.
FIG. 18 is a schematic, transverse cross-sectional view of an alternate embodiment of a filter assembly and associated filter housing and induction coil.
FIG. 19 is a perspective view of a cylindrical induction coil as used in the embodiment of FIG. 18.
FIG. 20 is a perspective view of a docking station for operating a sample fluid processing cartridge.
FIG. 21 is an enlarged, partial side view of the docking station.
FIG. 22 is a partial, side view of a cam driven actuator of the docking station.
FIG. 23 is a top plan view of a actuating ball guide plate of the cam driven actuator.
FIG. 24 is a top plan view of a plunger guide plate of the cam driven actuator with an induction coil.
FIG. 25 is a top perspective view of a cam of the cam driven actuator.
FIG. 26 is a top plan view of the cam.
FIG. 27 is a flow chart illustrating a process for processing (lysing) a sample using a sample processing cartridge.
FIG. 28 is a top plan view of the fluid sample processing cartridge illustrating a first step in the operation of the fluid sample processing cartridge.
FIG. 29 is a top plan view of the fluid sample processing cartridge illustrating a second step in the operation of the fluid sample processing cartridge.
FIG. 30 is a top plan view of the fluid sample processing cartridge illustrating a third step in the operation of the fluid sample processing cartridge.
FIG. 31 is a top plan view of the fluid sample processing cartridge illustrating a fourth step in the operation of the fluid sample processing cartridge.
FIG. 32 is a top plan view of the fluid sample processing cartridge illustrating a fifth step in the operation of the fluid sample processing cartridge.
FIG. 33 is a top plan view of the fluid sample processing cartridge illustrating a sixth step in the operation of the fluid sample processing cartridge.
FIG. 34 is a top plan view of the fluid sample processing cartridge illustrating a seventh step in the operation of the fluid sample processing cartridge.
FIG. 35 is a top plan view of the fluid sample processing cartridge illustrating an eighth step in the operation of the fluid sample processing cartridge.
FIG. 36 is a schematic view of an alternative system for separating cells from a sample and for lysing the separated cells.
FIG. 37 is a schematic, transverse cross-sectional view of an alternate inductively heatable filter assembly, a filter housing, and an induction coil.
FIG. 38 is a schematic, perspective view of a first example of an inductively heatable filter assembly.
FIG. 39 is a schematic, perspective view of a second example of an inductively heatable filter assembly.
FIG. 40 is a schematic, perspective view of a third example of an inductively heatable filter assembly.
FIG. 41 is a schematic drawing of a fluidic system for isolating an analyte from a sample using methods described herein and for analyzing the isolated microbial analyte to detect whether the sample includes an analyte of interest.
FIG. 42 is flow chart showing a method for detecting a target nucleic acid from within the isolated microbial cell analyte.

### DETAILED DESCRIPTION

While aspects of the subject matter of the present disclosure may be embodied in a variety of forms, the following description and accompanying drawings are merely intended to disclose some of these forms as specific examples of the subject matter. Accordingly, the subject matter of this disclosure is not intended to be limited to the forms or embodiments so described and illustrated.

### Definitions

Unless defined otherwise, all terms of art, notations and other technical terms or terminology used herein have the same meaning as is commonly understood by one of ordinary skill in the art to which this disclosure belongs. All patents, applications, published applications and other publications referred to herein are incorporated by reference in their entirety. If a definition set forth in this section is contrary to or otherwise inconsistent with a definition set forth in the patents, applications, published applications, and other publications that are herein incorporated by reference, the definition set forth in this section prevails over the definition that is incorporated herein by reference.

Unless otherwise indicated or the context suggests otherwise, as used herein, "a" or "an" means "at least one" or "one or more."

References in the specification to "one embodiment," "an embodiment," "a further embodiment," "an exemplary embodiment," "some embodiments," "various embodiments," "some aspects," "a further aspect," "aspects," etc., indicate that the embodiment(s) described may include a particular feature, structure, or characteristic, but every embodiment encompassed by this disclosure may not necessarily include the particular feature, structure, or characteristic. Moreover, such phrases are not necessarily referring to the same embodiment. Further, when a particular feature, structure, or characteristic is described in connection with an embodiment, such feature, structure, or characteristic is also a description in connection with other embodiments, whether or not explicitly described.

As used herein, the term "sample" refers to any substance suspected of containing at least one analyte of interest. The analyte of interest may be, for example, a nucleic acid, a protein, a prion, a chemical, or the like. Exemplary samples include biological, environmental, and industrial samples. The substance may be derived from any source, including an animal, an industrial process, the environment, a water source, a food product, or a solid surface (e.g., surface in a medical facility). Substances obtained from animals may include, for example, blood or blood products, urine, mucus, sputum, saliva, semen, tears, pus, stool, nasopharyngeal or genitourinary specimen obtained with a swab or other collection device, and other bodily fluids or materials including liquified samples from a plant or animal, such as liquified fecal matter, mucus, sputum, or tissues. The term "sample" will be understood to mean a specimen in its native form or any stage of processing.

As used herein, the term "receptacle" refers to any type of fluid container, including, for example, a tube, vial, cuvette, a well or cartridge or other article having one or more wells formed therein or attached thereto, microtiter plate, etc., that is configured to contain a sample or another fluid (collectively referred to herein as fluid). Tubes may be cylindrical (i.e., circular in cross-section) or non-cylindrical and may have flat or rounded closed ends. Non-limiting examples of exemplary receptacles include, for example, Aptima^{®} urine specimen transport tube (Hologic, Marlborough, MA), Aptima^{®} specimen transfer tube (Hologic, Marlborough, MA), BD Vacutainer^{®} (Becton Dickinson, Franklin Lakes, NJ), cartridges for use with the Novodiag^{®} system, etc. Unless otherwise stated or the context suggests otherwise, references to a particular type of receptacle are not to be considered exclusive of other types of receptacles.

As used in this disclosure, the term "pore" means one of multiple, minute openings, gaps, or interstices within a body and through which liquids, gases, and sufficiently minute particles may pass through the body from a first surface of the body to a second surface of the body. Each pore may be a discrete opening extending continuously through the body from the first surface to the second surface, or pores may comprise a plurality of randomly arranged and interconnected openings, gaps, or interstices disposed between the first and second surfaces.

As used in this disclosure, the term "pore size" or any reference to the size of a pore refers to a measure of each pore characterizing the size of the largest particle that will pass through the pore. For example, in the context of a pore having a circular or generally circular transverse shape, the pore size may refer to the diameter of the shape. In the context of pores having a more rectangular or oval shape, the pore size may refer to the smaller width dimension of the shape, which corresponds to the width of the largest particle that may pass through the pore.

To the extent used herein, the terms "first" and "second" preceding the name of an element (e.g., a component, apparatus, location, feature, or a portion thereof or a direction of movement, force, or other dynamic action) are used for identification purposes to distinguish between similar elements, and are not intended to necessarily imply order, nor are the terms "first" and "second" intended to preclude the inclusion of additional similar elements. Furthermore, use of the term "first" preceding the name of an element (e.g., a component, apparatus, location, feature, or a portion thereof or a direction of movement, force, or other dynamic action) does not necessarily imply or require that there be additional, e.g., "second," "third," etc., such element(s).

This description may use various terms describing relative spatial arrangements and/or orientations or directions in describing the position and/or orientation of a component, apparatus, location, feature, or a portion thereof or direction of movement, force, or other dynamic action. Unless specifically stated, or otherwise dictated by the context of the description, such terms, including, without limitation, top, bottom, above, below, under, on top of, upper, lower, left, right, in front of, behind, beneath, next to, adjacent, between, horizontal, vertical, diagonal, longitudinal, transverse, radial, axial, clockwise, counter-clockwise, etc., are used for convenience in referring to such component, apparatus, location, feature, or a portion thereof or movement, force, or other dynamic action represented in the drawings and are not intended to be limiting.

Unless otherwise indicated, or the context suggests otherwise, terms used herein to describe a physical and/or spatial relationship between a first component, structure, or portion thereof and a second component, structure, or portion thereof, such as, attached, connected, fixed, joined, linked, coupled, or similar terms or variations of such terms, shall encompass both a direct relationship in which the first component, structure, or portion thereof is in direct contact with the second component, structure, or portion thereof or there are one or more intervening components, structures, or portions thereof between the first component, structure, or portion thereof and the second component, structure, or portion thereof.

Unless otherwise stated, any specific dimensions mentioned in this description are merely representative of an exemplary implementation of a device embodying aspects of the disclosure and are not intended to be limiting.

To the extent used herein, the terms "about" or "approximately" apply to all numeric values and terms indicating specific physical orientations or relationships such as horizontal, vertical, parallel, perpendicular, concentric, or similar terms, specified herein, whether or not explicitly indicated. This term generally refers to a range of numbers, orientations, and relationships that one of ordinary skill in the art would consider as a reasonable amount of deviation to the recited numeric values, orientations, and relationships (i.e., having the equivalent function or result) in the context of the present disclosure. For example, and not intended to be limiting, this term can be construed as including a deviation of ±10 percent of the given numeric value, orientation, or relationship, provided such a deviation does not alter the end function or result of the stated value, orientation, or relationship. Therefore, under some circumstances as would be appreciated by one of ordinary skill in the art a value of about or approximately 1% can be construed to be a range from 0.9% to 1.1%.

As used herein, the term "adjacent" refers to being near (spatial proximity) or adjoining. Adjacent objects or portions thereof can be spaced apart from one another or can be in actual or direct contact with one another. In some instances, adjacent objects or portions thereof can be coupled to one another or can be formed integrally with one another.

As used herein, the terms "substantially" and "substantial" refer to a considerable degree or extent. When used in conjunction with, for example, an event, circumstance, characteristic, or property, the terms can refer to instances in which the event, circumstance, characteristic, or property occurs precisely as stated as well as instances in which the event, circumstance, characteristic, or property occurs to a close approximation, such as accounting for typical tolerance levels or variability of the embodiments described herein.

The term "fluid communication" means either direct fluid communication, for example, two regions can be in fluid communication with each other via an unobstructed fluid passageway connecting the two regions, or can be capable of being in fluid communication, for example, two regions can be capable of fluid communication with each other when they are connected via a fluid passageway that can comprise a valve disposed therein, wherein fluid communication can be established between the two regions upon opening the valve, for example, by dissolving a dissolvable valve, bursting a rupturable seal, or actuating a mechanical valve disposed in the fluid passageway.

The term "microfluidic device" or "microfluidic cartridge" refers to a device within which fluid may flow and in which at least a portion of any fluid passages, channels, chambers, etc. within which fluid flows or is retained is geometrically constrained to a small scale (for example, sub-millimeter) at which surface forces acting on the fluids meet or exceed volumetric forces.

As used herein, "sample" refers to any substance suspected of containing an organism, virus or cell of interest or, alternatively, an analyte derived from an organism, virus or cell of interest, or any substance suspected of containing an analyte of interest. The substance may be, for example, an unprocessed clinical specimen, such as a blood or genitourinary tract specimen, a buffered medium containing the specimen, a medium containing the specimen and lytic agents for releasing an analyte belonging to an organism, virus or cell, or a medium containing an analyte derived from an organism, virus or cell which has been isolated and/or purified ("extracted") in a receptacle or on a material or device. For this reason, the term "sample" will be understood to mean a specimen in its raw form or to any stage of processing to release, isolate and purify ("extract") an analyte derived from the organism, virus or cell. Thus, references to a "sample" may refer to a substance suspected of containing an analyte derived from an organism, virus or cell at different stages of processing and is not limited to the initial form of the substance.

The term "wash buffer" means a solution for removing proteins, salts and other contaminants from bound nucleic acids.

The term "elution buffer" means a solution for releasing a nucleic acid from a solid support, including a capture probe associated with a solid support. An elution buffer can destabilize at least one interaction that contributes to the association of the nucleic acid with the solid support. For example: where the nucleic acid is ionically associated, elution buffer can contain sufficient salt to destabilize the association; where the nucleic acid is hydrophobically associated, elution buffer can contain sufficient organic solvent or cosolvent to destabilize the association; where the nucleic acid is associated through base pairing (hybridization), elution buffer can contain sufficient denaturing agent to destabilize the association; and where the nucleic acid is associated through specific binding (e.g., a capture probe labeled with a tag, which is bound to a binding partner for the tag), the elution buffer can contain sufficient free tag to destabilize the association.

The term "amplification reagent" means a material containing one or more components needed for an amplification reaction. In a nucleic acid amplification, such components may include primers, nucleoside triphosphates, and/or cofactors needed for amplification of a target nucleic acid (e.g., divalent cations such as Mg++).

The term "biosensor," as used herein, means a device that measures biological or chemical reactions by generating signals proportional to the concentration of an analyte in the reaction.

The term "biochip" means a collection of miniaturized test sites (or microarrays) arranged in a regular pattern on a solid substrate, where each test site may include attached nucleic acid molecules that are distinct from the nucleic acid molecules of the other test sites, such that multiple nucleic acid analytes can be detected simultaneously at unique locations on the solid substrate.

The term "nucleic acid array," "microarray," or similar term, as used herein, refers to a type of biosensor comprising a solid support upon which a collection of target-specific nucleic acids have been placed at defined locations.

An "analyte," as used herein, refers to a substance or one or more constituents thereof that is for identification and/or characterization, such as, e.g., detection via a probe or sequencing. Examples of analytes include, without limitation, DNA, RNA, and protein. In the context of the present disclosure, analytes are constituents of cells (e.g., microbial cells).

A "nucleic acid" refers to a multimeric compound comprising nucleotides or analogs that have nitrogenous heterocyclic bases or base analogs linked together to form a polymer, including conventional RNA, DNA, mixed RNA-DNA, and analogs thereof.

"Nucleic acid amplification" or "amplification" refers to any well-known in vitro procedure that produces multiple copies of a target nucleic acid sequence. Examples of such procedures include transcription-associated methods, e.g., transcription-mediated amplification (TMA), nucleic acid sequence-based amplification (NASBA) and others (e.g., U.S. Patent Nos. 5,399,491, 5,554,516, 5,437,990, 5,130,238, 4,868,105, and 5,124,246), replicase-mediated amplification (e.g., U.S. Patent No. 4,786,600), polymerase chain reaction (PCR) (e.g., U.S. Patent Nos. 4,683,195, 4,683,202, and 4,800,159), ligase chain reaction (LCR) (e.g., EP Patent No. 0320308), and strand-displacement amplification (SDA) (e.g., U.S. Patent No. 5,422,252).

"Oligomer," "oligonucleotide," or "oligo" refers to a nucleic acid of generally less than 1,000 nucleotides (nt), including those in a size range having a lower limit of about 5 nt and an upper limit of about 900 nt. The term oligonucleotide does not denote any particular function to the reagent; rather, it is used generically to cover all such reagents described herein. Oligomers may be referred to by a functional name (e.g., capture probe, detection probe, primer, or promoter primer) but those skilled in the art will understand that such terms refer to oligomers.

By "amplicon" or "amplification product" is meant a nucleic acid molecule generated in a nucleic acid amplification reaction and which is derived from a nucleic acid analyte. An amplicon or amplification product contains a target nucleic acid sequence that may be of the same or opposite sense as the nucleic acid analyte.

"Probe molecule," "detection probe," or "probe" refers to an oligomer that hybridizes specifically to a target sequence, including an amplification product, under conditions that promote nucleic acid hybridization, for detection of a nucleic acid analyte. Detection may either be direct (i.e., probe hybridized directly to the target) or indirect (i.e., a probe hybridized to an intermediate structure that links the probe to the target). A probe's target-specific sequence generally refers to the specific sequence within a larger sequence which the probe hybridizes specifically. A detection probe may include target-specific sequence(s) and non-target-specific sequence(s). Such non-target-specific sequences can include sequences which will confer a desired secondary or tertiary structure, such as a hairpin structure, which can be used to facilitate detection and/or amplification.

"Label" or "detectable label" refers to a moiety or compound joined directly or indirectly to a probe that is detected or leads to a detectable signal. Direct joining may use covalent bonds or non-covalent interactions (e.g., hydrogen bonding, hydrophobic or ionic interactions, and chelate or coordination complex formation), whereas indirect joining may use a bridging moiety or linker (e.g., via an antibody or additional oligonucleotide(s), which amplify a detectable signal). Any detectable moiety may be used, e.g., radionuclide, ligand such as biotin or avidin, enzyme, enzyme substrate, reactive group, chromophore such as a dye or particle (e.g., latex or metal bead) that imparts a detectable color, luminescent compound (e.g., bioluminescent, phosphorescent, or chemiluminescent compound such as an acridinium ester ("AE") compound), and fluorescent compound (i.e., fluorophore). Fluorophores may be used in combination with a quencher molecule that absorbs light when in close proximity to the fluorophore to diminish background fluorescence. Detectably labeled probes include, for example, hydrolysis (e.g., TaqMan^{™}) probes (e.g., U.S. Patent No. 5,723,591), AE-labeled probes (e.g., U.S. Patent No. 5,656,207), molecular torches (e.g., U.S. Patent No. 6,361,945), and molecular beacons (e.g., U.S. Patent No. 6,150,097).

The term "capture probe" means a nucleic acid oligomer that hybridizes to a target sequence in a target nucleic acid by standard base pairing and joins to a binding partner on an immobilized probe to capture the target nucleic acid to a support. In one embodiment, "target capture" refers to a process in which a target nucleic acid is purified or isolated by hybridization to a capture probe. In another embodiment, "target capture" refers to direct immobilization of a target nucleic acid on a solid support. One example of a capture probe includes two binding regions: a sequence-binding region (e.g., target-specific portion) and an immobilized probe-binding region, usually on the same oligomer, although the two regions may be present on two different oligomers joined together by one or more linkers. Another embodiment of a capture probe uses a target-sequence binding region that includes random or non-random poly-GU, poly-GT, or poly U sequences to bind non-specifically to a target nucleic acid and link it to an immobilized probe on a support.

The term "primer" means an oligomer that hybridizes to a template nucleic acid and has a 3' end that is extended by polymerization. A primer can be optionally modified, e.g., by including a 5' region that is non-complementary to the target sequence. Such modification can include functional additions, such as tags, promoters, or other sequences that may be used or useful for manipulating or amplifying the primer or target oligonucleotide. Examples of primers incorporating tags, or tags and promoter sequences, are described in U.S. Patent No. 9,284,549. A primer modified with a 5' promoter sequence can be referred to as a "promoter-primer." A person of ordinary skill in the art of molecular biology or biochemistry will understand that an oligomer that can function as a primer can be modified to include a 5' promoter sequence and then function as a promoter-primer, and, similarly, any promoter-primer can serve as a primer with or without its 5' promoter sequence.

A "forward primer" is configured to hybridize to the (-) strand of a target nucleic acid and can have a sequence partially or completely identical to the sequence of the (+) strand of the target nucleic acid. A "reverse primer" is configured to hybridize to the (+) strand of a target nucleic acid and can have a sequence partially or completely identical to the sequence of the (-) strand of the target nucleic acid. Unless otherwise indicated, the (+) strand refers to the coding strand of a protein-coding nucleic acid and the transcribed strand of non-coding sequences such as ribosomal and transfer RNAs and their corresponding DNAs, and the (-) strand refers to the reverse complement of the (+) strand.

The term "assay" means a procedure for detecting and/or quantifying an analyte in a sample. A sample comprising or suspected of comprising the analyte is contacted with one or more reagents and subjected to conditions permissive for generating a detectable signal informative of whether the analyte is present or the amount (e.g., mass or concentration) of analyte in the sample.

The term "molecular assay" means a procedure for specifically detecting and/or quantifying a target molecule, such as a target nucleic acid. A sample comprising or suspected of comprising the target molecule is contacted with one or more reagents, including at least one reagent specific for the target molecule, and subjected to conditions permissive for generating a detectable signal informative of whether the target molecule is present. For example, where the molecular assay is PCR, the reagents include primers specific for the target and the generation of a detectable signal can be accomplished at least in part by providing a labeled probe that hybridizes to the amplicon produced by the primers in the presence of the target. Alternatively, the reagents can include an intercalating dye for detecting the formation of double-stranded nucleic acids.

The term "reagent" means any substance(s) participates in an assay, other than sample material and the products of the assay. Exemplary reagents for use in a molecular assay include nucleotides, enzymes, primers, probes, and salts.

The terms "microbe," "microorganism," and "microbial," as used herein, refer to bacteria, archaea, fungi, and protists. In some embodiments, a microbe is a prokaryote (i.e., a species of bacteria or archaea). In other embodiments, a microbe is a microbial species having a cell wall such as any species of bacteria, archaea, or fungi, or some protist species.

The term "asymmetric structure," as used herein in reference to a filter, refers to a filter with pores having variable pore sizes and wherein pores on one side of the filter are generally larger than pores on an opposite side of the filter.

A "nucleotide" as used herein is a subunit of a nucleic acid consisting of a phosphate group, a 5-carbon sugar, and a nitrogenous base (also referred to herein as "nucleobase"). The 5-carbon sugar found in RNA is ribose. In DNA, the 5-carbon sugar is 2'-deoxyribose.

The term "target sequence" or "target nucleic acid sequence" as used herein refers to the particular nucleotide sequence of a nucleic acid analyte that is to be amplified and/or detected. The "target sequence" includes the complexing sequences to which oligonucleotides (e.g., priming oligonucleotides and/or promoter oligonucleotides) complex during an amplification process (e.g., PCR or TMA). Unless the context clearly dictates otherwise, where the nucleic acid analyte is originally single-stranded, the term "target sequence" will also refer to the sequence complementary to the "target sequence" as present in the nucleic acid analyte, and where the nucleic acid analyte is originally double-stranded, the term "target sequence" refers to both the sense (+) and antisense (-) strands.

Reference to a numerical range herein (e.g., "X to Y" or "from X to Y") includes the endpoints defining the range and all values falling within the range.

### Detailed Description of Drawings

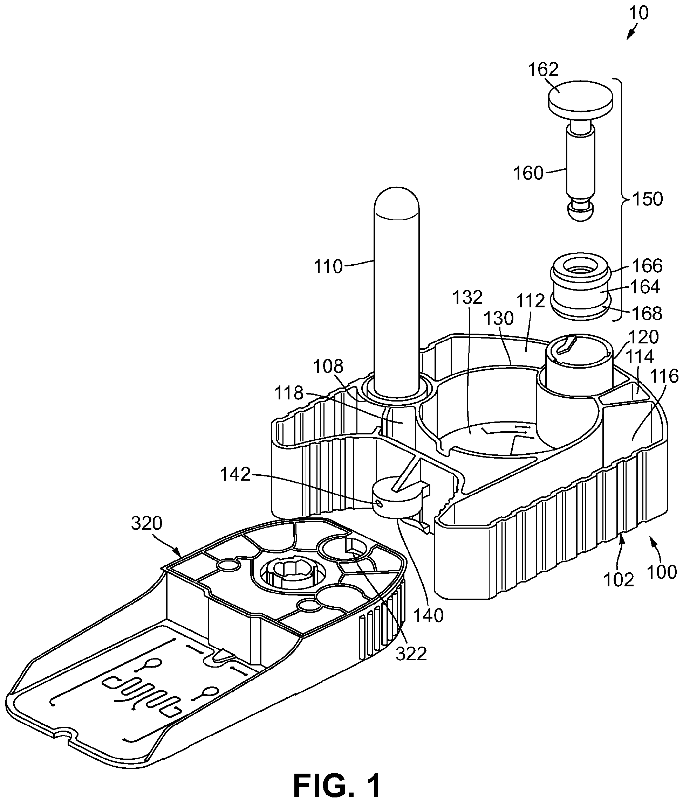

Referring to FIG. 1, a system 10 for separating cells from a sample and for lysing the separated cells may be operatively coupled to a reaction (assay) cartridge 320. Fundamentally, system 10 includes a fluid sample processing cartridge having a filter chamber within which is contained an inductively heatable filter assembly, including filter media and a conductive mesh disposed adjacent to or incorporated within the filter media. Sample fluid flows through the filter chamber, passing through the filter media and the conductive mesh, and the filter media is adapted to capture at least some cells contained in the sample, thereby separating the captured cells from a fluid component of the sample. An inductive coil disposed outside the filter chamber is situated with respect to the conductive mesh disposed inside the filter chamber so that when alternating current passes through the inductive coil, the coil generates alternating electromagnetic fields, and the conductive mesh is inductively heated (e.g., to at least 80°C (80°C - 150°C)), thereby heating the filter media and exposing cells captured thereby to the high temperature to lyse at least some of the captured cells. The material used to form the conductive mesh may be chosen in consideration of inductive (eddy current distribution) and thermal (heating transfer) properties so as to be completely heated as fast as possible, but also taking into account metal ion release and biocompatibility with a molecular amplification reaction to be performed after the thermal lysis phase.

The description herein of the system for separating cells from a sample and for lysing the separated cells includes filter media with an inductively-heatable conductive mesh to perform thermal lysis on cells captured in the filter media. In an alternate embodiment, however, the system may omit the conductive mesh and inductive coil, in which case the system includes only filter media disposed within a filter chamber to separate cells from a fluid component of the sample, and lysis, if performed at all, may be performed by non-thermal methods, such as chemical and/or mechanical lysis.

The sample processing cartridge may further include one or more fluid reservoirs and/or one or more functional connectors for connecting an external fluid container to each fluid connector, whereby one of the fluid reservoirs or external fluid containers contains a sample to be passed through the inductively heatable filter assembly. The system 10 may include a pump, such as a syringe, coupled to the sample processing cartridge, for moving fluid through the filter assembly, and a fluidic network including fluid channels and fluid flow control valves for selectively (e.g., one at a time) connecting the pump to each fluid reservoir or functional connector, thereby connecting the pump to the filter chamber, or connecting the filter chamber to, for example, a fluid discharge port of the fluid sample processing cartridge or to a waste reservoir of the cartridge or waste container connected to the cartridge.

Referring to FIGS. 1-4 and 7, an embodiment of a fluid sample processing cartridge 100, which may also be referred to as a fluid cartridge, a sample processing cartridge, or a fluid processing cartridge, includes a body 102 having a sample port at which sample material (e.g., fluid sample) may be introduced to downstream reservoirs or chambers within the cartridge 100 for processing. In the illustrated embodiment, sample material may be introduced via the sample port from a sample container well 108 configured to receive a sample container 110 (e.g., a sample collection tube, such as the Vacutainer^{®}), Becton Dickinson and Company, Franklin Lakes, NJ). In an alternate embodiment, sample material may be introduced via the sample port from a sample reservoir, or chamber, that is integrally formed with the body 102 and which includes an opening through which sample material may be introduced into the sample reservoir and a closure for closing the opening. The sample reservoir is in fluid communication with the sample port and is configured to receive and hold a sample fluid. Such an embodiment may include a cap or lid or other closure by which the sample reservoir may be closed after sample material is introduced to the sample reservoir. In one example, sample container well 108 may be closed off to form a sample reservoir, or chamber, by a top wall having an opening with a closable lid (not shown) by which sample material may be introduced to the sample reservoir.

Cartridge 100 shown in FIGS. 1-7 further includes a first reservoir 112, which may contain a first process fluid, a second reservoir 114, which may contain a second process fluid, and a third reservoir 116, which may contain a third process fluid. The embodiment shown in FIGS. 1 - 7 is exemplary; cartridge 100 may include more or less than three reservoirs containing or configured to contain process fluids, including no reservoirs. In the illustrated embodiment, body 102 further includes a syringe barrel 120, a waste reservoir 118, an outlet coupling hood 140, and discharge port 142. In one embodiment, syringe barrel 120 is a chamber, well, or cylinder that receives a syringe plunger for moving fluids within the body as described below. In another embodiment that does not employ a syringe to move fluids, body 102 may include a pump chamber or port (not shown) which may be coupled to a pump that moves fluids into and out of and/or through the pump chamber or pump port.

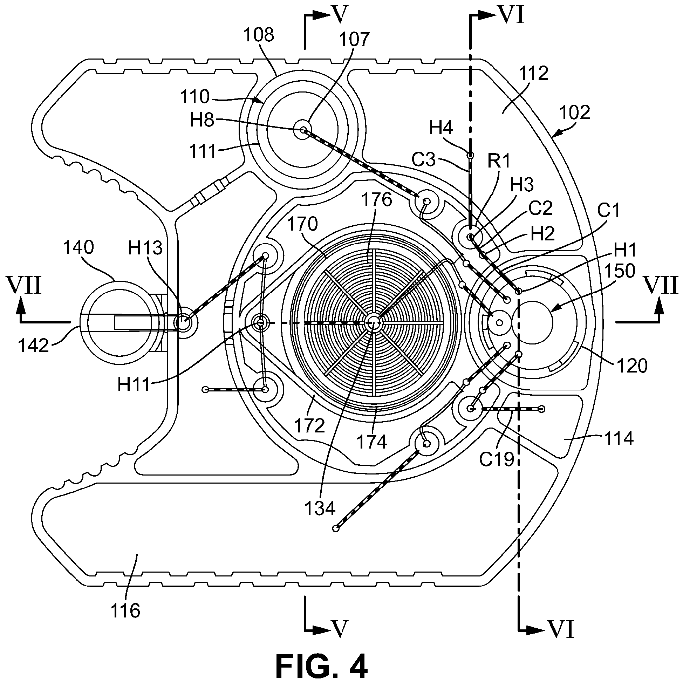

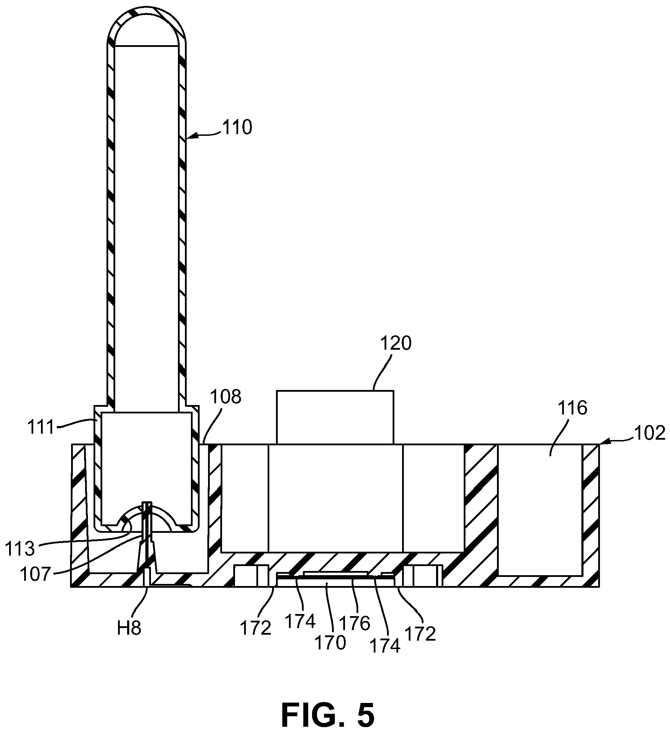

As shown in FIG. 5, which is a cross-section along the line V-V in FIG. 4, in one embodiment, a container 110 may comprise a tube with a cap 111. The container 110 is inverted, and the cap 111 is inserted into container well 108. A needle 107 extending upwardly from the bottom of the sample well 108 penetrates a septum or otherwise penetrable feature 113 of the cap 111. In an embodiment, needle 107 may be connected to container well 108 by a luer slip connector, luer lock, or other luer adapter. Alternatively, a custom needle can be positioned and secured within container well 108, for example, by an overmolding process.

The outlet coupling hood 140 is configured to couple the fluid cartridge 100 to the reaction cartridge 320, e.g., by operatively coupling the hood 140 to a process cartridge coupling port 322 of the reaction cartridge 320 to fluidly connect discharge port 142 to a fluid inlet port of reaction cartridge 320 to thereby deliver filtered and lysed sample material from sample processing cartridge 100 to reaction cartridge 320. Reaction cartridge 320 may be a microfluidic cartridge for performing molecular assays configured to detect the presence or absence of targeted nucleic acids within a sample, where detection of the targeted nucleic acids may be an indication of the presence of one or more pathogens. An exemplary cartridge for performing molecular diagnoses by detecting nucleic acids within a sample is described in U.S. Patent No. 10,654,039.

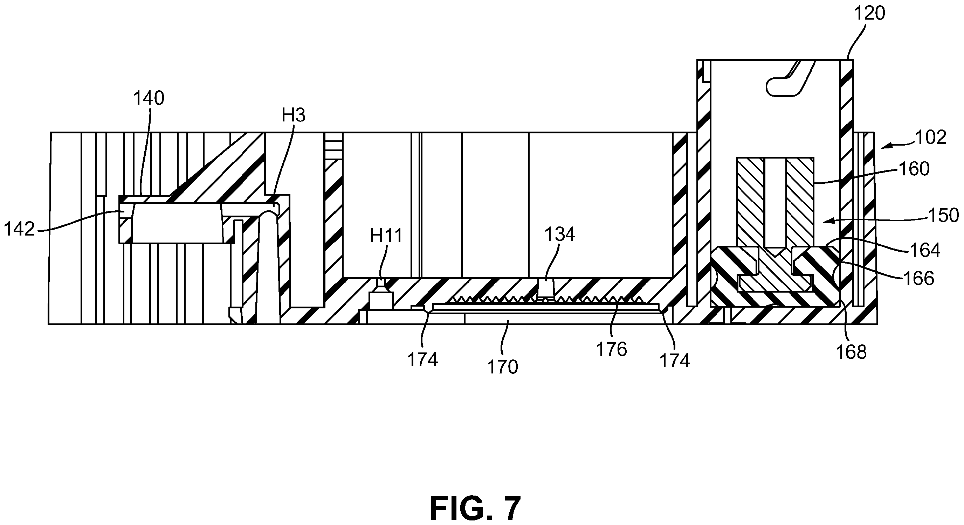

As shown in FIGS. 1, 4, and 7, in an embodiment, a syringe 150 may be operatively inserted into the syringe barrel 120 (syringe 150 and syringe barrel 120 may collectively be referred to as a "syringe"). Syringe 150 may include a syringe plunger 160, a plunger flange 162 at one end of plunger 160, and a plunger seal 164 disposed at an opposite end of plunger 160. FIG. 7, which is a cross-section along the line VII-VII in FIG. 4, shows plunger 160 and plunger seal 164. As shown in FIGS. 1 and 7, plunger seal 164 may include seal enhancing features, such as a top ring 166 and a bottom ring 168 providing enhanced sealing between the plunger seal 164 and an internal wall of the syringe barrel 120. In one example, the sample processing cartridge 100 includes a plunger seal 164 disposed within the syringe barrel 120, and the plunger 160 for engaging the plunger seal 164 is part of a docking station for receiving the sample processing cartridge 100 and actuating various functional components of the cartridge 100, such as syringe 150. An example of a docking station is described below.

In the illustrated embodiment, the reservoirs 112, 114, 116, the sample container well 108, the waste reservoir 118, and the syringe barrel 120 are disposed radially outside of a generally circular wall 130. A filter housing defines a filter chamber within which the inductively heatable filter assembly is disposed below a filter housing top wall 132 disposed within the circular wall 130 and above a filter housing bottom wall (not shown), as will be described in further detail below.

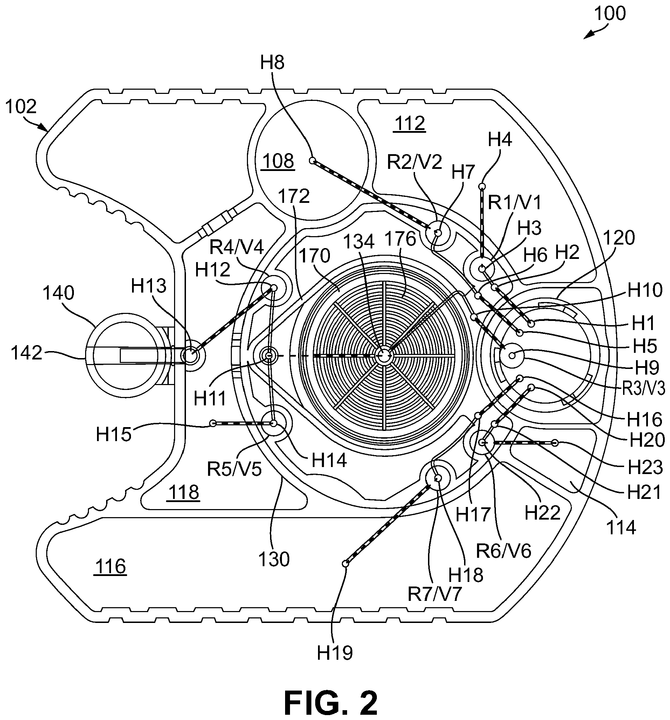

FIGS. 2-5 and 7 illustrate features of a filter chamber 170 within the body 102. FIG. 5 is a cross-section of the body 102 along the line V-V in FIG. 4, and FIG. 7 is a cross-section of the body 102 along the line VII-VII in FIG. 4, both of which show the filter chamber 170 in cross-section. The filter chamber 170 is delimited by a continuous wall 174, which, in the illustrated embodiment, is circular. An inductively heatable filter assembly, including filter media and a conductive mesh (not shown in FIGS. 2-5 and 7), is positioned within the filter chamber 170 and may be flat and have an outer peripheral shape conforming to the shape of the continuous wall 174. The filter assembly may be disposed below a top filter grill 176, and fluid enters the filter chamber 170 through a fluid inlet 134 located in the center of the top filter grill 176. Top filter grill 176 may include a plurality of concentric channels connected by strait radial channels so as to distribute the fluid entering the fluid inlet 134 substantially evenly across the surface of the filter assembly underlying the top filter grill 176. Similarly, the filter assembly may be disposed above a bottom filter grill (described below), and fluid exits the filter chamber 170 through a fluid outlet that may be located in the center of the bottom filter grill.

Filter chamber 170 may be enclosed by a filter holder (not shown in FIGS. 2-3 and 7) that fits within a perimeter wall 172 surrounding the continuous wall 174. An exemplary filter holder will be described in further detail below.

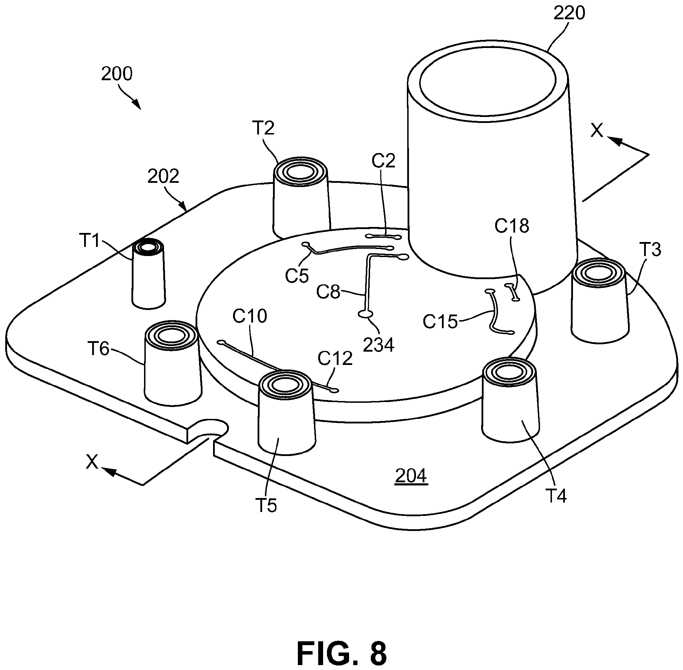

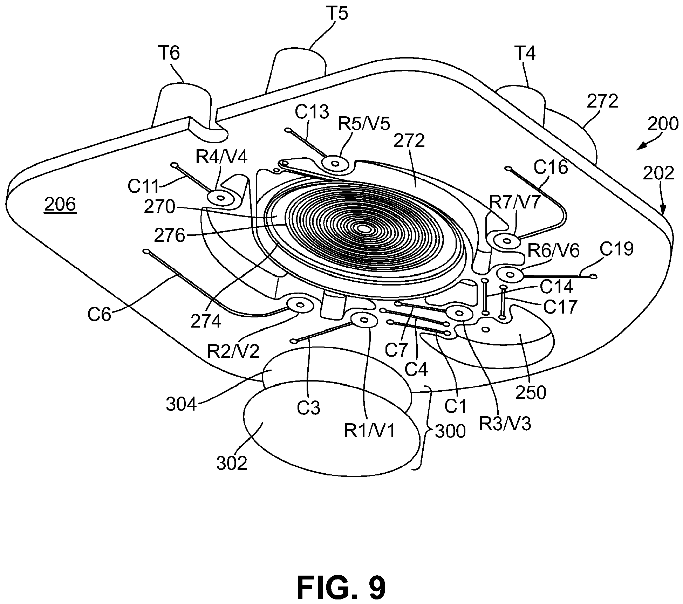

An alternative fluid sample processing cartridge 200, which also may be referred to as a fluid cartridge, a sample processing cartridge, or a fluid processing cartridge, is shown in FIGS. 8-15. Sample processing cartridge 200 includes a body 202 with a top surface 204 (see FIG. 8) and a bottom surface 206 (see FIG. 9). In the illustrated embodiment, body 202 includes a first functional connection T1, a second functional connection T2, a third functional connection T3, a fourth functional connection T4, a fifth functional connection T5, and a sixth functional connection T6. Although the body 202 may have more than or less than six functional connections, in the illustrated embodiment, functional connection T1 may be a sample port that is operably connectable to a sample fluid container, functional connections T2, T3, and T4 may be process fluid ports operably connectable to first, second, and third process fluid containers, respectively, functional connection T5 may be a waste port operably connectable to a waste container, and functional connection T6 may be connectable to a fluid collection container or to a reaction cartridge, such as reaction cartridge 300.

A body of a sample processing cartridge may include a combination of one or more fluid reservoirs - as with body 102 - and one or more functional connections - as with body 202.

Body 202 includes a pump chamber 220, which may be a syringe barrel that receives a syringe, such as syringe 150 described above. In an alternative embodiment, the body 202 may include a syringe port at which an external syringe pump may be operatively connected to the body.

The body 202, as well as body 102 described above, may be made by injection molding of a thermoplastic polymer material, such as cyclic olefin copolymers (COC) or cyclic olefin polymers (COP), or any thermoplastic polymer suitable for injection molding, with due consideration of nonspecific binding of molecules to the polymer surface and avoiding materials having excessive nonspecific binding. In one embodiment, body 102, 202 may be made of polypropylene (PP). Exemplary materials may also be chosen from the group including polycarbonate, polyacrylamide, polyethylene, polymethyl-methacrylate (PMMA), polydimetyl-siloxane (PDMS), polyvinyl chloride (PVC).

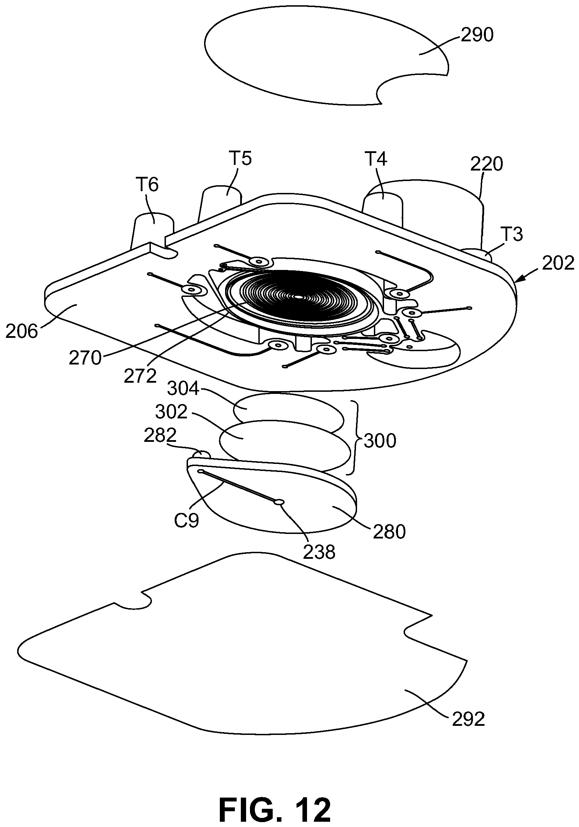

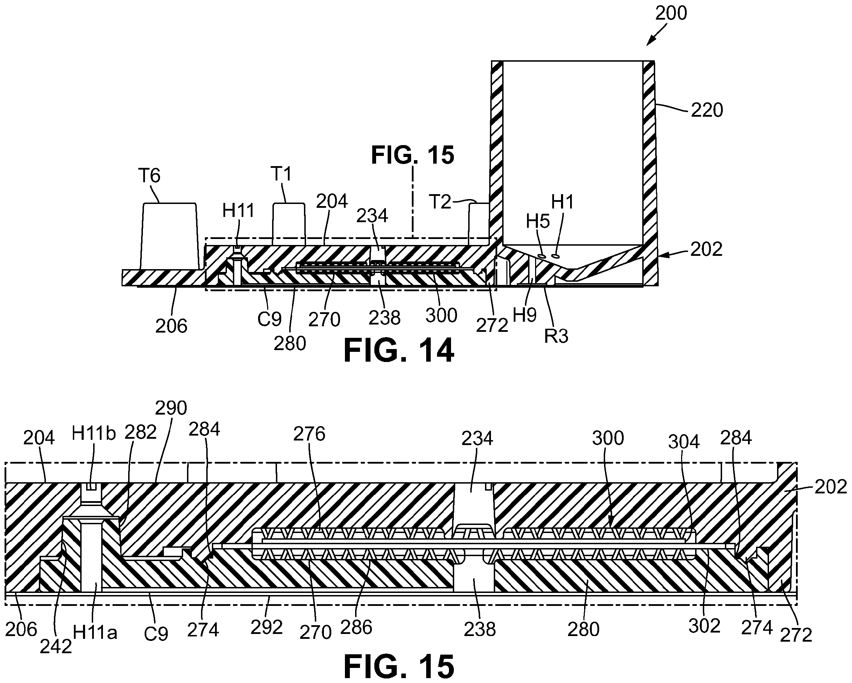

Referring to FIG. 9, which is a bottom perspective view of the body 202, a filter chamber 270 is defined by a continuous wall 274, which is circular in the illustrated embodiment. As also shown in FIG. 12, an inductively heatable filter assembly 300, comprising filter media 302 and a conductive mesh 304, is situated against a top wall 276 of the filter chamber 270 within the continuous wall 274, and is retained within the filter chamber 270 by a filter holder, or filter cover, 280 (see FIG. 12) that fits inside of a perimeter wall 272 formed in the body 202 (see also FIGS. 14 and 15). The conductive mesh may comprise a perforated plate (e.g., laser perforated) in which through holes are arranged in rows and columns. In one embodiment, holes have a diameter of about 0.04 mm and are spaced from adjacent holes in the same row or column by about 0.16 mm. In another embodiment, holes have a diameter of about 0.3 mm and are spaced from adjacent holes in the same row or column by about 0.6 mm. Holes in the perforated plate may have a diameter ranging from about 0.04 mm to about 0.3 mm with a hole-to-hole spacing within each row or column ranging from about 0.16 mm to about 0.6 mm. In this regard, the term "diameter" is not intended to limit the openings to a circular shape but is meant to refer to the maximum width of an opening of any shape. Rows and columns may be arranged so that holes in adjacent rows and columns are aligned, or rows and columns may be offset so that, for example, holes in every other row or every other column are aligned. The perforated plate may be formed from a conductive material, such as copper, stainless steel, or aluminum.

In other embodiments, as described below, the conductive mesh is formed as a woven wire mesh or a layer of sintered metal fibers.

In the illustrated embodiment, the conductive mesh 304 overlies the filter media 302, which, in this context, means the conductive mesh 304 is situated on top of the filter media 302 when the cartridge 200 is in its normal, operative orientation, as shown in FIG. 9. In an alternate configuration, the conductive mesh 304 underlies the filter media 302, which, in this context means the conductive mesh 304 is situated below the filter media 302 when the cartridge 200 is in its normal, operative orientation, as shown in FIG. 9. In still another configuration, the inductively heatable filter assembly may include at least two conductive meshes 304, one overlying the filter media 302 and one underlying the filter media 302. In a further configuration, the filter media 302 of the inductively heatable filter assembly may include two (or more) filter membranes with at least one conductive mesh 304 sandwiched between two filter membranes (i.e., the conductive mesh underlies the upper filter membrane and overlies the lower filter membrane). In yet another embodiment described below, the conductive mesh encircles the filter media.

In an embodiment, at least a portion of the conductive mesh 304 contacts at least a portion of the filter media 302. In an embodiment, conductive mesh 304 may be heat staked within the filter chamber 270, and the filter media 302 may comprise a filter membrane that is ultrasonically welded into the filter chamber 270.

As shown in FIGS. 14 and 15, filter holder 280 is secured within the continuous perimeter wall 272 in the bottom of the body 202 and includes a continuous groove 284 that receives the continuous wall 274 of the body 202. Filter holder 280 includes a raised button 282 that is frictionally secured (press fit) within a recess 242 formed in the body 202. The filter holder 280 may be secured to the body 202 by any suitable means, such as ultrasonic welding, e.g., at the continuous wall 274 disposed within the circular groove 284, and or by adhesives.

A fluid inlet 234 extends from the top surface 204 of the body 202 into the filter chamber 270, and a fluid outlet 238 extends through the filter holder 280 from the filter chamber 270. The inductively heatable filter assembly 300 is sandwiched between the top wall 276 and a bottom wall 286 of the filter chamber 270, which is part of the filter holder 280. Top wall 276 and/or bottom wall 286 may comprise liquid distribution grill (also referred to in this description as a filter grill) configured to distribute liquid entering the filter chamber 270 through the fluid inlet 234 evenly across the filter assembly 300 or to collect fluid that has passed through the filter assembly 300 toward the fluid outlet 238. Such liquid distribution grill may include concentric channels and straight radial channels (as in a typical syringe filter housing) to distribute fluid evenly across a filter assembly 300 within the filter chamber 270. Thus, fluid passes into the filter chamber 270 through the filter fluid inlet 234, is distributed across the filter assembly 300 by the liquid distribution grill of the top wall 276, passes through the filter assembly 300, is collected by the liquid distribution grill of the bottom wall 286 and directed toward the fluid outlet 238, and flows out of the filter chamber 270 through the fluid outlet 238.

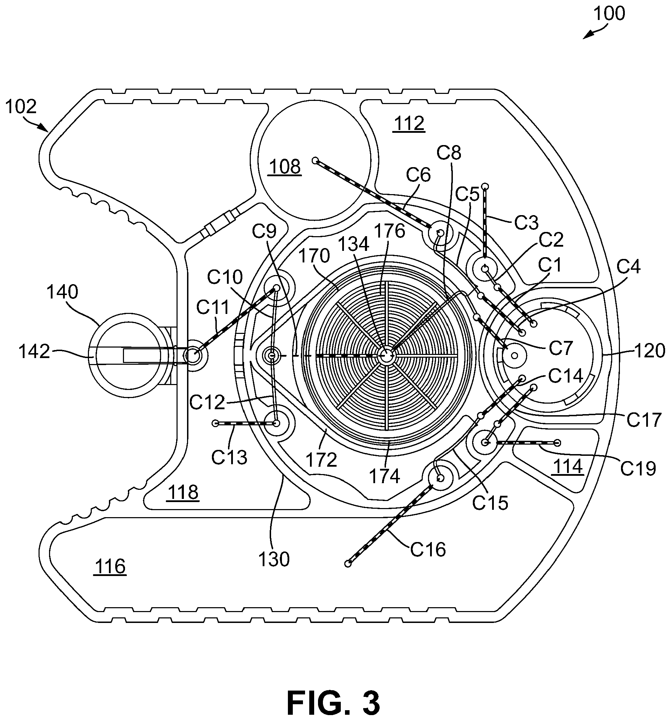

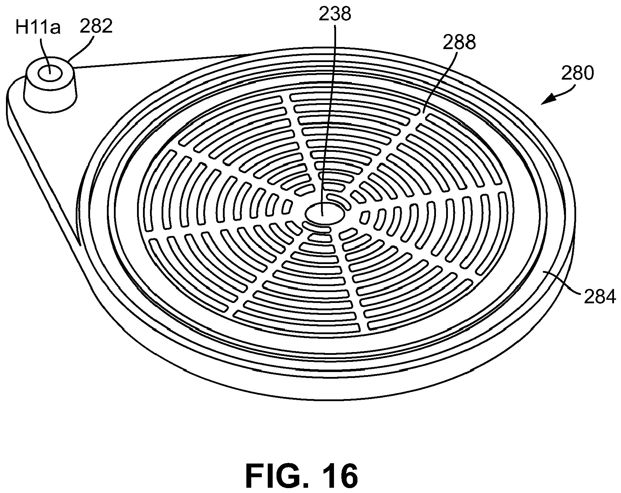

Top filter grill 176 is shown in FIGS. 2 and 3 and is described above. Referring to FIG. 16, filter holder 280 may include the bottom filter grill 288, which may be a mirror image of the top filter grill 176 described above. Like top filter grill 176, bottom filter grill 288 may include a plurality of concentric channels connected by strait radial channels so as to collect the fluid passing through the filter assembly 300 and direct the collected fluid to the fluid outlet 238.

The sample processing cartridge 100, 200 includes a fluidic network of channels (e.g., microchannels), in which various fluids circulate, and each channel may include at least one valve for controlling the circulation of such fluids in the corresponding channel.

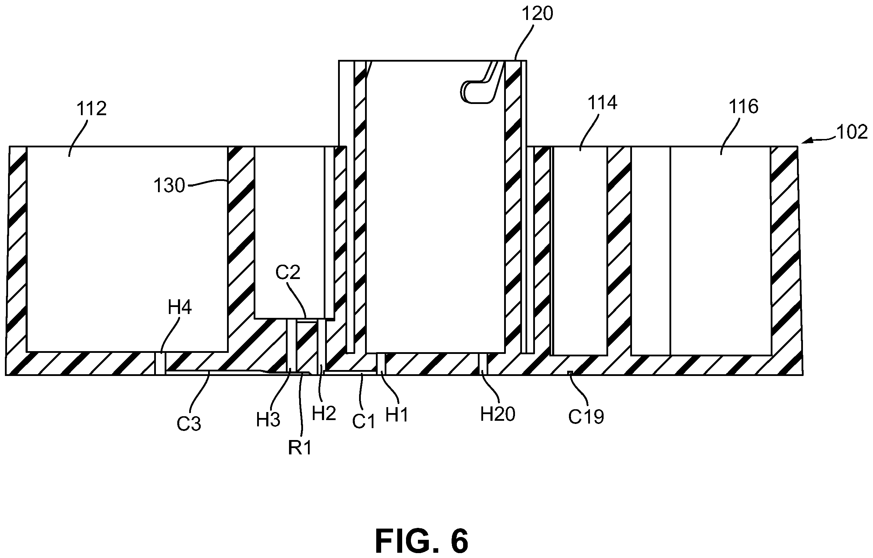

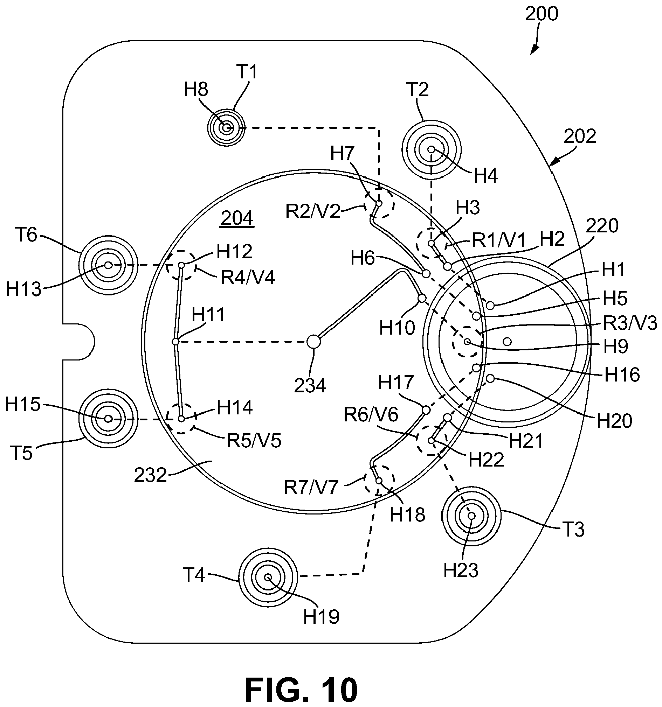

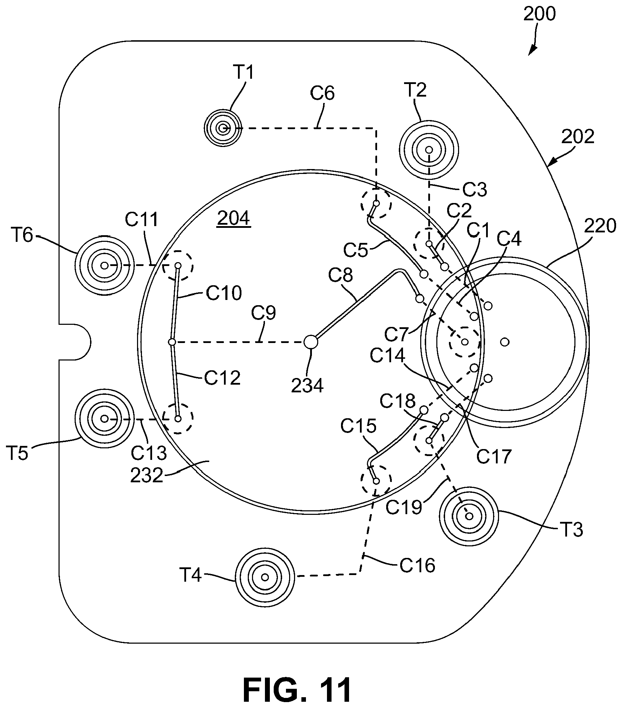

Elements of the fluidic network of sample processing cartridge 100 are described with reference to FIGS. 2 and 3, and elements of the fluidic network of sample processing cartridge 200 are described with reference to FIGS. 10 and 11. FIGS. 2 and 3 are identical top plan views of the sample processing cartridge 100, and FIGS. 6 and 7 are identical, top plan views of the sample processing cartridge 200. The views are duplicated in FIGS. 2 and 3 and in FIGS. 10 and 11 so that all relevant features can be identified between the duplicated views without having to crowd either view with reference numbers and lead lines.

Referring to FIGS. 3 and 11, a plurality of fluid flow channel segments C1-C19 are formed in the sample processing cartridge 100 and 200, respectively. All channel segments are formed in the respective body 102, 202, except channel segment C9, which is formed in the filter holder 280. Each channel segment is a groove formed in the top or bottom surface of the body 102 or 202 that is covered with a film to enclose the groove and form a channel. In FIGS. 3 and 11, channel segments formed from grooves disposed in the top surface of the body are depicted with solid lines, and channel segments formed from grooves disposed in the bottom surface of the body are depicted with dashed lines. Thus, in FIG. 11, for example, channel segments C2, C5, C8, C10, C12, C18, and C15 are formed of respective grooves cut into top surface 204 of body 202, e.g., parallel to and flush with the top surface 204 (see also FIG. 8). Channel segments C1, C3, C4, C6, C7, C11, C13, C14, C16, C17, and C19 are formed of respective grooves cut into bottom surface 206 of the body 202, e.g., parallel to and flush with the bottom surface 206 (see also FIG. 12). Channel segment C9 is formed of a groove cut into a bottom surface of the filter holder 280, e.g., parallel with and flush with the bottom surface of filter holder 280. Similarly, in FIG. 3, channel segments C2, C5, C8, C10, C12, C18, and C15 are formed of respective grooves cut into a top surface of body 102, e.g., parallel to and flush with the top surface. Channel segments C1, C3, C4, C6, C7, C11, C13, C14, C16, C17, and C19 are formed of respective grooves cut into a bottom surface of the body 102, e.g., parallel to and flush with the bottom surface 206.

In various embodiments, channel segments C1-C19 have a depth generally between 0.01 mm and 0.5 mm, for example between 0.2 mm and 0.4 mm, and, for example, about 0.3 mm. In various embodiments, the width of channel segments C-C19 may be about 0.4 mm - 0.5 mm.

Referring to FIGS. 2 and 10, each body 102, 202, includes through holes H1-H23. All through holes, except H11, extend from the bottom surface of the body 102 and into one of the reservoirs 112, 114, 116, the sample container well 108, the waste reservoir 118, the syringe barrel 120, or the region inside wall 130, or between the top surface and the bottom surface of the body 202, and may be perpendicular to the respective top and bottom surfaces. As shown in FIG. 15, hole H11 includes a first part H11a extending through raised button 282 of filter holder 280 (see also FIG. 16) and a second part H11b extending through the body 202. Hole H11 has a similar configuration for body 102 as shown in FIG. 7.

Combinations of through holes and channel segments form continuous channels connecting different parts of the sample processing cartridge 100, 200. Through holes may be terminal through holes - located at each end of a continuous channel - or intermediate through holes connecting channel segments formed on the top surface of the body to channel segments formed on the bottom surface of the body.

Referring to FIGS. 2 and 3, the channel segments C1 to C19 and through holes H1 to H23 form a network of fluidic channels in sample processing cartridge 100 as follows:
- Channel segments C1, C2, and C3, through hole H2 connecting bottom surface channel segment C1 with top surface channel segment C2, and through hole H3 connecting top surface channel segment C2 with bottom surface channel segment C3, form a continuous channel connecting through hole H1 in the bottom of the syringe barrel 120 of body 102 to through hole H4 in first reservoir 112 of the body 102 (see also FIG. 6, which is a cross-section along the line VI-VI in FIG. 4; syringe 150 is omitted from FIG. 6).
- Channel segments C4, C5, and C6, through hole H6 connecting bottom surface channel segment C4 with top surface channel segment C5, and through hole H7 connecting top surface channel segment C5 with bottom surface channel segment C6, form a continuous channel connecting through hole H5 in the bottom of the syringe barrel 120 of body 102 to through hole H8 in sample container well 108 of the body 102. Through hole H8 is in communication with needle 107 (see FIG. 5) and functions as the sample port.
- Channel segments C7 and C8 and through hole H10 connecting bottom surface channel segment C7 with top surface channel segment C8 form a continuous channel connecting through hole H9 in the bottom of the syringe barrel 120 of body 102 to fluid inlet 134 of the filter chamber 170.
- Channel segments C14, C15, and C16, through hole H17 connecting bottom surface channel segment C14 with top surface channel segment C15, and through holeH18 connecting top surface channel segment C15 with bottom surface channel segment C16, form a continuous channel connecting through hole H16 in the bottom of the syringe barrel 120 of body 102 to through hole H19 in third reservoir 116 of the body 102.
- Channel segments C17, C18, and C19, through hole H21 connecting bottom surface channel segment C17 with top surface channel segment C18, and through holeH22 connecting top surface channel segment C18 with bottom surface channel segment C19, form a continuous channel connecting through hole H20 in the bottom of the syringe barrel 120 of body 102 to through hole H23 in second reservoir 114 of the body 102.
- Channel segments C9, C10, and C11, through hole H11 connecting bottom surface channel segment C9 with top surface channel segment C10, and through hole H12 connecting top surface channel segment C10 with bottom surface channel segment C11, form a continuous first outlet channel connecting the fluid outlet of the filter chamber 170 to through hole H13 and discharge port 142 of the body 102.
- Channel segments C9, C12, and C13, through hole H11 connecting bottom surface channel segment C9 with top surface channel segment C12, and through hole H14 connecting top surface channel segment C12 with bottom surface channel segment C13, form a continuous second outlet channel connecting the fluid outlet of the filter chamber 170 to through hole H15 in the waste reservoir 118 of the body 102.

Referring to FIGS. 10 and 11, the channel segments C1 to C19 and through holes H1 to H23 form a network of fluidic channels in sample processing cartridge 200 as follows:
- Channel segments C1, C2, and C3, through hole H2 connecting bottom surface channel segment C1 with top surface channel segment C2, and through hole H3 connecting top surface channel segment C2 with bottom surface channel segment C3, form a continuous channel connecting through hole H1 in the bottom of the syringe barrel 220 of body 202 to through hole H4 of second functional connection T2 of the body 102.
- Channel segments C4, C5, and C6, through hole H6 connecting bottom surface channel segment C4 with top surface channel segment C5, and through hole H7 connecting top surface channel segment C5 with bottom surface channel segment C6, form a continuous channel connecting through hole H5 in the bottom of the syringe barrel 220 of body 202 to through hole H8 of first functional connection T1 of the body 102.
- Channel segments C7 and C8, through hole H10 connecting bottom surface channel segment C7 with top surface channel segment C8, form a continuous channel connecting through hole H9 in the bottom of the syringe barrel 220 of body 202 to fluid inlet 234 of the filter chamber 270.

- Channel segments C14, C15, and C16, through hole H17 connecting bottom surface channel segment C14 with top surface channel segment C15, and through holeH18 connecting top surface channel segment C15 with bottom surface channel segment C16, form a continuous channel connecting through hole H16 in the bottom of the syringe barrel 220 of body 202 to through hole H19 of fourth functional connection T4 of the body 102.
- Channel segments C17, C18, and C19, through hole H21 connecting bottom surface channel segment C17 with top surface channel segment C18, and through holeH22 connecting top surface channel segment C18 with bottom surface channel segment C19, form a continuous channel connecting through hole H20 in the bottom of the syringe barrel 220 of body 202 to through hole H23 of third functional connection T3 of the body 102.
- Channel segments C9, C10, and C11, through hole H11 connecting bottom surface channel segment C9 (formed in filter holder 280) with top surface channel segment C10, and through hole H12 connecting top surface channel segment C10 with bottom surface channel segment C11, form a continuous channel connecting the fluid outlet 238 of the filter chamber 270 to through hole H13 of sixth functional connection T6 of the body 102.
- Channel segments C9, C12, and C13, through hole H11 connecting bottom surface channel segment C9 (formed in filter holder 280) with top surface channel segment C12, and through hole H14 connecting top surface channel segment C12 with bottom surface channel segment C13, form a continuous channel connecting the fluid outlet 238 of the filter chamber 270 to through hole H15 of fifth functional connection T5 of the body 102.

Each of through holes H6, H7, H9, H12, H13, H17, and H21 has associated therewith a recess R1, R2, R3, R4, R5, R7, and R6, respectively. Each recess R1-R7 is formed on and extends into the bottom surface of body 102 or bottom surface 206 of body 202. That is, unlike channel segments formed on the bottom surface of body 102 and body 202 shown in dashed lines, recesses R1-R7 are shown in solid lines although they are formed on the bottom surface of body 102 and body 202. In various embodiments, is cylindrical in shape with a diameter of between 1 mm and 10 mm, for example between 2 mm and 8 mm, and for example of about 4 mm, and a depth of between 0.02 mm and 0.4 mm, for example between 0.05 mm and 0.15 mm, and for example about 0.1 mm. Each of the recesses R1 to R7 has a diameter greater than the diameter of its corresponding through hole and may be positioned concentrically with respect to its corresponding through hole H6, H7, H9, H12, H13, H21, and H17 so that an annular surface within each recess between the outer periphery of the recess and the corresponding through hole defines a valve seat.

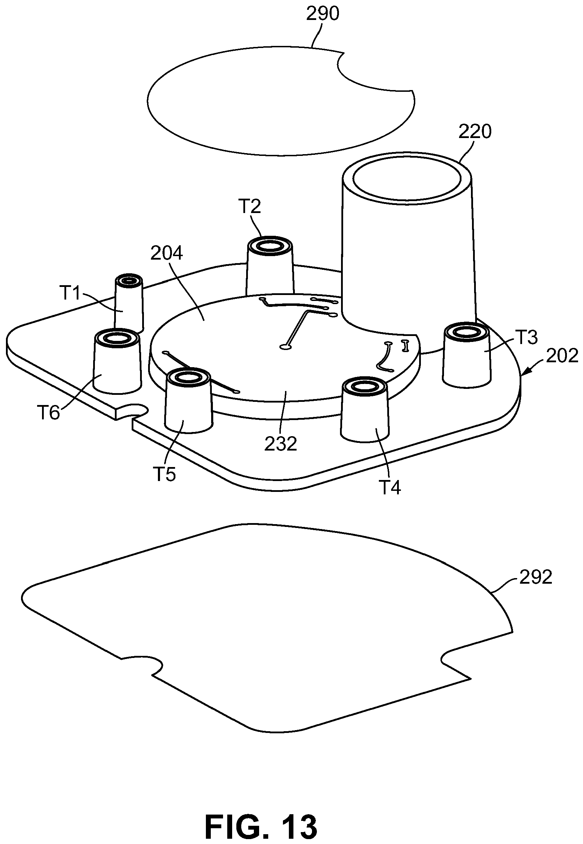

As shown in FIGS. 12 and 13, channel segments C2, C5, C8, C10, C12, C18, and C15 are formed by a top material 290 (e.g., a layer formed by a foil, film, or a panel) bonded, e.g., by laser welding, to a portion of the top surface 204 of the body 204 to cover and enclose the grooves formed in the top surface 204. Channel segments C1, C3, C4, C6, C7, C9, C11, C13, C14, C16, C17, and C19 are formed by a bottom material 292 (e.g., a flexible film or membrane) bonded, e.g., by laser welding, to the bottom surface 206 of the body 204 and the filter holder 280 to cover and enclose the grooves formed in the bottom surface 206 of the body and the bottom surface of the filter holder 280.

Top material 290 and bottom material 292 may be made from a thermoplastic film (e.g., polypropylene) of about 0.1-0.2 mm thickness, bonded or welded to the top surface 204 and the bottom surface 206, respectively, of the body 202, by thermo-welding, e.g., by laser-welding, bonding, adhering or chemical linking methods. Top material 290 and bottom material 292 cooperate with the grooves, the through holes and the recesses to form a plurality of channels, or microchannels, and valves.

The bottom material 292 is deformable (e.g., elastically deformable) and is bonded over a valve seat formed by each of the recesses R1 to R7, respectively, formed in bottom surface 206 of the body 202 to define valves V1 to V7, respectively. Similarly, an elastically deformable bottom material is bonded over a valve seat formed by each of the recesses R1 to R7, respectively, formed in bottom surface of the body 102 to define valves V1 to V7, respectively.

A surface of the bottom material 292 situated across each recess R1 to R7 is, when not deformed, approximately planar and parallel to the bottom surface 206 of the body 202. When the material disposed over a recess is not deformed, fluid is able to pass through the corresponding through hole and an associated channel segment terminating at the recess surrounding the through hole. Material 292 is capable of being deformed locally by an external actuator (an example of which is described below). Local deformation of the bottom material 292 at each recess R1 to R7 under the action of this external actuator opens or closes the corresponding valve V1 to V7. More precisely, localized deflection of the bottom material 292 opposite the valve seat of each recess R1 to R7, presses the material into contact with the valve seat, i.e., the annular surface at the base of the recess surrounding the through hole, thereby obstructing, or blocking, fluid flow through the corresponding through holes H6, H7, H9, H12, H13, H21, and H17, whose diameter is less than that of each recess R1 to R7. The deflection or deformation of the material 292 into contact with the valve seat may be elastic so that upon release of a force generated by the external actuator, the material 292 generally assumes its non-deformed condition approximately planar and parallel to the bottom surface 206 of the body 202, thereby permitting fluid flow through the corresponding through hole.

As no recesses, or valves, are formed on the top surface 204 of the body 202, it is not necessary for top material 290 to be locally deformable so as to effect a valve in an associated recess. Accordingly, top material 290 may be formed of a rigid or partially rigid plate.
- Valves V1 to V7 control fluid flow through the fluidic network of sample processing cartridge 100 (FIG. 2) and through the fluidic network of sample processing cartridge 200 (FIG. 10) as follows:
- Valve V1 controls fluid flow between first reservoir 112 and syringe barrel 120 of sample processing cartridge 100 or between second functional connection T2 and syringe barrel 220 of sample processing cartridge 200;
- Valve V2 controls fluid flow between sample container well 108 and syringe barrel 120 of sample processing cartridge 100 or between first functional connection T1 and syringe barrel 220 of sample processing cartridge 200;
- Valve V3 controls fluid flow between syringe barrel 120 and fluid inlet 134 of the filter chamber 170 of sample processing cartridge 100 or between syringe barrel 220 and fluid inlet 234 of the filter chamber 270 of sample processing cartridge 200;
- Valve V4 controls fluid flow between a fluid outlet of the filter chamber 170 and the fluid discharge port 142 of sample processing cartridge 100 or between fluid outlet 238 of the filter chamber 270 and the sixth functional connection T6 of sample processing cartridge 200;
- Valve V5 controls fluid flow between a fluid outlet of the filter chamber and the waste reservoir 118 of sample processing cartridge 100 or between fluid outlet 238 of the filter chamber 270 and the fifth functional connection T5 of sample processing cartridge 200;
- Valve V6 controls fluid flow between second reservoir 114 and syringe barrel 120 of sample processing cartridge 100 or between third functional connection T3 and syringe barrel 220 of sample processing cartridge 200; and
- Valve V7 controls fluid flow between third reservoir 116 and syringe barrel 120 of sample processing cartridge 100 or between fourth functional connection T4 and syringe barrel 220 of sample processing cartridge 200.

The channel segments C1-C19, through holes H1-H23, and valves V1-V7 form a fluid flow network.

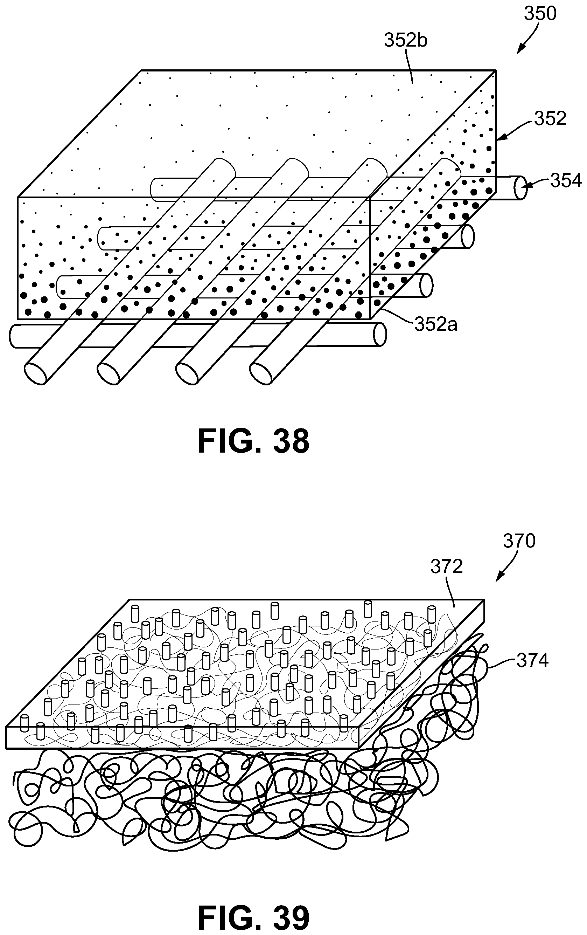

In various embodiments, the inductively heatable filter assembly may include filter media, such as one or more filter elements, or filter membranes, and a conductive mesh that will heat up when exposed to alternating electromagnetic fields generated by an induction coil operatively positioned with respect to the conductive mesh. FIGS. 38, 39, and 40 are schematic, perspective views of three examples of an inductively heatable filter assembly.

One concept of the disclosure is to use size selection filtering for enrichment of microbial organisms from a vast background matrix volume (such as, blood or other biological matrix), thus leading both to enrichment of the analyte concentration in the sample as well as replacing the sample matrix with one more compatible for downstream analytics procedures (e.g., PCR or other detection methods, including immunological detection). Accordingly, a large sample volume can be processed for detecting analytes that may be present in very low concentrations. Size selection is achieved by using specific filters with selected pore sizes and filter material, optionally with asymmetric structure such that pore size decreases moving downstream through the filter membrane. In some examples, prokatyoric and eukaryotic target organism (bacteria and yeast) are trapped with filters having pore sizes of about 0.22 µm, by way of example, and are composed of, e.g., polyethersulfone (PES) or some other applicable membrane material. Selected membrane size and material allows efficient capture of the organisms containing the target analytes, and, moreover, the membrane type is not easily clogged even by using larger sample volume or by excessive washing of the membrane for particles smaller than the pore size. To bring the analytes to the downstream analytics processes (such as, PCR, isothermal RNA/DNA amplification, protein or other biomolecule analyte detection), on-filter lysis is used. As described herein, microbial/ pathogenic organisms captured by the filter are lysed/disintegrated by heat applied to the filter, which releases analyte molecules from the intact organisms, allowing the analytes to be released from the capturing filter.

As shown in FIG. 38, a filter membrane of a filter assembly 350 may be a porous substrate 352 (e.g., a sponge or sponge-like substrate). Particularly suitable filters for use in the systems and methods described herein include filters comprising polyethersulfone (PES), cellulose, nylon, polyvinylidene fluoride (Poly(1,1-difluoroethylene), PVDF), polycarbonate, or glass (e.g., borosilicate glass) fiber. In certain variations, the filter has pores with a size of at least about 0.1 µm. In other variations, the filter has a pore size of about 1.0 µm or less (e.g., a pore size of between about 0.1 µm and about 1.0 µm, a pore size of between about 0.2 µm and about 1.0 µm, or a pore size of about 0.22 µm). In other, non-mutually exclusive variations, the porous substrate comprises an asymmetric structure, characterized by a larger pore size on an upstream side 352a of the porous substrate (i.e., a side of the filter facing a fluid inlet to the filter chamber) and a smaller pore size on a downstream side 352b of the porous substrate (i.e., a side of the filter facing a fluid outlet from the filter chamber). The asymmetric pore sizes provide a pre-filter functionality: the larger pores trap larger particles on top of the membrane, whereas smaller particles are able to continue to the interior or through the membrane, according to their size. The pore size progressively gets smaller through the thickness of the filter membrane, thus trapping particles at different parts (depths) of the membrane according to their sizes. In contrast, filter media with generally homogenous pore sizes gather all particles larger than the pore size at the same level, or depth, within the filter membrane, thus clogging the membrane more easily. Because the asymmetric filter membrane is less likely to clog, it provides a larger effective membrane area.

In some embodiments, the pore size may be between about 5 µm and about 20 µm on the upstream 352a side and up to about 0.2 µm on the downstream side 352b, and the porous substrate 352 may have a thickness of 160 to 185 µm. In some examples, the pore size may decrease continuously from the upstream side 352a to the downstream side 352b. In other examples, the porous substrate 352 may comprise two or more layers, each having a generally constant pore size and where the pore size of each layer decreases progressing from the upstream side 352a to the downstream side 352b.

A conductive mesh 354 may comprise a metal woven wire mesh (e.g., a conductive material, such as, copper, stainless steel, or aluminum) having openings (e.g., between adjacent strands of mesh) of about 40 µm to about 300 µm and a thickness of about 130 µm to 350 µm and may be disposed so as to overlap the upstream side 352a (as shown), the downstream side 352b, or both the upstream and downstream sides of the filter media. Where a wire mesh 354 overlaps the upstream side 352a of the filter media, the size of openings in the wire mesh in one embodiment should be sufficiently large to allow the passage of red blood cells, which are generally 6 to 8 µm in diameter. Another consideration for a woven mesh is the amount and proximity of the overlapping wires in the mesh. If openings in the wire mesh are very small, meaning that a relatively large number of overlapping wires (i.e., the warp and the weft wires) in close proximity to each other, the high density of wires can result in the mesh overheating from the inductive heating. On the other hand, if the openings in the wire mesh are large, meaning that a relatively smaller number of overlapping wires (i.e., the warp and the weft wires) are relatively spread out from each other, the low density of wires can result in the conductive mesh not providing sufficient heating to lyse cells captured in the filter. Thus, the openings in the wire mesh must be large enough to allow passage of particles (e.g., cells), but not so great that there is inadequate material to heat the cells captured in the filter. Taking these considerations into account, the inventors believe that openings in the wire mesh of about 40 µm to about 300 µm will be suitable for many applications.

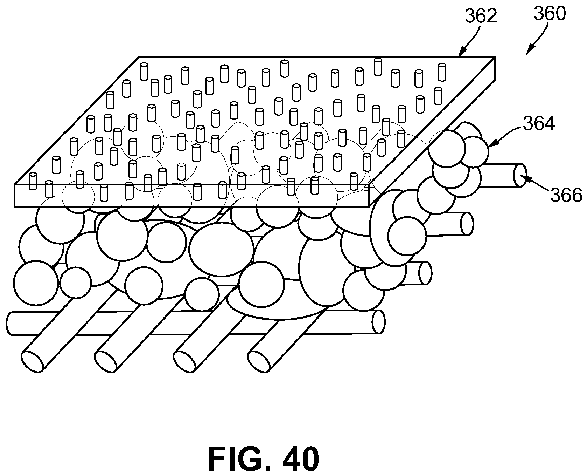

In an alternate embodiment, as shown in FIG. 40, the inductively heatable filter assembly 360 may include filter media comprising a perforated sheet 362, e.g., a track etched plate or membrane, on a downstream side of the filter assembly 360 overlapping a substrate 364 of plastic sintered particles (e.g., polyethylene, polypropylene, polytetrafluoroethylene, or polyvinyllidene fluoride) on an upstream side of the filter assembly 360. The track etched plate 362 (e.g., Oxyphen GmbH, Wetzikon, Switzerland or TRAKETCH^{®} membranes (PET/PET 0.2 VENT) (article number 090038) (SABEU GmbH & Co. KG, Northeim, Germany)) may have a pore size of about 0.2 µm and a thickness of about 22 µm, and the sintered plastic particle substrate 364 may have a pore size of 5 to 15 µm and a thickness of 450 to 600 µm. Exemplary Oxyphen filter media include those set forth in Table 1 below:

**Table 1**

| Filter | Type | Code | Pore size (µm) | Thickness (µm) |
|---|---|---|---|---|
| Unique-Mem | Track etched PET | 20.FX00.205.803 | 8 | 11 |
| Ro-Track | Fibers+Track-etched | 32.GX02. 328.202 | Pre-filter + 0.2 | 170 |
| Unique-Mem | Track-etched | 20.GX00 328 202 | 0.2 | 22 |

Exemplary sintered plastic substrates are available from Porex Filtration Group^{®}, Porex Corporation, Fairburn, Georgia, USA.

A conductive mesh 366 may comprise a metal woven wire mesh (e.g., a conductive material, such as, copper, stainless steel, or aluminum) having openings (e.g., between adjacent strands of the mesh) of about 60 µm and a thickness of about 130 µm to 350 µm and may be disposed so as to overlap the upstream side, the downstream side, or both the upstream and downstream sides of the filter media. A plate with small pores can also be produced by processes other than track etching, such as electrospinning or dry wet casting (for ultrafiltration - up to 1 nm of pores): polymer mixing with solvents (NMP, DMA) and non-solvents (H2O or EtOH or IPA).

In an alternate embodiment, as shown in FIG. 39, an inductively heatable filter assembly 370 may include a track etched plate 372 on a downstream side of the assembly 370 overlapping a substrate 374 of sintered metal fibers (e.g., a conductive material, such as, copper, stainless steel, or aluminum). The track etched plate 372 may have a pore size of about 0.2 µm and a thickness of 22 µm, and the sintered metal fiber substrate 374 may have a spacing between adjacent fibers of about 3 µm to 100 µm (e.g., about 7 µm being typical) and a thickness of about 270 µm. In this embodiment, the sintered metal fiber substrate 374 functions both as filter media and an inductively heatable conductive mesh.

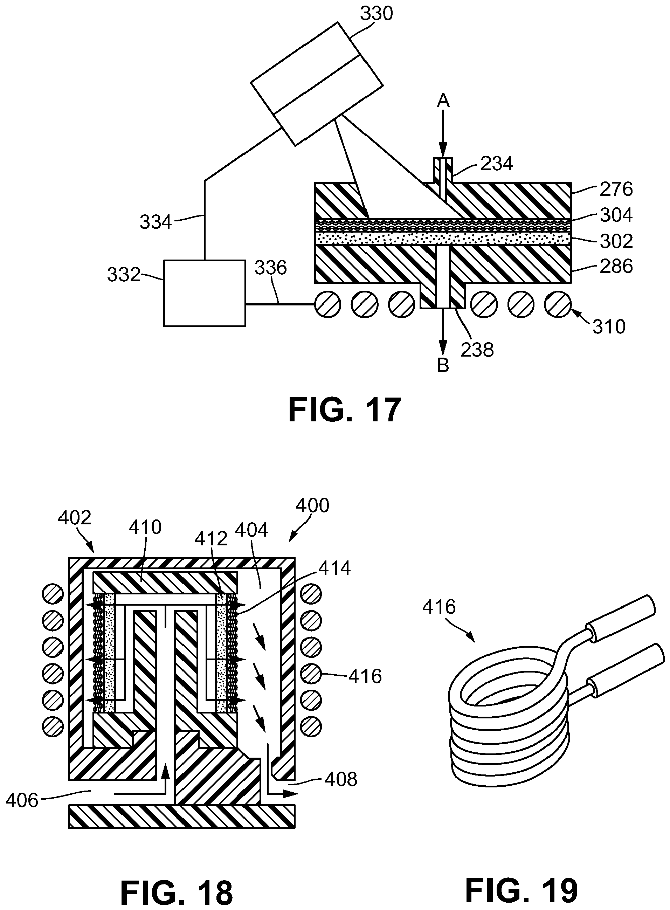

The basic functioning of an inductively heatable filter assembly is described with reference to FIG. 17, which is a schematic, transverse cross-sectional view of a filter assembly, filter housing, and induction coil along with a control system for controlling the induction coil. An inductively heatable filter assembly comprising filter media, such as a filter membrane 302 and a conductive mesh 304, is disposed within a filter chamber formed within a filter housing as represented by the filter chamber top wall 276 and the filter chamber bottom wall 286 (side walls extending between top wall 276 and bottom wall 286 are omitted to simplify the drawing). Fluid to be filtered is directed into the fluid inlet 234 formed in the top wall 276 in direction "A" and passes through the filter membrane 302 and conductive mesh 304 before exiting the filter chamber through the fluid outlet 238 in direction "B." An induction coil 310 is disposed outside the filter chamber adjacent to a wall of the filter housing. In the illustrated embodiment, the induction coil 310 is a flat coil adjacent the bottom wall 286 and surrounding the fluid outlet 238 and may be made from copper. The conductive mesh 304 and the induction coil 310 are positioned with respect to each other such that as the induction coil 310 is energized, i.e., alternating current is applied to the induction coil 310 via a relay for selectively allowing current from a driver to generate alternating electromagnetic fields, the conductive mesh 304 heats up, thereby applying heat to any material, such as cells, captured in the filter membrane 302. In various embodiments, the induction coil 310 is spaced from the conductive mesh 304 by about 1.5 mm to 4.0 mm (e.g., about 3 mm). In an exemplary embodiment, the conductive mesh is heated to a temperature of at least 80 ° C (80°C - 150°C) for a period of, e.g., 2 to 20 minutes, depending on the nature of the material to be lysed.

A control system may be provided to regulate the temperature of the conductive mesh 304. As shown in FIG. 17, in the illustrated embodiment, a temperature sensor 330 (e.g., a contactless temperature sensor, such as an infrared temperature sensor) monitors the temperature of the conductive mesh 304 and communicates the detected temperature to a controller 332 via a communication path 334 (wired or wireless). Controller 332 controls the amount of current applied to the coil 310 to control the strength of the alternating electromagnetic fields generated by the induction coil 310 as represented by control line 336 connecting the controller 332 to the inductive coil 310. Controller 332 may comprise a computer executing control software for controlling the strength of the alternating electromagnetic fields generated by the induction coil 310 based at least in part on temperature signals received from the temperature sensor 330 (i.e., part of a temperature feedback control circuit). Controller 332 may incorporate or be in controlling communication with a power source connected to the induction coil and configured to pass alternating current through the induction coil 310 to generate alternating electromagnetic fields in the induction coil 310 or otherwise energize the induction coil 310. In an embodiment, current provided to the induction coil may be pulsed - for example to avoid overheating plastic components where high lysing temperatures are required. Controller 332 may be programmed to control the period and magnitude of the pulsed current to achieve a desired temperature, with feedback from the temperature sensor 330. A suitable controller includes a DC12-30V ZVS Tesla Coil SGTC marx generator high voltage driver plate board arc igniter induction heating power module no taps (ICStation (www.icstation.com)). A suitable temperature sensor includes an RS PRO V Output Signal Infrared Temperature Sensor (RS Components Ltd. Corby, UK).

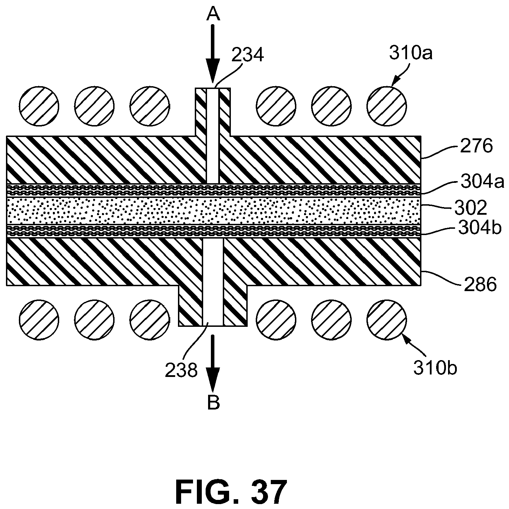

An alternate configuration of a filter assembly is shown in FIG. 37. An inductively heatable filter assembly is disposed within a filter chamber formed within a filter housing as represented by the filter chamber top wall 276 and the filter chamber bottom wall 286 (side walls extending between top wall 276 and bottom wall 286 are omitted to simplify the drawing). The inductively heatable filter assembly comprises filter media, such as a filter membrane 302, and two conductive meshes 304a, 304b disposed, respectively, above and below the filter media 302 (i.e., conductive mesh 304a overlies filter media 302 and conductive mesh 304b underlies filter media 302). An induction coil 310b is disposed outside the filter chamber adjacent to a wall of the filter housing. In the illustrated embodiment, the induction coil 310b is a flat coil adjacent the bottom wall 286 and surrounding the fluid outlet 238 and is configured to cause mesh 304a and mesh 304b to heat up when the flat coil energized. An alternate embodiment includes an additional induction coil 310a adjacent the top wall 276 and surrounding the fluid inlet 234. Induction coil 310a is configured to cause mesh 304a to heat up when energized (and may also cause mesh 304b to heat up if the coil 310a and mesh 304b are in sufficient proximity), and induction coil 310b is configured to cause mesh 304b to heat up when energized (and may also cause mesh 304a to heat up if the coil 310a and mesh 304b are in sufficient proximity). To avoid possible interference between coils 310a and 310b, the coils could be energized in alternating fashion, so both coils are never energized at the same time. Fluid to be filtered is directed into the fluid inlet 234 formed in the top wall 276 in direction "A" and passes through conductive mesh 304a, filter membrane 302, and conductive mesh 304b before exiting the filter chamber through the fluid outlet 238 in direction "B."

Aspects of an alternate configuration of a filter assembly and associated induction coil are shown in FIGS. 18 and 19. FIG. 18 illustrates a schematic cross-section of a filter assembly 400 including a filter housing 402 defining a filter chamber 404 with a fluid inlet 406 and a fluid outlet 408. The fluid inlet 406 is in communication with an internal plenum 410. Filter media, which may comprise a filter element 412 that may be cylindrical or generally cylindrical, surrounds the plenum 410, and a conductive mesh 414 that may be cylindrical or generally cylindrical, surrounds the filter element 412. In one embodiment, the conductive mesh 414 is disposed radially outside the filter element 412 so as to at least partially encompass, or surround, the filter media. A cylindrical induction coil 416 (see FIG. 19) is disposed outside the filter housing 402. Sample material enters the filter housing 402 through the fluid inlet 406 and enters the plenum 410, which distributes the fluid internally of the cylindrical filter element 412 and cylindrical conductive mesh 414. Fluid distributed by the plenum 410 then passes through both the filter element 412 and the mesh 414 and exits the filter housing 402 through the filter outlet 408, which is located radially outwardly from the conductive mesh 414. As alternating electromagnetic fields are generated by the induction coil 416, the conductive mesh 414 heats up, thereby applying heat to cellular material captured by the filter element 412.

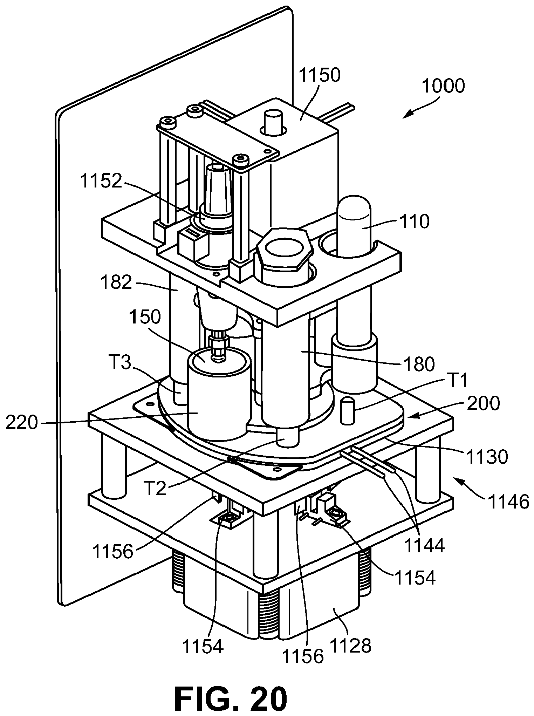

The above-described sample processing cartridge 100 or 200 may be inserted in a docking station 1000, a perspective view of which is shown in FIG. 20. Docking station 1000 includes a pump actuator (for example, where the pump comprises a syringe (such as syringe 150), a mechanism for actuating the syringe plunger 160), a valve actuator for selectively actuating one or more of valves V1-V7 (e.g., actuating one or more, but not all, valves at a time), and an induction coil for inductively heating a conductive mesh of an inductively heatable filter assembly. Docking station 1000 is specifically configured to operate sample processing cartridge 200. Variations of the docking station are contemplated to operate different sample processing cartridge configurations.

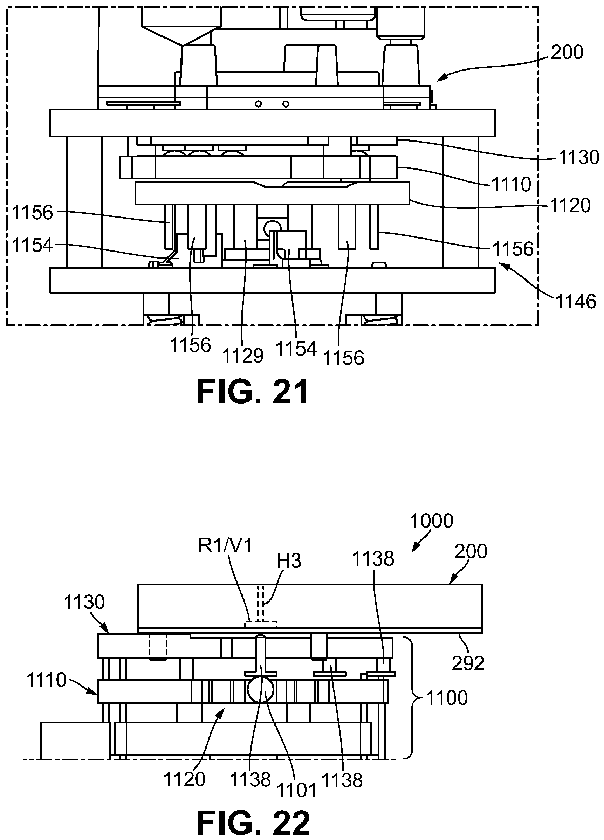

In the illustrated embodiment, as shown in FIGS. 20 and 21, sample processing cartridge 200 is operatively positioned within the docking station 1000, supported by a dock 1146, which, in the illustrated embodiment, comprises upper and lower plates connected together in a spaced-apart arrangement by spacers. A sample container 110 is coupled to first functional connection T1, a first process fluid container 180 is coupled to second functional connection T2, and a third process fluid container 182 is coupled to third functional connection T3. Although not visible in FIG. 20, a fourth process fluid container may be coupled to fourth functional connection T4, a waste container may be coupled to fifth functional connection T5, and a collection container may be coupled to sixth functional connection T6.

A mechanism for actuating a syringe 150 may include a syringe actuator 1152 connected to syringe and configured to selectively move the syringe up and down within the syringe barrel 220 and powered by a syringe actuator motor 1150. As noted above, the syringe, such as syringe 150, may be part of the docking station 1000, and the syringe seal, such as syringe seal 164, may be a component of the cartridge 100 or 200.

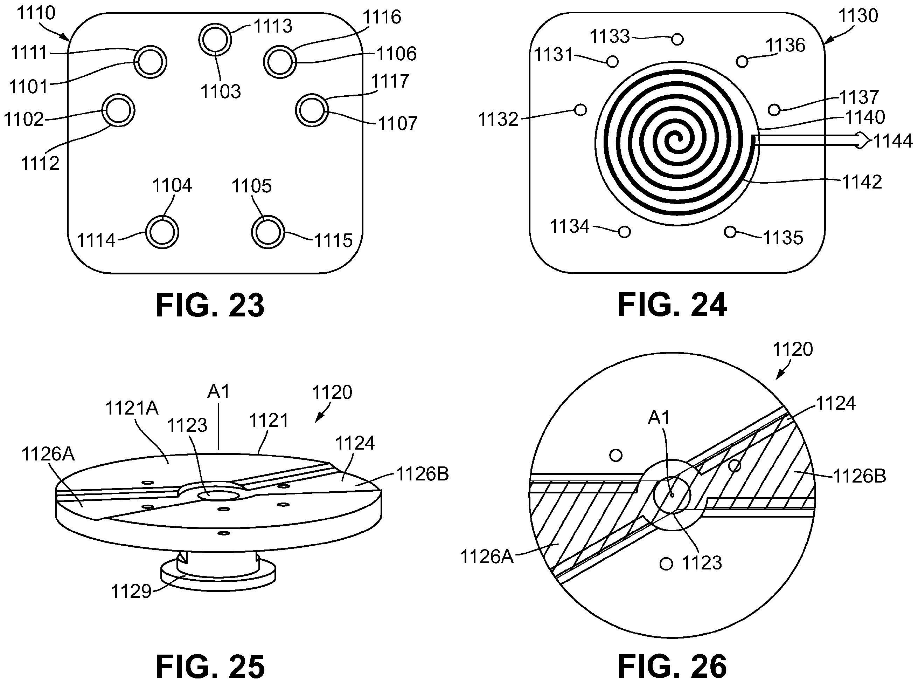

The valve actuator of docking station 1000 includes a rotational-motion cam-driven actuator 1100, more specifically shown in FIGS. 21-26. The cam-driven actuator 1100 includes a cam 1120 (see FIGS. 25-26), which, in the illustrated embodiment, includes a cylindrical, or disc-shaped, part 1121. Cam 1120 includes a first surface 1121A and a central opening 1123 and is rotatable about an axis of rotation A1, e.g., by a motor 1128 (see FIG. 20) that rotates a drive hub 1129 coaxially coupled to the central opening 1123.

Cam 1120 further includes on first surface 1121A a cam recess 1124 (represented by cross-hatching in FIG. 26) extending along two radii of the cylindrical part 1121 on diametrically opposed sides of the central opening 1123. The profile of this cam recess 1124 includes bottom surfaces 1126A and 1126B on the opposed sides of the central opening 1123.

The cam-driven actuator 1100 also includes an actuating ball guide plate 1110 (see FIGS. 21-23) with seven guide holes 1111-1117 arranged circularly and extending perpendicularly through the guide plate 1110. Guide holes 1111-1117 are configured to guide seven actuating balls 1101-1107 of the cam-driven actuator 1100 (see FIG. 22, in which one actuating ball 1101 is shown, and FIG. 23), each actuating ball 1101-1107 having a ball diameter allowing the ball to pass through an associated one of the guide holes 1111-1117.

As shown in FIGS. 22 and 23, the guide plate 1110 of the rotational-motion cam-driven actuator 1100 is located above the cam 1120 so that the actuating balls 1101-1107 rest on the first surface 1121A of the cam 1120.

The diameter of each the guide hole 1111-1117, the diameter of each associated actuating ball 1101-1107, and the thickness of the guide plate 1110 are selected so that:
- Where the cam 1120 is rotationally positioned so that an actuating ball 1101-1107 rests on a planar part of the first surface 1121A of the cam 1120 (as in FIG. 22), the actuating ball, maintained in place by its corresponding guide hole 1111-1117, projects above a top surface of the guide plate 1110; and
- Where the cam 1120 is rotationally positioned so that an actuating ball 1101-1107 rests on bottom surface 1126A or 1126B of the cam recess 1124 of the cam 1120, the actuating ball, guided by its corresponding guide hole 1111-1117, is recessed relative to the top surface of the guide plate 1110 as compared to when the actuating ball rests on the first surface 1121A.

Therefore, with rotational motion of the cam 1120 around the axis of rotation A1, each actuating ball 1101-1107, guided by its corresponding guide hole 1111-1117, moves in a direction parallel to the axis of rotation A1 between an engaged position where the actuating ball 1101-1107 contacts surface 1121a of the cam 1120 and projects from the guide hole 1111-1117 (i.e., above the top surface of the guide plate 1110) and a disengaged position where the actuating ball 1101-1107 contacts bottom surface 1126A or 1126B of the cam recess 1124 and is recessed compared to its engaged position.

In an embodiment as shown in FIGS. 25 and 26, where recess 1124 extends diametrically to opposed sides of the central recess 1123, at most two of the actuating balls 1101-1107 can be in a disengaged position while the other actuating balls are in an engaged position.

As shown in FIG. 22, the cam actuator 1100 includes a series of external actuators in the form of plungers corresponding in number to the number of valves included in the sample processing cartridge. The illustrated embodiment includes seven plungers 1138 for actuating the illustrated sample processing cartridge 200, each located above a corresponding actuating ball 1101-1107. Cam actuator 1100 further includes a plunger guide plate 1130 including plunger guide holes 1131-1137 (see FIG. 24) formed therethrough for guiding each of the plungers 1138 in their translation motion.

As shown in FIGS. 22 and 23, the plunger guide plate 1130 is situated directly below the processing cartridge 200. As shown in FIG. 24, plunger guide plate 1130 includes an opening 1140 within which is disposed an induction coil 1142 that will be positioned outside of and in close proximity to the filter chamber of the processing cartridge 200 within the docking station 1000. As noted, the induction coil may be made from copper. Exemplary dimensions include a diameter of 43.0 ± 1.0 mm and a thickness of about 1.3 mm, and a suitable inductive coil includes a pancake coil (WT505090-10K2-A11-G) (TDK Corporation, Tokyo, Japan).

Power to induction coil 1142 (for generating alternating electromagnetic fields) may be carried from a power source on power lines, such as lines 1144 shown in FIG. 24.

In the cam-driven actuator 1100, the cylindrical holes 1111-1117, the actuating balls 1101-1107, and the plungers 1138 are arranged circularly at equal radial distances from the axis of rotation A1. The seven valves V1 to V7 of sample processing cartridge 200 are arranged so as to be mechanically actuated together by the cam-driven actuator 1100. More precisely, valves V1 to V7 are arranged so that there is an associated plunger 1138, an associated guide hole 1111-1117, and an associated actuating ball 1101-1107 opposite each of the valves V1 to V7.

Accordingly, when an actuating ball 1101 is in an engaged position, it places the corresponding plunger 1138 also in an engaged position where it exerts pressure on a portion of the bottom material 292 of the sample processing cartridge 200 disposed over the recess R1 and valve seat of the valve V1, so as to deform the bottom material 292 against the valve seat to seal the corresponding through hole H3, thus closing the valve V1. And when an actuating ball 1101 is in a disengaged position (as shown in FIG. 17), the plunger 1138 is in a retracted, disengaged position where it does not exert pressure to the portion of bottom material 292 over the recess R1, so that the material 292 is at rest opposite the valve seat of recess R1 of the valve V1 and the corresponding through hole H3 is not blocked and the valve V1 is then open.

The cam-driven actuator 1100 of docking station 1000 may include a control system for controlling operation of motor 1128 so as to control the position of cam 1120 and control (i.e., select) which of valves V1 to V7 is in an open or closed position. As shown in FIGS. 20 and 21, in an embodiment, docking station 1000 may include one or more optical sensors 1154 (e.g., slotted optical sensors mounted on a top surface of the lower plate of the dock 1146) that detect each of one or more sensor flags 1156 projecting below the cam 1120. Passage of a sensor flag 1156 by a sensor 1154 is detected by the sensor 1154, to thereby detect or confirm a specific rotational position of the cam 1120. Docking station 1000 may include other sensors for the control system, such as a position and force sensors (not shown) for the syringe and/or syringe actuator 1152. Detecting the force applied to the syringe may be useful to detect any possible clogging in the syringe.

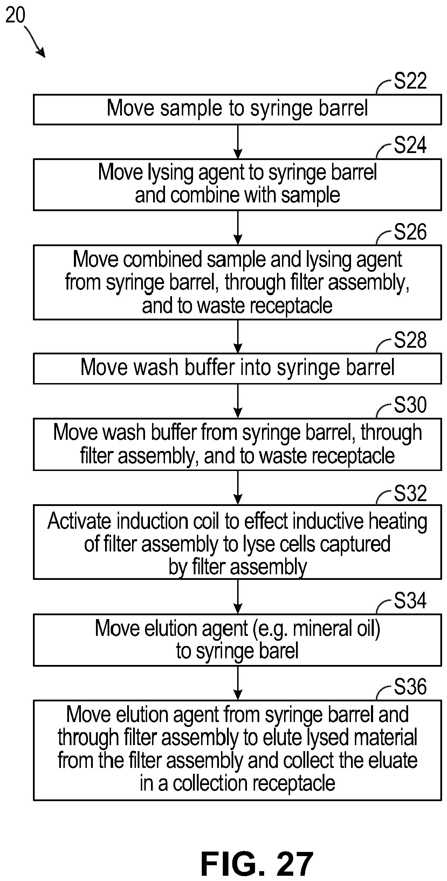

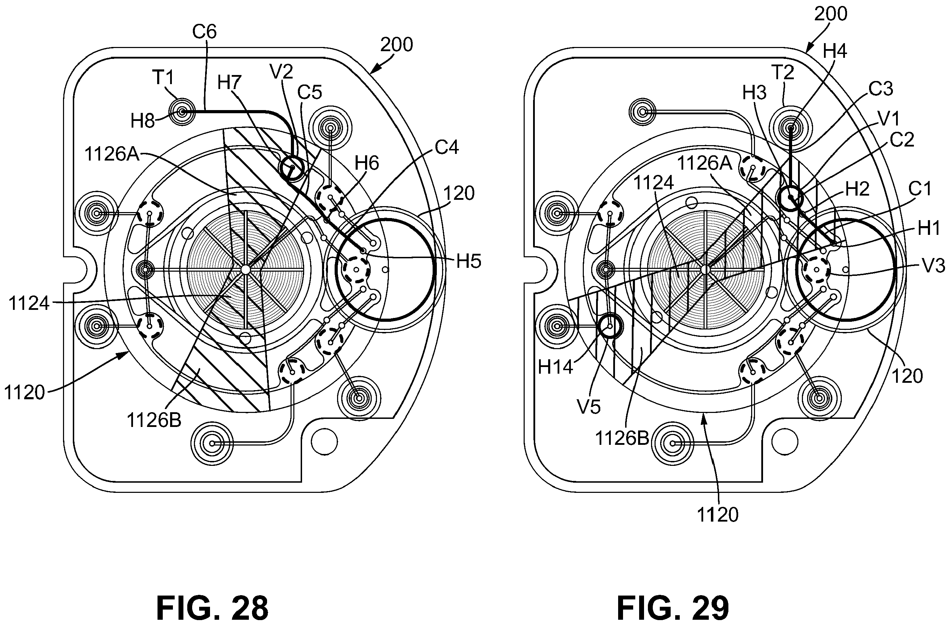

An exemplary operation of the sample processing cartridge 200 - i.e., selective operation of the valves V1-V7 and movement of sample, buffer, and other fluids within the cartridge - is described with reference to FIGS. 28-35. FIG. 27 is a flow chart illustrating a process 20 for processing (lysing) a sample using the sample processing cartridge 200. This exemplary process includes the following set up. The sample processing cartridge 200 is placed in docking station 1000, a sample container (e.g., Vacutainer^{®} blood collection tube) is coupled to functional connection T1, a receptacle containing a lysing solution, such as a hemolytic solution, is coupled to functional connection T2, a receptacle containing mineral oil is coupled to functional connection T3, a receptacle containing wash/lysis buffer is coupled to functional connection T4, a waste receptacle is coupled to functional connection T5, and a fluid outlet, e.g., eluate collection, receptacle is coupled to functional connection T6. The actuating balls 1101-1107 are initially in their engaged positions so that each of the valves V1-V7 is in a closed condition. A suitable hemolytic solution is described in U.S. Patent No. 11,162,091, to Gao et al., entitled "Blood Cell Lysis Reagent." In one example, a hemolytic solution is a Tris-buffered hemolysis solution comprising 4 M guanidine hydrochloride + 4% v/v polysorbate 20 (Tween-20) + 4% w/v Saponin + 40.5 mM Tris-HCI.

In a first step S22 shown in FIG. 27, sample is moved to the syringe barrel from a sample container. As illustrated in FIG. 28, cam 1120 has been rotated by motor 1128 to position bottom surface 1126A of cam recess 1124 beneath valve V2 so that the actuating ball 1102 and the associated plunger 1138 within plunger guide hole 1132 corresponding to valve V2 are in their disengaged positions and valve V2 is in an open condition (actuating ball 1102, plunger 1138, and plunger guide hole 1132 are not shown in FIG. 28). Accurate positioning of the cam 1120 may be confirmed by a control system controlling motor 1128 in accordance with one or more signals received from the one or more optical sensors 1154 detecting the one or more sensor flags 1156 projecting below the cam 1120. With valve V2 open, through hole H7 is open and the sample receptacle connected to through hole H8 at functional connection T1 is in fluid communication with through hole H5 within syringe barrel 120 by the channel formed by channel segments C4 and C5 connected by through hole H6 and channel segments C5 and C6 connected by through hole H7. Syringe actuator 1152 is driven by syringe actuator motor 1150 to actuate the syringe and draw sample from the sample container at functional connect T1 into the syringe barrel 120 (syringe actuator 1152 and syringe actuator motor 1150 are not shown in FIG. 28).

In a second step S24 shown in FIG. 27, lysing agent is moved to the syringe barrel from a first fluid reservoir and combined with sample. As illustrated in FIG. 29, cam 1120 has been rotated by motor 1128 to position bottom surface 1126A of cam recess 1124 beneath valve V1 so that the actuating ball 1101 and the associated plunger 1138 within plunger guide hole 1131 corresponding to valve V1 are in their disengaged positions and valve V1 is in an open condition (actuating ball 1101, plunger 1138, and plunger guide hole 1131 are not shown in FIG. 29). Accurate positioning of the cam 1120 may be confirmed by a control system controlling motor 1128 in accordance with one or more signals received from the one or more optical sensors 1154 detecting the one or more sensor flags 1156 projecting below the cam 1120. With valve V1 open, through hole H3 is open and the process fluid receptacle (e.g., containing a lysing solution, such as a hemolytic solution) connected to through hole H4 at functional connection T2 is in fluid communication with through hole H1 within syringe barrel 120 by the channel formed by channel segments C1 and C2 connected by through hole H2 and channel segments C2 and C3 connected by through hole H3. Syringe actuator 1152 is driven by syringe actuator motor 1150 to actuate the syringe and draw process fluid (e.g., hemolytic solution or hemolysis) from the process fluid receptacle at functional connect T2 into the syringe barrel 120 (syringe actuator 1152 and syringe actuator motor 1150 are not shown in FIG. 29).

Note also in FIG. 29 that bottom surface 1126B of cam recess 1124 is positioned beneath valve V5 so that the actuating ball 1105 and the associated plunger 1138 within plunger guide hole 1135 corresponding to valve V5 are in their disengaged positions and valve V5 is in an open condition (actuating ball 1105, plunger 1138, and plunger guide hole 1135 are not shown in FIG. 29). Nevertheless, no fluid will flow through hole H14 corresponding to valve V5 since the channel connecting hole H14 to the syringe barrel 120 is closed by valve V3.

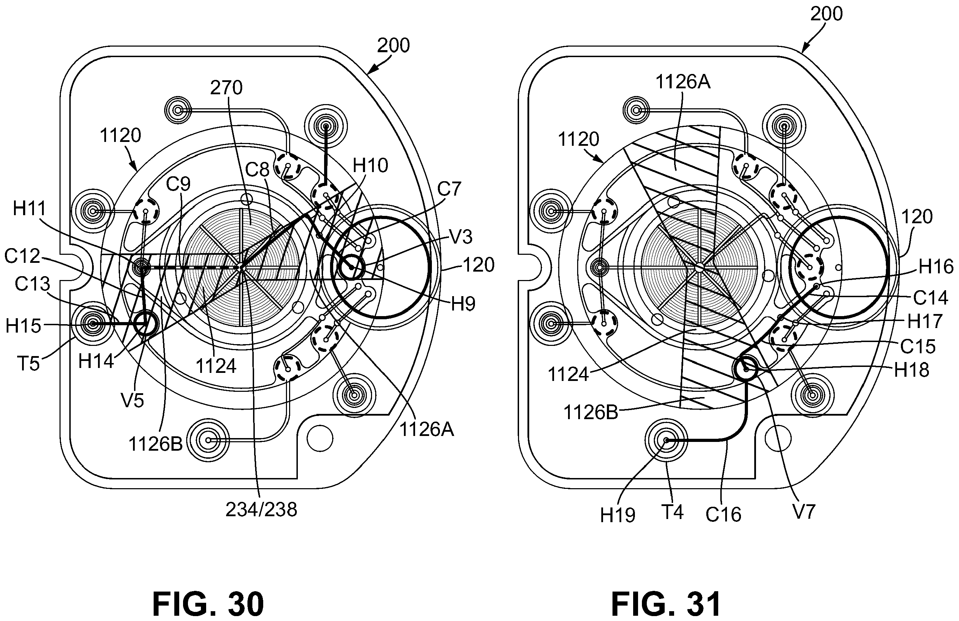

In a third step S26 shown in FIG. 27, the combined sample and lysing agent is moved from the syringe barrel, through the filter assembly, and to the waste receptacle. As illustrated in FIG. 30, cam 1120 has been rotated by motor 1128 to position bottom surface 1126A of cam recess 1124 beneath valve V3 so that the actuating ball 1103 and the associated plunger 1138 within plunger guide hole 1133 corresponding to valve V3 are in their disengaged positions and valve V3 is in an open condition (actuating ball 1103, plunger 1138, and plunger guide hole 1133 are not shown in FIG. 30). In addition, bottom surface 1126B of cam recess 1124 is positioned beneath valve V5 so that the actuating ball 1105 and the associated plunger 1138 within plunger guide hole 1135 corresponding to valve V5 are in their disengaged positions and valve V5 is in an open condition (actuating ball 1105, plunger 1138, and plunger guide hole 1135 are not shown in FIG. 30). Accurate positioning of the cam 1120 may be confirmed by a control system controlling motor 1128 in accordance with one or more signals received from the one or more optical sensors 1154 detecting the one or more sensor flags 1156 projecting below the cam 1120. With valves V3 and V5 open, through holes H9 and H14 are open, and the waste receptacle connected to through hole H15 at functional connection T5 is in fluid communication with through hole H9 within syringe barrel 120 by the channel formed by channel segments C7 and C8 connected by through hole H10, channel segment C8 and filter chamber 270 connected by filter chamber fluid inlet 234, filter chamber 270 and channel segment C9 connected by filter chamber fluid outlet 238, channel segments C9 and C12 connected by through hole H11, and channel segments C12 and C13 connected by through hole H14. Syringe actuator 1152 is driven by syringe actuator motor 1150 to actuate the syringe and expel the mixture of sample and first process fluid (e.g., a hemolytic solution) from the syringe barrel and propel the mixture through the filter chamber 270 where the mixture will pass through the filter assembly contained therein, and into the waste receptacle coupled to functional connection T5 (syringe actuator 1152 and syringe actuator motor 1150 are not shown in FIG. 30).

In a fourth step S28 shown in FIG. 27, wash buffer is moved to the syringe barrel from a second fluid reservoir. As illustrated in FIG. 31, cam 1120 has been rotated by motor 1128 to position bottom surface 1126B of cam recess 1124 beneath valve V7 so that the actuating ball 1107 and the associated plunger 1138 within plunger guide hole 1137 corresponding to valve V7 are in their disengaged positions and valve V7 is in an open condition (actuating ball 1107, plunger 1138, and plunger guide hole 1137 are not shown in FIG. 31). Accurate positioning of the cam 1120 may be confirmed by a control system controlling motor 1128 in accordance with one or more signals received from the one or more optical sensors 1154 detecting the one or more sensor flags 1156 projecting below the cam 1120. With valve V7 open, through hole H18 is open, and the process fluid receptacle (e.g., containing wash buffer) connected to through hole H19 at functional connection T4 is in fluid communication with through hole H16 within syringe barrel 120 by the channel formed by channel segments C14 and C15 connected by through hole H17 and channel segments C15 and C16 connected by through hole H18. Syringe actuator 1152 is driven by syringe actuator motor 1150 to actuate the syringe and draw process fluid (e.g., wash buffer) from the process fluid receptacle at functional connect T4 into the syringe barrel 120 (syringe actuator 1152 and syringe actuator motor 1150 are not shown in FIG. 31).

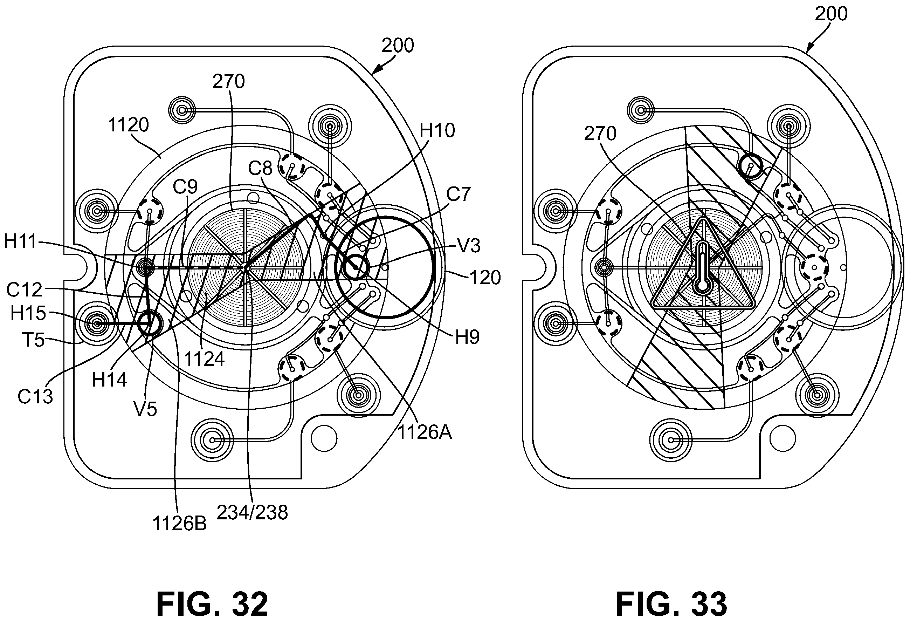

In a fifth step S30 shown in FIG. 27, wash buffer is moved from the syringe barrel, through the filter assembly, and to the waste receptacle. As illustrated in FIG. 32, cam 1120 has been rotated by motor 1128 to position bottom surface 1126A of cam recess 1124 beneath valve V3 so that the actuating ball 1103 and the associated plunger 1138 within plunger guide hole 1133 corresponding to valve V3 are in their disengaged positions and valve V3 is in an open condition (actuating ball 1103, plunger 1138, and plunger guide hole 1133 are not shown in FIG. 32). In addition, bottom surface 1126B of cam recess 1124 is positioned beneath valve V5 so that the actuating ball 1105 and the associated plunger 1138 within plunger guide hole 1135 corresponding to valve V5 are in their disengaged positions and valve V5 is in an open condition (actuating ball 1105, plunger 1138, and plunger guide hole 1135 are not shown in FIG. 32). Accurate positioning of the cam 1120 may be confirmed by a control system controlling motor 1128 in accordance with one or more signals received from the one or more optical sensors 1154 detecting the one or more sensor flags 1156 projecting below the cam 1120. With valves V3 and V5 open, through holes H9 and H14 are open, and the waste receptacle connected to through hole H15 at functional connection T5 is in fluid communication with through hole H9 within syringe barrel 120 by the channel formed by channel segments C7 and C8 connected by through hole H10, channel segment C8 and filter chamber 270 connected by filter chamber fluid inlet 234, filter chamber 270 and channel segment C9 connected by filter chamber fluid outlet 238, channel segments C9 and C12 connected by through hole H11, and channel segments C12 and C13 connected by through hole H14. Syringe actuator 1152 is driven by syringe actuator motor 1150 to actuate the syringe and expel the third process fluid (wash buffer) from the syringe barrel, through the filter chamber 270, where the mixture will pass through the filter assembly contained therein to wash the material previously captured in the filter assembly, and into the waste receptacle coupled to functional connection T5 (syringe actuator 1152 and syringe actuator motor 1150 are not shown in FIG. 32).

In a sixth step S32 shown in FIG. 27, an induction coil is activated to effect inductive heating of filter assembly to lyse cells captured by filter assembly. As illustrated in FIG. 33, the induction coil 1142 within plunger guide plate 1130 of the docking station 1000 (see FIG. 24) is activated with alternating current passing through the coil 1142 to generate alternating current electromagnetic fields that heat the conductive mesh of the filter assembly within the filter chamber 270 thereby heating the filter media within the filter chamber 270 to lyse cells captured in the filter media.

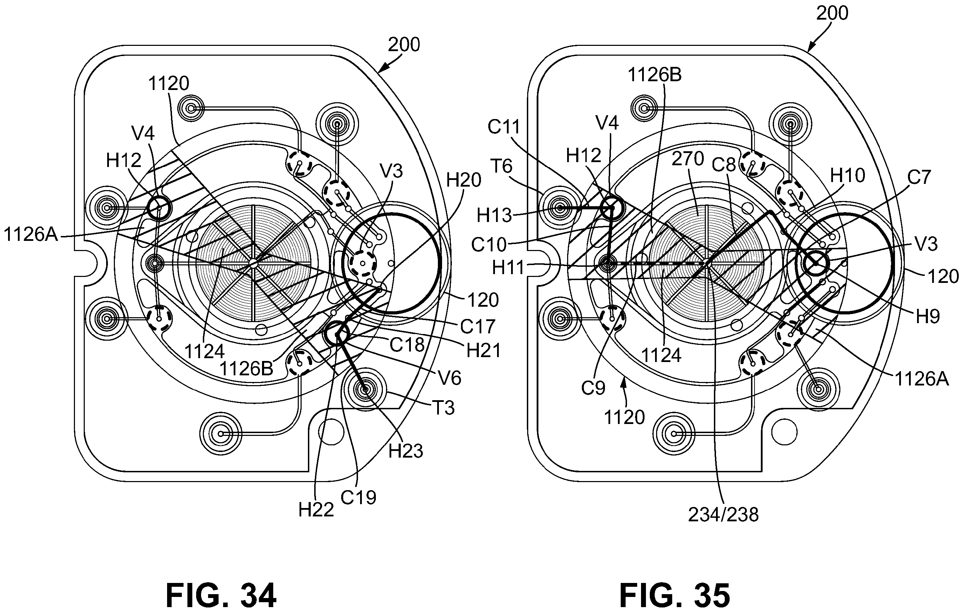

In a seventh step S34 shown in FIG. 27, elution agent is moved to the syringe barrel from a third fluid reservoir. As illustrated in FIG. 34, cam 1120 has been rotated by motor 1128 to position bottom surface 1126B of cam recess 1124 beneath valve V6 so that the actuating ball 1106 and the associated plunger 1138 within plunger guide hole 1136 corresponding to valve V6 are in their disengaged positions and valve V6 is in an open condition (actuating ball 1106, plunger 1138, and plunger guide hole 1136 are not shown in FIG. 34). Accurate positioning of the cam 1120 may be confirmed by a control system controlling motor 1128 in accordance with one or more signals received from the one or more optical sensors 1154 detecting the one or more sensor flags 1156 projecting below the cam 1120. With valve V6 open, through hole H22 is open and the process fluid receptacle (e.g., containing mineral oil) connected to through hole H23 at functional connection T3 is in fluid communication with through hole H20 within syringe barrel 120 by the channel formed by channel segments C17 and C18 connected by through hole H21 and channel segments C18 and C19 connected by through hole H22. Syringe actuator 1152 is driven by syringe actuator motor 1150 to actuate the syringe and draw process fluid (e.g., mineral oil) from the process fluid receptacle at functional connect T3 into the syringe barrel 120 (syringe actuator 1152 and syringe actuator motor 1150 are not shown in FIG. 34).

Note also in FIG. 34 that bottom surface 1126A of cam recess 1124 is positioned beneath valve V4 so that the actuating ball 1104 and the associated plunger 1138 within plunger guide hole 1134 corresponding to valve V4 are in their disengaged positions and valve V4 is in an open condition. Nevertheless, no fluid will flow through hole H12 corresponding to valve V4 since the channel connecting hole H12 to the syringe barrel 120 is closed by valve V3 (actuating ball 1104, plunger 1138, and plunger guide hole 1134 are not shown in FIG. 34).

In an eighth step S36 shown in FIG. 27, elution agent is moved from syringe barrel and through filter assembly, to elute lysed material from the filter assembly and collect the eluate in a collection receptacle. As illustrated in FIG. 35, cam 1120 has been rotated by motor 1128 to position bottom surface 1126A of cam recess 1124 beneath valve V3 so that the actuating ball 1103 and the associated plunger 1138 within plunger guide hole 1133 corresponding to valve V3 (actuating ball 1103, plunger 1138, and plunger guide hole 1133 are not shown in FIG. 35) are in their disengaged positions and valve V3 is in an open condition. In addition, bottom surface 1126B of cam recess 1124 is positioned beneath valve V4 so that the actuating ball 1104 and the associated plunger 1138 within plunger guide hole 1134 corresponding to valve V4 are in their disengaged positions and valve V4 is in an open condition (actuating ball 1104, plunger 1138, and plunger guide hole 1134 are not shown in FIG. 35). Accurate positioning of the cam 1120 may be confirmed by a control system controlling motor 1128 in accordance with one or more signals received from the one or more optical sensors 1154 detecting the one or more sensor flags 1156 projecting below the cam 1120. With valves V3 and V4 open, through holes H9 and H12 are open, and the collection receptacle connected to through hole H13 at functional connection T6 is in fluid communication with through hole H9 within syringe barrel 120 by the channel formed by channel segments C7 and C8 connected by through hole H10, channel segment C8 and filter chamber 270 connected by filter chamber fluid inlet 234, filter chamber 270 and channel segment C9 connected by filter chamber fluid outlet 238, channel segments C9 and C10 connected by through hole H11, and channel segments C10 and C11 connected by through hole H112. Syringe actuator 1152 is driven by syringe actuator motor 1150 to actuate the syringe and expel the second process fluid (e.g., mineral oil) from the syringe barrel, through the filter chamber 270, where the mineral oil passes through the filter assembly contained therein to elute the lysed material captured in the filter assembly, and into the collection receptacle coupled to functional connection T6 (syringe actuator 1152 and syringe actuator motor 1150 are not shown in FIG. 35). In one embodiment, the volume of the filter chamber 270 may be about 300 µl, so that about 300 µl of fluid is collected during this eighth step.

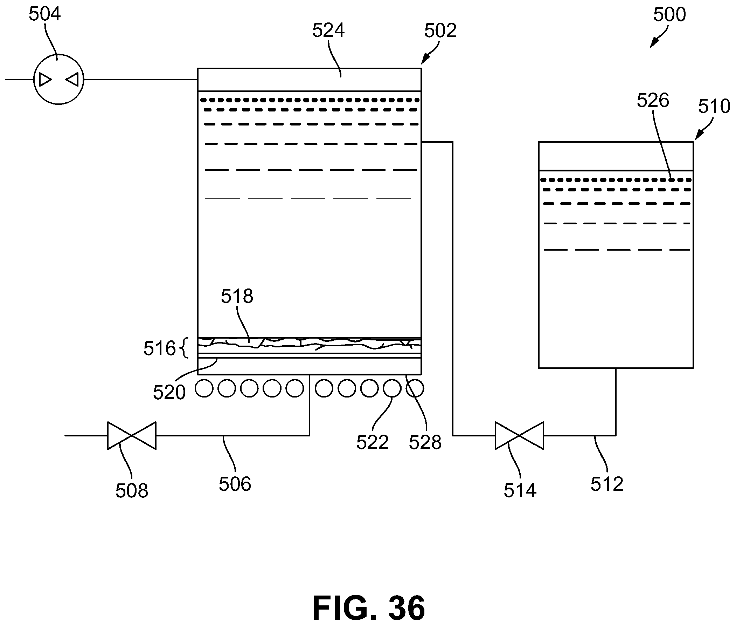

FIG. 36 shows an alternative system 500 for separating cells from a sample and for lysing the separated cells. System 500 includes an integrated sample reservoir/filter chamber 502, configured to receive and hold a sample fluid 524, and an outlet fluid path 506 through which the sample fluid 524 may be removed from the reservoir 502. Outlet path 506 may comprise a channel or conduit and may incorporate a valve 508 for selectively controlling flow through the path 506, and outlet path 506 may be connected to a downstream chamber and/or processing system, such as a waste chamber and/or an amplification/detection cartridge (not shown in FIG. 36). System 500 may further include an inductively heatable filter assembly 516 positioned within the integrated sample reservoir/filter chamber 502 so that sample fluid exiting the reservoir/filter chamber 502 via the outlet path 506 passes through the filter assembly 516. Thus, unlike embodiments described above in which the filter assembly is disposed within a filter chamber that is in communication with, but distinct from, a sample reservoir or sample container, system 500 includes an integrated sample reservoir and filter chamber.

Inductively heatable filter assembly 516 may include filter media 518 (e.g., a filter membrane or membranes, sintered materials, a track etched plate, etc.) and one or more conductive meshes 520. Conductive mesh 520 may underlie filter media 518 as shown in FIG. 36, conductive mesh 520 may overlie filter media 518, or conductive mesh 520 may overlie and underlie filter media 518. System 500 may further include an inductive coil 522 positioned with respect to a wall 528 of the integrated sample reservoir/filter chamber 502 so that when the coil 522 is energized with alternating currents to generate alternating electromagnetic fields, the conductive mesh(es) 520 will heat up, thereby heating the filter media 518 and any materials captured by the filter media 518. System 500 may further include a pump 504 (which may comprise a syringe or other source of positive and/or negative pressure) in communication with the sample reservoir/filter chamber 502 for sending sample fluid 524 out of the reservoir 502 through the outlet path 506.

System 500 may further include one or more reservoirs 510 containing a process fluid 526 and connected via a fluid path 512 to the integrated sample reservoir/filter chamber 502. Fluid path 512 may comprise a channel or conduit and may incorporate a valve 514 for controlling fluid flow through the fluid path 512. Reservoir 510 may contain mineral oil or other elution buffers for removing lysed materials from the filter media 518 after a thermal lysing procedure. Process fluid may be drawn into the reservoir 502 by opening valve 514, closing valve 508, and operating pump 504 in reverse to draw process fluid 526 from the reservoir 510, through the fluid path 512, and into the reservoir/filter chamber 502. Process fluid is passed through the filter assembly 516 by closing valve 514, opening valve 508, and operating pump 504 in a forward direction to force process fluid 526 from the reservoir 502, through the filter assembly 516, and through the fluid path 506.

Valve 508 and/or valve 514 may be configured like valves V1-V7 described above, with a flexible material disposed over a recess formed in a surface of a body concentrically with a hole through the body so that localized deflection or deformation of the material opposite the valve seat of the recess presses the material into contact with the valve seat, i.e., the annular surface at the base of the recess surrounding the through hole, thereby blocking fluid flow through the corresponding through hole.

### Fluidics Schematics

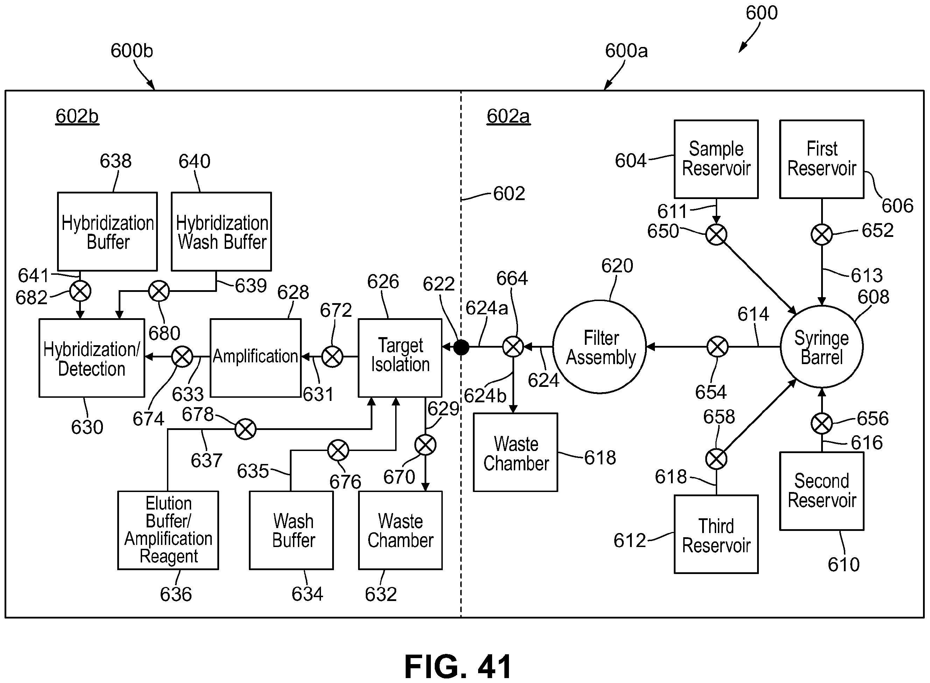

FIG. 41 is a schematic, block diagram of a fluidic system as described herein. System 600 may comprise a fluidic cartridge (e.g., a microfluidic cartridge) configured to be operably coupled to a processing device, or docking station, (not shown) and including a substrate which may include a first substrate portion 602a that contains components of a first fluidic system 600a for separating cellular material (analyte) from a sample by filter media and a second substrate portion 602b that contains components of a second fluidic system 600b for analyzing cellular material, e.g., to amplify an analyte and/or detect the presence of an analyte of interest. Referring to the disclosure above, first substrate portion 602a may correspond to body 102 of sample processing cartridge 100 or body 202 of sample processing cartridge 200, and second substrate 602b may correspond to reaction cartridge 320. A dashed line 602 separates first substrate portion 602a and first fluidic system 600a from second substrate portion 602b and second fluidic system 600b. Dashed line 602 signifies that first substrate portion 602a and second substrate portion 602b could comprise a single, contiguous substrate, or first substrate portion 602a and second substrate portion 602b could be separate, discrete substrates (e.g., separate cartridges) that can be fluidically coupled to one another, for example, at fluid coupling 622, which may correspond to coupling hood 640 and process cartridge coupling port 322 described above.

First substrate portion 602a and second substrate portion 602b may be made from suitable materials that can be molded and/or machined to form features of the first and second fluidic systems 600a, 600b and which does not adversely react with sample or reaction process materials. Suitable materials include thermoplastic polymer material, such as cyclic olefin copolymers (COC) or cyclic olefin polymers (COP), or any thermoplastic polymer suitable for injection molding. In one embodiment, first substrate portion 602a and second substrate portion 602b may be made of polypropylene (PP). Other exemplary materials may be chosen from the group including polycarbonate, polyacrylamide, polyethylene, polymethyl-methacrylate (PMMA), polydimetyl-siloxane (PDMS), and polyvinyl chloride (PVC).

Channels may be defined by grooves formed in an outer surface of the first substrate portion 602a or second substrate portion 602b and covered with a film secured to the outer surface of the substrate portion. Chambers may be defined by recessed cavities formed in the outer surface of the first substrate portion 602a or second substrate portion 602b and covered with a film secured to the outer surface of the substrate portion. Valves may comprise a blockage within a channel that is modifiable from a blocking state preventing fluid flow through the associated channel to an open state permitting fluid flow through the associated channel. The valve may comprise a dissolvable or rupturable blockage or a flexible seal that is manipulable, e.g., by an external actuator, between a blocking state and an open state. In one embodiment, the valve is modifiable between the blocking state and open state and vice versa so as to enable selective fluid flow control through the associated channel. Elements of an exemplary cartridge design and associated processing device that may be applicable to the methods and systems described herein are described in U.S. Patent No. 10,654,039.

In some embodiments, the first fluidic system 600a provides a platform for lysing cellular material contained within a sample by filtering the cellular material from a fluid sample and applying a lysing process to cellular material captured in the filter, such as, chemical and/or heat.

First fluidic system 600a may include one or more functional chambers, up to a plurality of functional chambers, including a sample chamber or reservoir 604, a first reservoir 606, which may contain a first process fluid, a second reservoir 610, which may contain a second process fluid, and a third reservoir 612, which may contain a third process fluid. First fluidic system 600a may further include a syringe barrel 608 (e.g., a first distribution chamber), a filter assembly 620 which may be contained within a filter chamber and may include a conductive mesh, as described above, for effecting an on-filter thermal lysis, and a waste chamber 618.

First fluidic system 600a may further include a fluid channel arrangement including channels (i.e., a first network of fluid channels) and associated flow control valves (i.e., a first plurality of valves) configured to selectively (e.g., one at a time) provide (a) fluid communication between the syringe barrel 608 and each of the sample chamber 604, first reservoir 606, second reservoir 610, and third reservoir 612 and (b) fluid communication between the filter assembly 620 and the syringe barrel 608 and between the filter assembly 620 and the waste chamber 618 or fluid coupling 622.

Solutions and other fluid materials contained in reservoirs of the first fluidic system 600a may be initially stored as dried, reconstitutable materials (e.g., dried or lyophilized enzymes or reagents), and such dried materials may be initially stored in a corresponding reservoir at which a reconstitution reagent is combined with the dried material to form a solution within the reservoir. Reconstitution reagent(s) may be initially stored in a reservoir that is external to the first fluidic system 600a or in one or more additional reservoir(s) that are part of the first fluidic system 600a. The fluid channel arrangement of the first fluidic system 600a may also include channels and valves for transporting reconstitution reagent(s) to reservoir(s) containing dried materials.

The first fluidic system 600a includes a first fluid channel arrangement comprising a first network of fluid channels providing selective fluid connectivity between various reservoirs, chambers, and other components of the system. The first fluid channel arrangement may include a first fluidic network including one or more fluid channels, or conduits, through which fluid may flow and one of more flow control valves for controlling fluid flow through a particular channel of the first fluid channel arrangement (e.g., controlling fluid flow by permitting or preventing fluid flow through the channel). For example, in system 600a shown in FIG. 41, sample reservoir 604 may be connected by a channel 611 to syringe barrel 608 through a valve 650 that is configured to be manipulated (e.g., by an actuator of a processing device to which the first fluidic system 600a is coupled) to selectively prevent fluid flow from the sample reservoir 604 or direct fluid flow from the sample reservoir 604 to the syringe barrel 608 via channel 611.

First reservoir 606 may be connected by a channel 613 to syringe barrel 608 through a valve 652 that is configured to be manipulated (e.g., by an actuator of a processing device to which the first fluidic system 600a is coupled) to selectively prevent fluid flow from the first reservoir 606 or direct fluid flow from the first reservoir 606 to the syringe barrel 608 via channel 613.

Second reservoir 610 may be connected by a channel 616 to syringe barrel 608 through a valve 656 that is configured to be manipulated (e.g., by an actuator of a processing device to which the first fluidic system 600a is coupled) to selectively prevent fluid flow from the second reservoir 610 or direct fluid flow from the second reservoir 610 to the syringe barrel 608 via channel 616.

Third reservoir 612 may be connected by a channel 618 to syringe barrel 608 through a valve 658 that is configured to be manipulated (e.g., by an actuator of a processing device to which the first fluidic system 600a is coupled) to selectively prevent fluid flow from the third reservoir 612 or direct fluid flow from the third reservoir 612 to the syringe barrel 608 via channel 618.

Syringe barrel 608 may be connected by a channel 614 to filter assembly 620 through a valve 654 that is configured to be manipulated (e.g., by an actuator of a processing device to which the first fluidic system 600a is coupled) to selectively prevent fluid flow from the syringe barrel 608 or direct fluid flow from the syringe barrel 608 to the filter assembly 620 via channel 614.

Filter assembly 620 may be connected by channel 624 to a valve 664, which is connected by channel 624a to fluid coupling 622 and is connected by channel 624b to waste chamber 618. Valve 664 is configured to be manipulated (e.g., by an actuator of a processing device to which the first fluidic system 600a is coupled) to selectively (a) prevent fluid flow through channel 624, (b) direct fluid flow from the filter assembly 620 to fluid coupling 622 via channel 624a and channel 624, or (c) direct fluid flow from the filter assembly 620 to waste chamber 618 via channel 624b and channel 624.

In some embodiments, the second fluidic system 600b provides a platform for performing one or more processes associated with analyzing an analyte, including, for example, isolating a target analyte (target capture), amplification of the target analyte, and detection of the target analyte. Second fluidic system 600b may include one or more functional chambers up to a plurality of functional chambers, such as sample preparation chamber 626 (which may constitute or comprise a target isolation chamber or target capture chamber as shown in FIG. 35), an amplification chamber 628, a hybridization/detection chamber (or analysis chamber) 630, a hybridization buffer reservoir 638, a hybridization wash buffer reservoir 640, a wash buffer reservoir 634, and an elution buffer/amplification reagent reservoir 636.

Second fluidic system 600b may include a waste chamber 632, or, in an alternate embodiment, first fluidic system 600a and second fluidic system 600b may share a common waste chamber.

Solutions and other fluid materials contained in reservoirs of the second fluidic system 600b may be initially stored as dried, reconstitutable materials (e.g., dried or lyophilized reagents), and such dried materials may be initially stored in a corresponding reservoir at which a reconstitution reagent is combined with the dried material to form a solution within the reservoir. Reconstitution reagent(s) may be initially stored in a reservoir that is external to the second fluidic system 600b or in one or more additional reservoir(s) that are part of the second fluidic system 600b. The second fluidic system 600b includes a second fluid channel arrangement providing selective fluid connectivity between various reservoirs, chambers, and other components of the system. The second fluid channel arrangement may include a second fluidic network including one or more fluid channels, or conduits, (a second network of fluid channels) through which fluid may flow and one or more flow control valves (which may comprise a second plurality of valves) for controlling fluid flow through a particular channel of the second fluid channel arrangement (e.g., controlling fluid flow by permitting fluid flow through a channel or preventing fluid flow through the channel).

In one embodiment, the second fluidic system 600b may include a second syringe barrel, or second distribution chamber, (not shown) for selectively drawing sample material from the first fluidic system 600a to the second fluidic system 600b. Second syringe barrel may be similar to syringe barrel 608 of first fluidic system 600a and may be operatively coupled to a syringe, such as syringe 150, and, in one embodiment, the second fluidic network is configured to selectively (e.g., one at a time) permit fluid flow: (i) between the target isolation chamber 626 and the second syringe barrel, (ii) between the second syringe barrel and the amplification chamber 628, and (iii) between the second syringe barrel and the analysis chamber 630.

Second fluidic system 600b includes a second fluid channel arrangement providing selective fluid connectivity between various reservoirs, chambers, and other components of the system. The second fluid channel arrangement may include a number of fluid channels, or conduits, through which fluid may flow in a first or second direction and valves for controlling fluid flow through a particular conduit. For example, in system 600b shown in FIG. 41, wash buffer reservoir 634 may be connected by a channel 635 to target isolation chamber 626 through a valve 676 that is configured to be manipulated (e.g., by an actuator of a processing device to which the second fluidic system 600b is coupled) to selectively prevent fluid flow from the wash buffer reservoir 634 or direct fluid flow from the wash buffer reservoir 634 to the target isolation chamber 626 via channel 635.

Elution buffer/amplification reagent reservoir 636 may be connected by a channel 637 to target isolation chamber 626 through a valve 678 that is configured to be manipulated (e.g., by an actuator of a processing device to which the second fluidic system 600b is coupled) to selectively prevent fluid flow from the elution buffer/amplification reagent reservoir 636 or direct fluid flow from the elution buffer/amplification reagent reservoir 636 to the target isolation chamber 626 via channel 637.

Target isolation chamber 626 may be connected by a channel 629 to waste chamber 632 through a valve 670 that is configured to be manipulated (e.g., by an actuator of a processing device to which the second fluidic system 600b is coupled) to selectively prevent fluid flow through channel 629 or direct fluid flow from the target isolation chamber 626 to the waste chamber 632 via channel 629.

Target isolation chamber 626 may be connected by a channel 631 to amplification chamber 628 through a valve 672 that is configured to be manipulated (e.g., by an actuator of a processing device to which the second fluidic system 600b is coupled) to selectively prevent fluid flow through channel 631 or direct fluid flow from the target isolation chamber 626 to the amplification chamber 628 via channel 631.

In embodiments in which a protocol for analyte detection does not include a process for isolating and purifying the microbial nucleic acid, target isolation chamber 626, wash buffer reservoir 634, elution buffer/amplification reagent reservoir 636, and waste chamber 632, as well as channels 629, 631, 635, and 637 and valves 670, 672, 676, and 678, may be omitted from the second fluidic system 600b. If the target isolation chamber 626 is omitted, amplification chamber 628 may be connected to filter assembly 620 of the first fluidic system 600a via channels 624, 624a, and fluid coupling 622 controlled by valve 664.

Amplification chamber 628 may be connected by a channel 633 to hybridization/detection chamber 630 through a valve 674 that is configured to be manipulated (e.g., by an actuator of a processing device to which the second fluidic system 600b is coupled) to selectively prevent fluid flow through channel 633 or direct fluid flow from the amplification chamber 628 to the hybridization/detection chamber 630 via channel 633.

Hybridization wash buffer reservoir 640 may be connected by a channel 639 to hybridization/detection chamber 630 through a valve 680 that is configured to be manipulated (e.g., by an actuator of a processing device to which the second fluidic system 600b is coupled) to selectively prevent fluid flow from the hybridization wash buffer reservoir 640 or direct fluid flow from the hybridization wash buffer reservoir 640 to the hybridization/detection chamber 630 via channel 639.

Hybridization buffer reservoir 638 may be connected by a channel 641 to hybridization/detection chamber 630 through a valve 682 that is configured to be manipulated (e.g., by an actuator of a processing device to which the second fluidic system 600b is coupled) to selectively prevent fluid flow from the hybridization buffer reservoir 638 or direct fluid flow from the hybridization buffer reservoir 638 to the hybridization/detection chamber 630 via channel 641.

In an embodiment, hybridization chamber 630 comprises a biosensor for detecting the presence of specific target molecules in the sample. The biosensor interacts with the target molecule by ligation. One example of a biosensor is a microarray (e.g., nucleic acid array) or biochip. With such a microarray or biochip, the identity and quantity of a target nucleic acid in a sample may be detected by measuring the level of association of the target sequence with probes specifically provided for the sequence. In nucleic acid microarray or biochip technologies, a set of probe nucleic acids, each having a defined sequence, is immobilized on a solid support or substrate, e.g., a nucleic acid array, in such a way that each probe occupies a predetermined position. One advantage of a microarray or biochip is that it enables simultaneous detection across all the probe nucleic acids with the same optical system (e.g., a camera). The processing device to which the second fluidic system 600b is coupled may include means for optical excitation of the array of the second fluidic system 600b and means for optical detection of an optical signal that is representative of the nucleic acid in the sample analyzed by the second fluidic system 600b.

In other embodiments, amplification and detection may occur in the same chamber.

### Analyte Isolation/Detection

Following release and recovery of microbial cell analytes, a method for isolating an analyte as described above may further include analyzing the isolated microbial analyte. The type of assay depends on the analyte.

For example, in some variations wherein the analyte is a nucleic acid, analyzing the isolated nucleic acid includes amplification. In such embodiments, the isolated nucleic acid analyte is used as a template in an in vitro nucleic acid amplification reaction, utilizing at least two amplification oligomers flanking a target sequence within the nucleic acid analyte, to generate an amplification product corresponding to the target sequence. A nucleic acid analyte can be amplified using methods such as isothermal amplification reactions (e.g., transcription mediated amplification (TMA), nucleic acid sequence-based amplification (NASBA), loop mediated isothermal amplification (LAMP), polymerase spiral reaction (PSR) (Liu et al., Sci. Rep. 5: 12723, 2015), ligase chain reaction (LCR), and other isothermal amplification methods), or temperature cycling amplification reactions (e.g., polymerase chain reaction (PCR) or other temperature cycling amplification methods), or other amplification methods. In particular embodiments comprising PCR, the PCR may be selected from quantitative PCR (qPCR) and real time PCR (RT-PCR).

Amplification may be performed with or without prior capture of the nucleic acid analyte. In some variations comprising a capture step, the nucleic acid analyte of interest is captured by hybridizing the nucleic acid to an immobilized capture probe attached to a solid support prior to the amplification step.

Detection of the amplified nucleic acid analyte products can be performed during amplification (real-time) or following amplification (end-point) by using any known method. Amplified nucleic acids may be detected in solution phase or by immobilizing them on a solid support (e.g., a nucleic acid array) and detecting labels associated with them (e.g., an intercalating agent such as ethidium bromide). Some detection methods use detection probes complementary to a sequence in the amplified product and detect the presence of the probe:product complex (e.g., by detecting a label joined to the probe), or use a complex of probes to amplify the signal detected from amplified products (see, e.g., U.S. Pat. Nos. 5,424,413, 5,451,503 and 5,849,481). Other detection methods use a probe in which signal production is linked to the presence of the target sequence because a change in signal results only when the labeled probe binds to amplified product, such as in a molecular beacon, molecular torch, or hybridization switch probe (e.g., U.S. Pat. Nos. 5,118,801, 5,210,015, 5,312,728, 5,538,848, 5,541,308, 5,656,207, 5,658,737, 5,925,517, 6,150,097, 6,361,945, 6,534,274, 6,835,542, and 6,849,412; and U.S. Pub. No. 2006/0194240 A1). Such probes typically use a label (e.g., fluorophore) attached to one end of the probe and an interacting compound (e.g., quencher) attached to another location of the probe to inhibit signal production from the label when the probe is in one conformation ("closed") that indicates it is not hybridized to amplified product, but a detectable signal is produced when the probe is hybridized to the amplified product which changes its conformation (to "open"). Detection of a signal from directly or indirectly labeled probes that specifically associate with the amplified product indicates the presence of the target nucleic acid that was amplified.

In particular variations, an amplification and detection assay for analyzing isolated nucleic acid is a qPCR assay. Such assays include forward and reverse primers for target amplification, and a target-specific detection probe labeled with a fluorophore at the 5'-end and a quencher at the 3'-end (also referred to as a hydrolysis probe or TAQMAN probe). In this format, the quencher decreases the fluorescence intensity of the fluorophore through a process such as excited state reactions, energy transfer, complex-formation or collisional quenching. If the target nucleic acid is present in the sample, the probe binds to the complementary sequence within the amplification target region. As the polymerase extends the 3'-end of the primer and synthesizes a nascent complementary strand, a 5' to 3' exonuclease activity of the polymerase degrades the bound probe, thereby releasing the quencher so that it no longer has an effect on the fluorophore. This is seen in increasing fluorescence intensity, according to the rate of amplification.

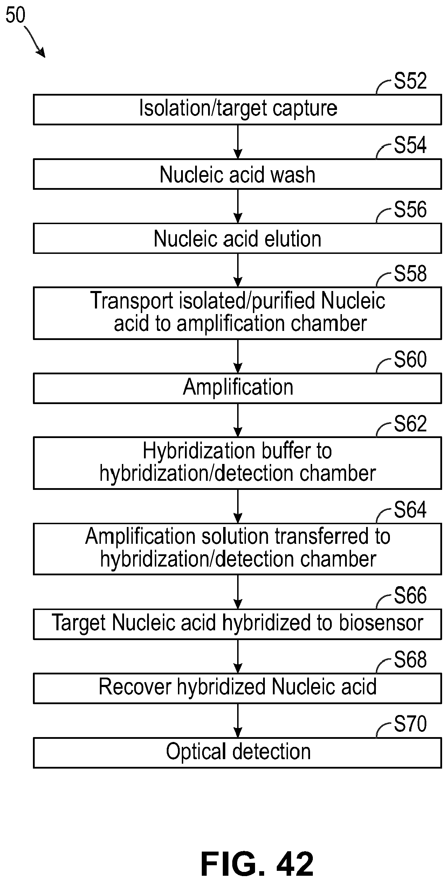

FIG. 42 is a flow chart showing a method 50 for analyzing an analyte (e.g., nucleic acid) received from the lysis process, for example, by isolating, amplifying, and detecting a target nucleic acid, for example, as is described in U.S. Patent No. 10,654,039. The method illustrated in FIG. 42 is described below with reference to system 600 shown in FIG. 41.

For a process in which capture of the nucleic acid is performed prior to amplification, target capture or isolation is performed in step S52. In one example, step S52 is accomplished by actuating one or more valves to selectively connect target isolation chamber 626 to filter assembly 620, and eluate is transported directly from the filter assembly 620 to the target isolation chamber 626 without first being received in an intermediate elution chamber. The target isolation chamber 626 may contain a binding substrate or membrane, such as a silica-like membrane, for selective binding to at least one nucleic acid of interest. The binding substrate or membrane may comprise, for example, a gel, beads, or a paper filter for nucleic acid binding and concentration. More specifically, the binding substrate or membrane may comprise, agarose gel, silica beads, and/or filter paper, such as cellulose.

Once the binding to the binding substrate or membrane is completed, the waste chamber 632 is selectively connected to the target isolation chamber 626 (for example, by opening valve 670 and closing valves 672, 676, and 678), and a portion of the sample containing unbound, non-target analyte is transported from the target isolation chamber 626 to the waste chamber 632 via channel 629, while the captured, target nucleic acid is retained within target isolation chamber 626.

In step S54, bound target nucleic acid within target isolation chamber 626 is recovered by washing it in order to remove inhibitors and purify the nucleic acid. In one example, step S54 is accomplished by selectively connecting wash buffer reservoir 634 to target isolation chamber 626 (for example, by opening valve 676 and closing valves 670, 672, and 678), transporting wash buffer via channel 635 from wash buffer reservoir 634 to target isolation chamber 626, and washing the binding membrane within the target isolation chamber 626 with the wash buffer. In an embodiment, the same operation may be repeated with a second nucleic acid wash buffer contained in another wash buffer reservoir (not shown). Used wash buffer may be directed to waste chamber 632 by selectively connecting waste chamber 632 to target isolation chamber 626 (for example, by selectively opening valve 670 while valves 672, 676, and 678 are closed) and transporting used wash buffer from the target isolation chamber 626 to the waste chamber 632 via channel 629.

In step S56, the nucleic acid bound to the binding membrane is eluted with an elution buffer, which may also include an amplification reagent. In an embodiment, elution buffer/amplification reagent reservoir 636 is selectively connected to target isolation chamber 626 (for example, by opening valve 678 and closing valves 670, 672, and 676) and elution buffer is transported from elution buffer/amplification reagent reservoir 636 via channel 637 to target isolation chamber 626 to elute the nucleic acid bound to the binding membrane. An amplification-mix solution contained in the elution buffer/amplification reagent reservoir 636 can be used as an elution buffer.

At the end of step S56, an isolated nucleic acid sample remains.

In step S58, after elution, the amplification chamber 628 is selectively connected to the target isolation chamber 626 (for example, by selectively opening valve 672 while valves 670, 676, and 678 are closed) and the isolated nucleic acid is transported via channel 631 from target isolation chamber 626 to the amplification chamber 628 for amplification. As noted, the step of isolating and purifying an analyte of interest within the second fluidic system 600b may be omitted, and thus, steps S52, S54, and S56 may be viewed as optional, depending on the protocol employed. In such an embodiment, lysed analyte from the first fluidic system 600a is delivered to the amplification chamber 628 from the filter assembly 620. In such an arrangement, amplification reagents may be pre-stored in the amplification chamber 628 or may be transferred to the amplification chamber 628 from a separate amplification reagent reservoir, before or after sample material is transferred into the amplification chamber 628.

In step S60, a nucleic acid amplification reaction may be performed in amplification chamber 628 by standard amplification protocols (typically any amplification method, including but not limited to, polymerase chain reaction (PCR), reverse transcriptase PCR, isothermal amplification, and other amplification protocols, including those described above) achieving a very good sensitivity and specificity for up to 20 markers. In an embodiment, a set of primers may have been immobilized (e.g., lyophilized) within amplification chamber 628 during the cartridge manufacturing process. These primers may re-suspended when the amplification-mix is transferred to amplification chamber 628. The amplification-mix may contain, in the case of PCR, a thermostable DNA polymerase, deoxynucleotide triphosphates, magnesium, and a reaction buffer at suitable concentrations for efficient amplification of nucleic acid templates. At the end of step S60, one obtains an amplified nucleic acid sample, provided target nucleic acid was originally present in the sample.

In step S62, hybridization buffer reservoir 638 is selectively connected to hybridization/detection chamber 630 (for example, by selectively opening valve 682 while valves 674 and 680 are closed) and hybridization buffer is transported from hybridization buffer reservoir 638 via channel 641 to hybridization/detection chamber 630.

Next, in step S64, amplification chamber 628 is selectively connected to hybridization/detection chamber 630 (for example, by selectively opening valve 674 while valves 672, 680, and 682 are closed) and amplification solution is transported via channel 633 from amplification chamber 628 to hybridization/detection chamber 630, thereby putting the amplified nucleic acid sample in contact with the hybridization buffer transferred in step S62.

In step S66, the amplified nucleic acid sample may be placed into contact with the biosensor (e.g., the nucleic acid microarray or biochip) within the hybridization/detection chamber 630 in such a way that complementary sequences can bind to an immobilized probe. After the removal of unbound material, the bound sequences are ready for detection and measurement. In one embodiment, in step S66 the amplified nucleic acid sample is hybridized, for a period of several minutes, e.g., about 30 minutes, in the hybridization/detection chamber 630.

In step S68, hybridization wash buffer reservoir 640 is selectively connected to hybridization/detection chamber 630 (for example, by selectively opening valve 680 while valves 674 and 682 are closed) and hybridization wash buffer is transported via channel 639 from the hybridization wash buffer reservoir 640 to hybridization/detection chamber 630 to recover hybridized nucleic acid. A hybridized nucleic acid sample is therefore obtained.

In a variant of the analysis method, a nucleic acid melting procedure at the end of hybridization may be added and would allow an increase in detection specificity.

In step S70, an array image is obtained and analyzed. The detection of the interaction between the target nucleic acids and the probes of the biosensor are performed by an optical detection device. The localized hybridization may be detected by, e.g., the emission of a chromogenic signal. Herein, "chromogenic signal" is to be understood as any light signal emitted directly, or indirectly, after excitation by a suitable light source or after chemical or enzymatic transformation. Chromogenic signals include colorimetric, photoluminescent, fluorescent, chemoluminescent, bioluminescent signals, or the like. Such signals are either directly emitted by the molecules of interest, or emitted by detectable elements (tags), which are added and/or grafted thereto.

The processing device to which the second fluidic system 100b is coupled may include an optical detector, such as a fluorescence reader configured to obtain a fluorescent image of the biosensor surface. For that purpose, the biosensor is illuminated (excited) with a light source at the wavelength of excitation of the fluorophore marking the target molecules, and an adapted optical system forms an image of the fluorescence of the biosensor at the wavelength of emission of the fluorophores. The light intensity of each point of this image is related to the quantity of fluorophores present at the corresponding point of the biosensor, which is itself proportional to the number of target molecules that have been selectively attached at this place during the hybridization phase, which makes it possible to collect information (often quantitative) about the nucleic acid content of the sample.

### Hardware and Software

Aspects of the subject matter disclosed herein may be implemented via control and computing hardware components, software (which may include firmware), data input components, and data output components. Hardware components include computing and control modules (e.g., system controller(s)), such as microprocessors, embedded controllers, application specific integrated circuits (ASICS), and computers, configured to effect computational and/or control steps by receiving one or more input values, executing one or more algorithms stored on non-transitory machine-readable media (e.g., software) that provide instruction for manipulating or otherwise acting on or in response to the input values, and output one or more output values. Such outputs may be displayed or otherwise indicated to a user for providing information to the user, for example information as to the status of the instrument or of a process being performed thereby, or such outputs may comprise inputs to other processes and/or control algorithms. Data input components comprise elements by which data is input for use by the control and computing hardware components. Such data inputs may comprise signals generated by sensors or scanners, such as, position sensors, speed sensors, accelerometers, environmental (e.g., temperature) sensors, motor encoders, barcode scanners, or RFID scanners, as well as manual input elements, such as keyboards, stylus-based input devices, touch screens, microphones, switches, manually operated scanners, etc. Data inputs may further include data retrieved from memory. Data output components may comprise hard drives or other storage media, monitors, printers, indicator lights, or audible signal elements (e.g., chime, buzzer, horn, bell, etc.).

All possible combinations of elements and components described in the specification or recited in the claims are contemplated and considered to be part of this disclosure. It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein.

While the subject matter of this disclosure has been described and shown in considerable detail with reference to certain illustrative embodiments, including various combinations and sub-combinations of features, those skilled in the art will readily appreciate other embodiments and variations and modifications thereof as encompassed within the scope of the present disclosure. Moreover, the descriptions of such embodiments, combinations, and sub-combinations is not intended to convey that the claimed subject matter requires features or combinations of features other than those expressly recited in the claims. Accordingly, the scope of this disclosure is intended to include all modifications and variations encompassed within the scope of the following appended claims.

## Claims

1. A method comprising the steps of:
(a) moving a sample through filter media and a conductive mesh disposed adjacent the filter media, wherein the filter media and the conductive mesh are contained within a filter chamber, and wherein the filter media includes pores or interstices that are sized so as to prevent the passage of at least some cells contained within the sample to thereby separate the cells from a fluid component of the sample flowing through the filter media; and
(b) after step (a), energizing an induction coil disposed outside the filter chamber and positioned to effect inductive heating of the conductive mesh when the induction coil is energized to inductively heat the conductive mesh and thermally lyse cells separated from the fluid component of the sample by the filter media, such that nucleic acids are released from the separated cells.

2. The method of claim 1, further comprising, prior to step (a), combining the sample with a lysing solution, wherein step (a) comprises moving the sample and the lysing solution through the filter media and the conductive mesh, optionally further comprising, after step (a) and before step (b), the step of moving a wash buffer through the filter media and the conductive mesh, and optionally further comprising, after step (b), the step of moving an elution buffer through the filter media and the conductive mesh.

3. The method of claim 1 or claim 2, further comprising the steps of:
monitoring a temperature of the conductive mesh; and
controlling the energizing of the induction coil based on the temperature of the conductive mesh.

4. The method of any one of claims 1-3, wherein the induction coil at least partially overlies or underlies the conductive mesh.

5. The method of any one of claims 1-4, wherein the conductive mesh is comprised of a metal or combination of metals, and wherein the metal optionally includes one or more of iron, copper, stainless steel, nickel, and/or aluminum.

6. The method of any one of claims 1-5, wherein the conductive mesh overlies or underlies the filter media or wherein the conductive mesh overlies and underlies the filter media, and wherein at least part of the conductive mesh is optionally in contact with at least part of the filter media.

7. The method of any one of claims 1-6, wherein the filter media and the conductive mesh are flat and optionally parallel to each other.

8. The method of any one of claims 1-7, wherein the thickness of the conductive mesh ranges from 130 µm - 350 µm.

9. The method of any one of claims 1-8, wherein the filter media has a generally cylindrical configuration, wherein the conductive mesh and the induction coil have generally cylindrical configurations arranged coaxially with the filter media, and wherein the conductive mesh is optionally positioned radially outwardly of the filter media so that the conductive mesh at least partially encompasses the filter media.

10. The method of claim 9, comprising a plenum positioned within the filter chamber to receive fluid at the fluid inlet and introduce the fluid into the filter chamber at a location that is radially inward of the filter media, wherein the fluid flows radially outwardly through the filter media and the conductive mesh, and wherein the fluid outlet is located radially outwardly of the conductive mesh.

11. The method of any one of claims 1-10, wherein the conductive mesh comprises a metal woven wire mesh and wherein the metal woven wire mesh optionally has openings of about 60 µm, or the conductive mesh comprises sintered metal fibers and wherein the sintered metal fibers optionally define openings of about 3 µm to 100 µm, or wherein the conductive mesh comprises a perforated metal plate having openings formed through the perforated plate and wherein each of the openings optionally has a maximum width ranging from about 0.04 mm to about 0.3 mm.

12. The method of any one of claims 1-11, wherein the filter media comprises a porous substrate, the porous substrate is optionally made of polyethersulfone, cellulose, nylon, polyvinylidene fluoride (Poly(1,1-difluoroethylene), PVDF), polycarbonate, or glass fiber, the porous substrate optionally includes pores with a size of between about 0.1 µm and about 1.0 µm, and the porous substrate optionally has a thickness of about 160 µm to about 185 µm.

13. The method of claim 12, wherein step (a) comprises moving a sample through the porous substrate from a first side of the porous substrate to a second side of the porous substrate, and wherein the porous substrate includes pores decreasing in size from the first side of the porous substrate to the second side of the porous substrate, the first side of the porous substrate optionally includes pores with a size of between about 5 µm and about 20 µm and wherein the second side of the porous substrate optionally includes pores with a size of up to about 0.2 µm in diameter, the porous substrate optionally comprises multiple layers of decreasing pore size progressing from the first side of the porous substrate to the second side of the porous substrate.

14. The method of any one of claims 1-11, wherein the filter media comprises a track etched membrane optionally having pores of about 0.2 µm in size and optionally having a thickness of about 22 µm, and wherein the filter media optionally includes a layer of sintered plastic particles optionally made of polyethylene, polypropylene, polytetrafluoroethylene, or polyvinyllidene fluoride, interstices between the sintered plastic particles optionally range in size from about 5 µm to about 15 µm, and the layer of sintered plastic particles optionally has a thickness of about 450 µm to 650 µm.

15. The method of any one of claims 1-14, wherein step (a) is performed in a fluid cartridge comprising:
a syringe barrel for receiving a syringe plunger or a syringe port for coupling a syringe to the fluid cartridge, and optionally including a syringe plunger received within the syringe barrel or coupled to the syringe port;
a sample reservoir or a sample port for connecting an external sample receptacle to the fluid cartridge;
one or more process fluid reservoirs;
a waste chamber;
a fluid discharge port from which a fluid is discharged from the cartridge; and
a fluid flow network comprising fluid channels and flow control valves, and wherein the method further comprises selectively opening and closing each of the flow control valves to selectively permit flow: (i) from the sample reservoir or the sample port to the syringe barrel or the syringe port, (ii) from the syringe barrel or the syringe port to the filter chamber and from the filter chamber to the waste chamber, (iii) from at least one of the one or more process fluid reservoirs to the syringe barrel or the syringe port, and (iv) from the syringe barrel or the syringe port to the filter chamber and from the filter chamber to the fluid discharge port, wherein the fluid cartridge optionally comprises a body made from plastic, and wherein the plastic optionally comprises polypropylene, polycarbonate, polyacrylamide, polyethylene, polymethyl-methacrylate (PMMA), polydimetyl-siloxane (PDMS), or polyvinyl chloride (PVC).
